(19) Europäisches Patentamt
European Patent Office
Office européen des brevets

(11) EP 0 757 990 B1

(12) EUROPÄISCHE PATENTSCHRIFT

(45) Veröffentlichungstag und Bekanntmachung des Hinweises auf die Patenterteilung:
**30.03.2005 Patentblatt 2005/13**

(51) Int Cl.[7]: **C07D 401/04**, C07D 498/04, C07D 471/04, C07D 487/04, A61K 31/47, A61K 31/535, A61K 31/54

(21) Anmeldenummer: **96113744.5**

(22) Anmeldetag: **30.06.1989**

(54) **7-(1-Pyrrolidinyl)-3-chinolon- und -naphthyridoncarbonsäure-Derivate, Verfahren zu ihrer Herstellung sowie substituierte mono- und bicyclische Pyrrolidinderivate als Zwischenprodukte zu ihrer Herstellung, und sie enthaltende antibakterielle Mittel und Futterzusatzstoffe**

7-(1-Pyrrolidoinyl)-3-quinolone and naphthyridone carboxylic acid derivatives, method for their preparation and for substituted mono- and bicyclic pyrrolidine intermediates, and their antibacterial and feed additive compositions

Dérivés d'acides 7-(1-pyrrolidinyl)-3-quinolone et -naphtyridone carboxyliques, procédé pour leur préparation, ainsi que des dérivés mono- et bicycliques substitués de pyrrolidines comme intermédiaires pour leur réalisation, leurs compositions antibacté rielles et additifs alimentaires

(84) Benannte Vertragsstaaten:
**AT BE CH DE ES FR GB GR IT LI LU NL SE**

(30) Priorität: **15.07.1988 DE 3824072**
**01.03.1989 DE 3906365**

(43) Veröffentlichungstag der Anmeldung:
**12.02.1997 Patentblatt 1997/07**

(62) Dokumentnummer(n) der früheren Anmeldung(en) nach Art. 76 EPÜ:
**89111950.5 / 0 350 733**

(73) Patentinhaber: **Bayer HealthCare AG**
**51368 Leverkusen (DE)**

(72) Erfinder:
- **Petersen, Uwe, Dr.**
  **51375 Leverkusen (DE)**
- **Schenke, Thomas, Dr.**
  **51469 Bergisch-Gladbach (DE)**
- **Krebs, Andreas, Dr.**
  **51519 Odenthal (DE)**
- **Grohe, Klaus, Dr.**
  **51519 Odenthal (DE)**
- **Schriewer, Michael, Dr.**
  **51519 Odenthal (DE)**
- **Haller, Ingo, Dr.**
  **42111 Wuppertal (DE)**
- **Metzger, Karl Georg, Dr.**
  **42115 Wuppertal (DE)**
- **Endermann, Rainer, Dr.**
  **42113 Wuppertal (DE)**
- **Zeiler, Hans-Joachim, Dr.**
  **42113 Wuppertal (DE)**

(74) Vertreter: **Hansen, Bernd, Dr. Dipl.-Chem. et al**
**Hoffmann Eitle,**
**Patent- und Rechtsanwälte,**
**Arabellastrasse 4**
**81925 München (DE)**

(56) Entgegenhaltungen:
**EP-A- 0 106 489** **EP-A- 0 241 206**

- **JOURNAL OF MEDICINAL CHEMISTRY, Bd. 27, Nr. 12, 1984, WASHINGTON US, Seiten 1543-1549, XP002015009 H. EGAWA ET AL.: "Pyridonecarboxylic acids as antibacterial agents"**
- **CHEMICAL ABSTRACTS, vol. 104, no. 7, 17.Februar 1986 Columbus, Ohio, US; abstract no. 50861t, Seite 521; XP002015010 & JP-A-60 126 284 (DAINIPPON) 5.Juli 1985**
- **CHEMICAL ABSTRACTS, vol. 111, no. 11, 11.September 1989 Columbus, Ohio, US; abstract no. 97210q, M. IWATA ET AL.: "Preparation of 7-pyrrolidino-1,4-dihydo-4-oxo-1,8-naphthy ridine- or ..." Seite 742; XP002015011 & JP-A-06 403 181 (SANKYO) 6.Januar 1989**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 757 990 B1

- **CHEMICAL ABSTRACTS, vol. 111, no. 17, 23.Oktober 1989 Columbus, Ohio, US; abstract no. 153779w, K. CHIBA ET AL.: "Preparation of 1,4-dihydro-4-oxoquinoline-3-carboxylic acids" Seite 721; XP002015012 & JP-A-01 056 673 (DAINIPPON) 3.März 1989**

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft neue 7-(1-Pyrrolidinyl)-3-chinolon- und -naphthyridoncarbonsäure-Derivate, Verfahren zu ihrer Herstellung sowie diese enthaltende antibakterielle Mittel und Futterzusatzstoffe.

Es ist bereits eine Reihe von 3-Chinolon- und Naphthyridoncarbonsäuren bekanntgeworden, die in 7-Position durch einen Pyrrolidinyl-Ring substituiert sind. Deutsche Patentanmeldung 3 318 145, Europäische Patentanmeldungen 106 489 und 153 826.

Es wurde gefunden, daß die 7-(1-Pyrrolidinyl)-3-chinolon-und -naphthyridoncarbonsäure-Derivate der Formel (I)

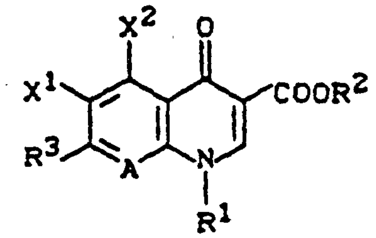

(I),

in welcher

$X^1$ für Halogen,

$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 4 Kohlenstoffatomen oder (5-Methyl-2-oxo-1,3-dioxo3-4-yl)-methyl,

$R^3$ für einen Rest der Struktur

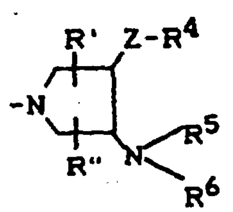

,

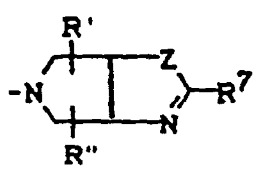

,

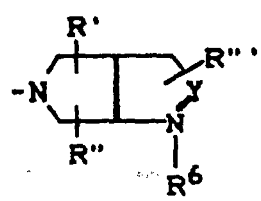

steht, worin

$R^4$ für H, $C_1$-$C_4$-Alkyl, Aryl, $C_1$-$C_4$-Acyl

$R^5$ für H, $C_1$-$C_4$-Alkyl, OH, $OCH_3$, wobei $R^4$ und $R^5$ gemeinsam auch eine gegebenenfalls durch Methyl ein- oder zweifach substituierte $C_1$-$C_3$-Alkylenbrücke bedeuten können,

$R^6$ für H, gegebenenfalls durch Hydroxy substituiertes $C_1$-$C_4$-Alkyl, sowie Aryl, Heteroaryl, Benzyl, $C_1$-$C_4$-Alkoxycarbonyl, $C_1$-$C_4$-Acyl, (5-Methyl-2-oxo-1,3-dioxol-4-yl)-methyl oder $C_3$-$C_6$-Cycloalkyl,

$R^7$ für H oder $C_1$-$C_4$-Alkyl,

R' für H, $CH_3$ oder Phenyl,

R" für H, $CH_3$ oder Phenyl,

R''' für H oder $CH_3$,

Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,

Z für O oder S stehen kann,

A für N oder C-$R^8$ steht, worin

$R^8$ für H, Halogen, Methyl, Cyano, Nitro, Hydroxy oder Methoxy steht oder auch gemeinsam mit $R^1$ eine Brücke der Struktur

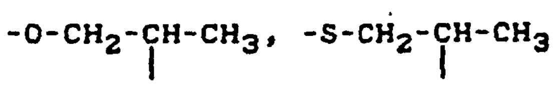

oder

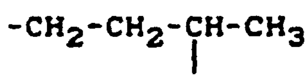

bilden kann,
und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung insbesondere im grampositiven Bereich aufweisen.

Bevorzugt sind die Verbindungen der Formel (I)

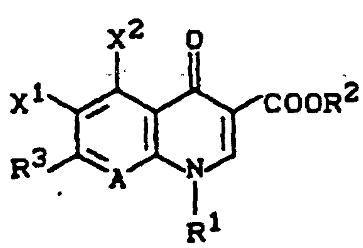

(I),

in welcher

$X^1$ für Fluor oder Chlor,

$X^2$ für Wasserstoff, Amino, Alkylamino mit 1 bis 2 Kohlenstoffatomen, Dimathylamino, Hydroxy, Methoxy, Mercapto, Methylthio, Phenylthio, Fluor, Chlor,

$R^1$ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 3 Kohlenstoffatomen oder (5-Methyl-2-oxo-1,3-dioxol-4-yl)-methyl,

$R^3$ für einen Rest der Struktur

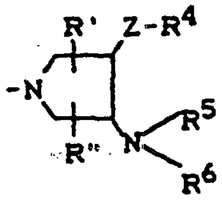 , 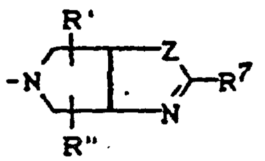 , 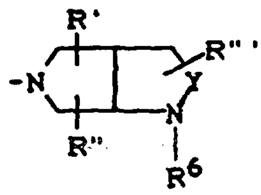

steht, worin

$R^4$ für H, $C_1$-$C_3$-Alkyl, $C_1$-$C_2$-Acyl

$R^5$ für H, $C_1$-$C_3$-Alkyl, OH, $OCH_3$, wobei $R^4$ und $R^5$ gemeinsam auch eine gegebenenfalls durch Methyl ein- oder zweifach substituierte $C_1$-$C_2$-Alkylenbrücke bedeuten können,

$R^6$ für H, gegebenenfalls durch Hydroxy substituiertes $C_1$-$C_3$-Alkyl, sowie Phenyl, Benzyl, $C_1$-$C_4$-Alkoxycarbonyl, $C_1$-$C_2$-Acyl, (5-Methyl-2-oxo-1,3-dioxol-4-yl)-methyl oder $C_3$-$C_5$-Cycloalkyl,

$R^7$ für H oder $C_1$-$C_2$-Alkyl,

R' für H oder $CH_3$,

R" für H oder $CH_3$,

R"' für H oder $CH_3$,

Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,

Z für O oder S stehen kann,

A für N oder C-$R^8$ steht, worin

$R^8$ für H, Fluor, Chlor, Brom, Methyl, Nitro, Hydroxy oder Methoxy steht oder auch gemeinsam mit $R^1$ eine Brücke der Struktur

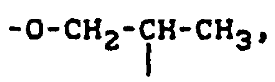

bilden kann.

Besonders bevorzugt sind die Verbindungen der Formel (I)

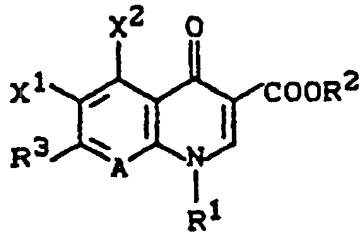 (I),

in welcher

$X^1$ für Fluor,

$X^2$ für Wasserstoff, Amino, Methylamino, Fluor,

$R^1$ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,

$R^2$ für Wasserstoff, Alkyl mit 1 bis 2 Kohlenstoffatomen,

$R^3$ für einen Rest der Struktur

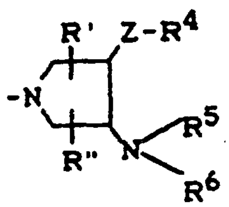 , 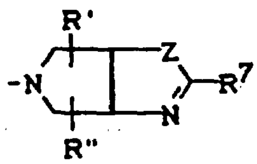 , 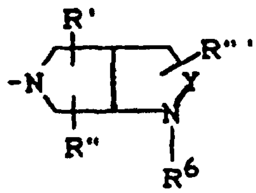

steht, worin

$R^4$ für H, $C_1$-$C_2$-Alkyl, Acetyl,

$R^5$ für H, $C_1$-$C_2$-Alkyl, wobei $R^4$ und $R^5$ gemeinsam auch eine gegebenenfalls durch Methyl substituierte $C_1$-$C_2$-Alkylenbrücke bedeuten können,

$R^6$ für H, $CH_3$, $C_2H_5$, $HOCH_2CH_2$, Benzyl, $C_1$-$C_4$-Alkoxycarbonyl, $C_1$-$C_2$-Acyl,

$R^7$ für H oder $CH_3$,

R' für H oder $CH_3$,

R" für H oder $CH_3$,

R"' für H oder $CH_3$,

Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,

Z für O oder S stehen kann,

A für N oder C-$R^8$ steht, worin

$R^8$ für H, Fluor oder Chlor steht, oder auch gemeinsam mit $R^1$ eine Brücke der Struktur

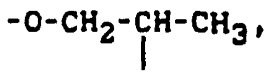

bilden kann.

Weiterhin wurde gefunden, daß man die Verbindungen der Formel (I) erhält, wenn man Verbindungen der Formel (II)

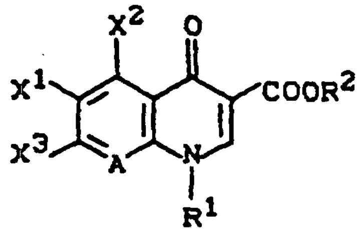

(II),

in welcher
$R^1$, $R^2$, $X^1$ und $X^2$ die oben angegebene Bedeutung haben und

$X^3$ für Halogen, insbesondere Fluor oder Chlor steht, mit Verbindungen der Formel (III)

$$R^3\text{-H} \qquad \text{(III)},$$

in welcher
$R^3$ die oben angegebene Bedeutung hat,

gegebenenfalls in Gegenwart von Säurefängern umsetzt, und gegebenenfalls in $R^3$ enthaltene Schutzgruppen abspaltet (Methode A).

Erfindungsgemäße Verbindungen der Formel (I)

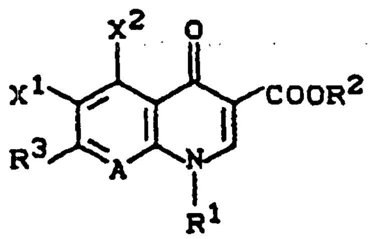

(I),

in welcher
$X^1$, $R^1$, $R^2$, $R^3$ und A die oben angegebene Bedeutung haben und

$X^2$ für Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,

können auch erhalten werden, indem man eine Verbindung der Formel (IV)

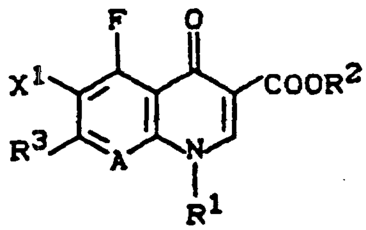

(IV),

in welcher
$X^1$, $R^1$, $R^2$, $R^3$ und A die oben angegebene Bedeutung haben,
mit Verbindungen der Formel (V)

$$X^2\text{-H} \quad (V),$$

in welcher
$X^2$ die oben angegebene Bedeutung hat, gegebenenfalls in Gegenwart von Säurefängern umsetzt (Methode B).

Erfindungsgemäße Verbindungen der Formel (Ia)

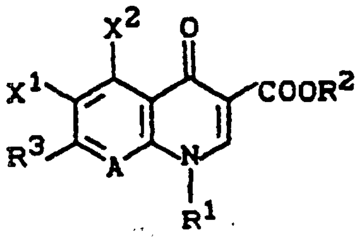

(Ia),

in welcher
$X^1$, $X^2$, $R^1$, $R^2$ und A die oben angegebene Bedeutung haben und $R^3$ für einen Rest der Struktur

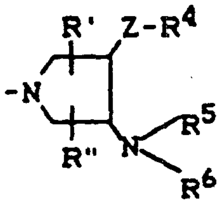

,

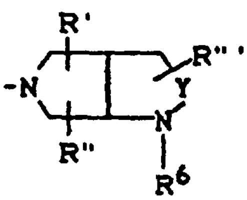

steht, worin
$R^4$, $R^5$, $R^6$, R', R", R"', Y und Z die oben angegebene Bedeutung haben,
können auch erhalten werden, indem man eine Verbindung der Formel (VI)

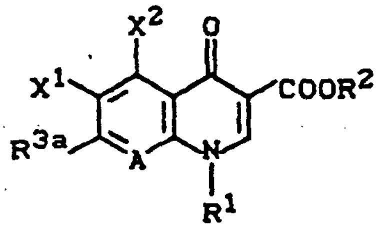

(VI),

in welcher
$X^1$, $X^2$, $R^1$, $R^2$ und A die oben angegebene Bedeutung haben und

$R^{3a}$ für einen Rest der Struktur

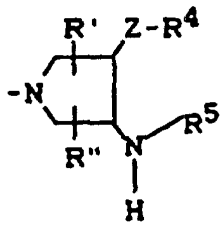

oder

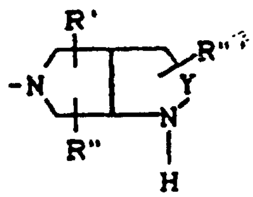

steht,
worin $R^4$, $R^5$, R', R", R"', Y und Z die oben angegebene Bedeutung haben,
mit Verbindungen der Formel (VII)

$$R^6\text{-}X^a \quad \text{(VII)},$$

in welcher

$R^6$ die oben angegebene Bedeutung hat und
$X^a$ für Chlor, Brom, Iod, Hydroxy oder Acyloxy steht,

gegebenenfalls in Gegenwart von Säurefängern umsetzt (Methode C).

Verwendet man beispielsweise 1-Cyclopropyl-6,7,8-trifluor-1,4-dihydro-4-oxo-3-chinolincarbonsäure und 1-Methyl-octahydropyrrolo[3,4-b]pyridin als Ausgangsstoffe, so kann der Reaktionsverlauf durch folgendes Formelschema wiedergegeben werden:

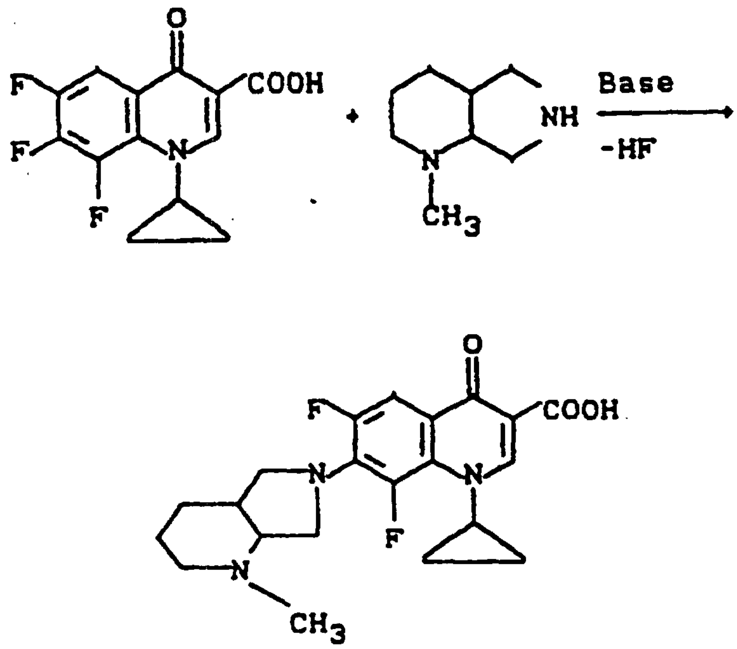


Verwendet man beispielsweise 7-Chlor-6-fluor-1-(4-fluorphenyl)-1,4-dihydro-4-oxo-1,8-naphthyridin-3-carbonsäure und cis-3-tert.-Butoxycarbonylamino-4-methoxy-pyrrolidin als Ausgangsstoffe, so kann der Reaktionsverlauf durch das folgende Formelschema wiedergegeben werden:

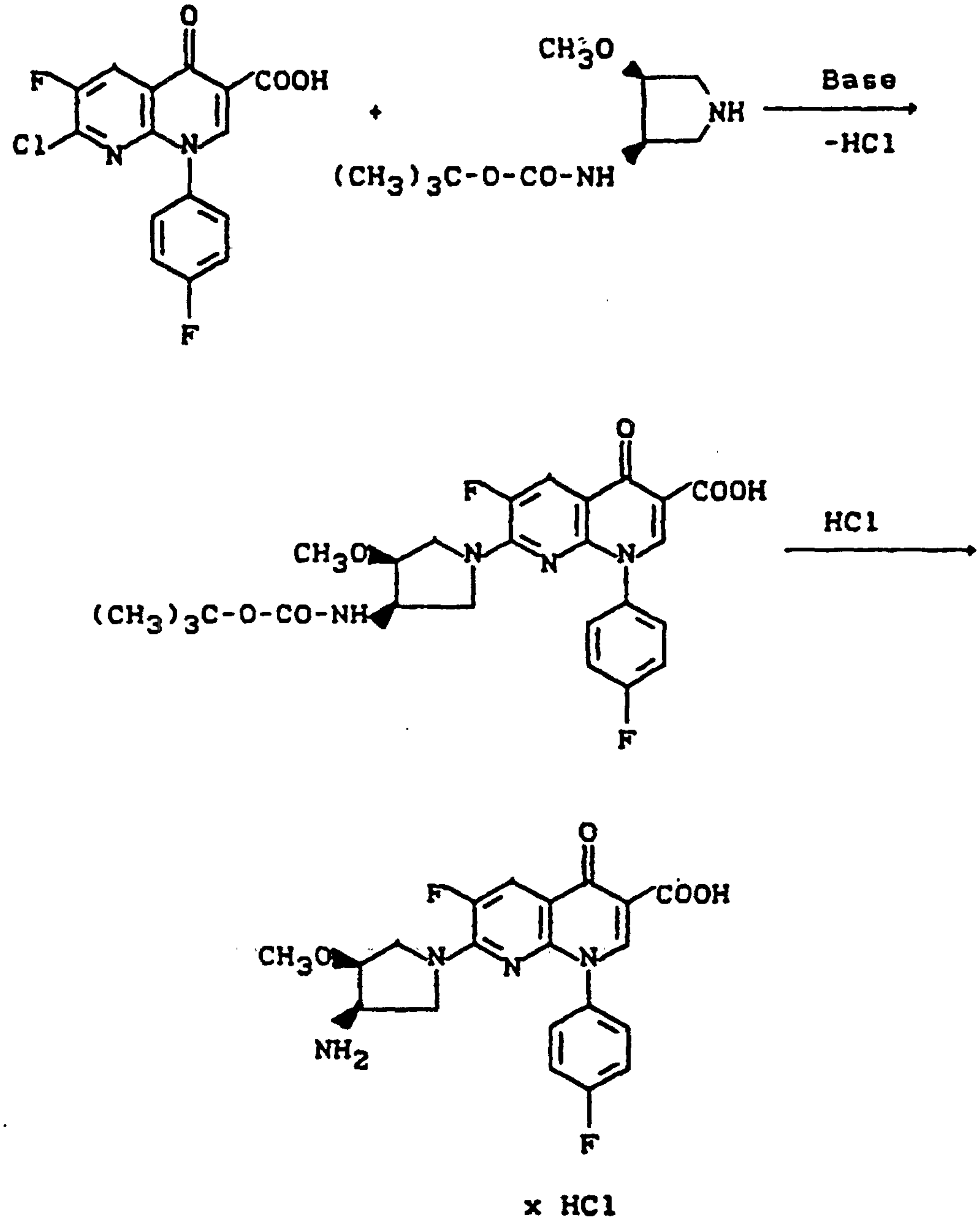


Verwendet man beispielsweise 1-Cyclopropyl-5,6,8-trifluor-1,4-dihydro-7-(2-methyl-2,7-diazabicyclo[3.3.0]oct-3-yl)-4-oxo-3-chinolincarbonsäure und Ammoniak als Ausgangsstoffe, so kann der Reaktionsablauf durch folgendes Formelschema wiedergegeben werden:

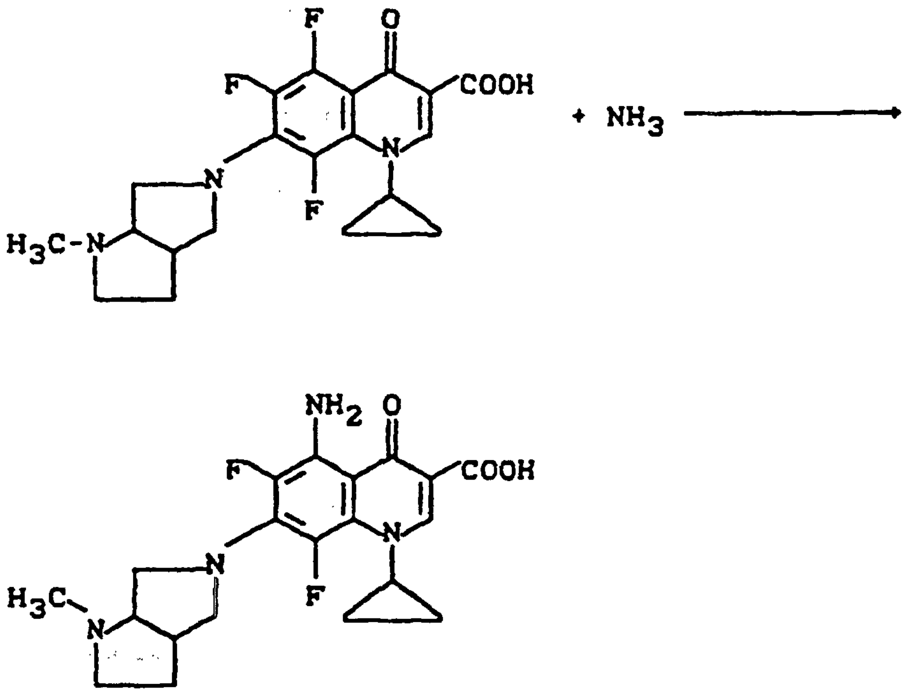


Verwendet man beispielsweise 1-Cyclopropyl-7-(2,7-diazabicyclo[3.3.0]oct-7-yl)-6-fluor-1,4-dihydro-4-oxo-3-chinolincarbonsäure und Ethanol/Chlorwasserstoff als Ausgangsstoffe, so kann der Reaktionsverlauf durch folgendes Formelschema wiedergegeben worden:

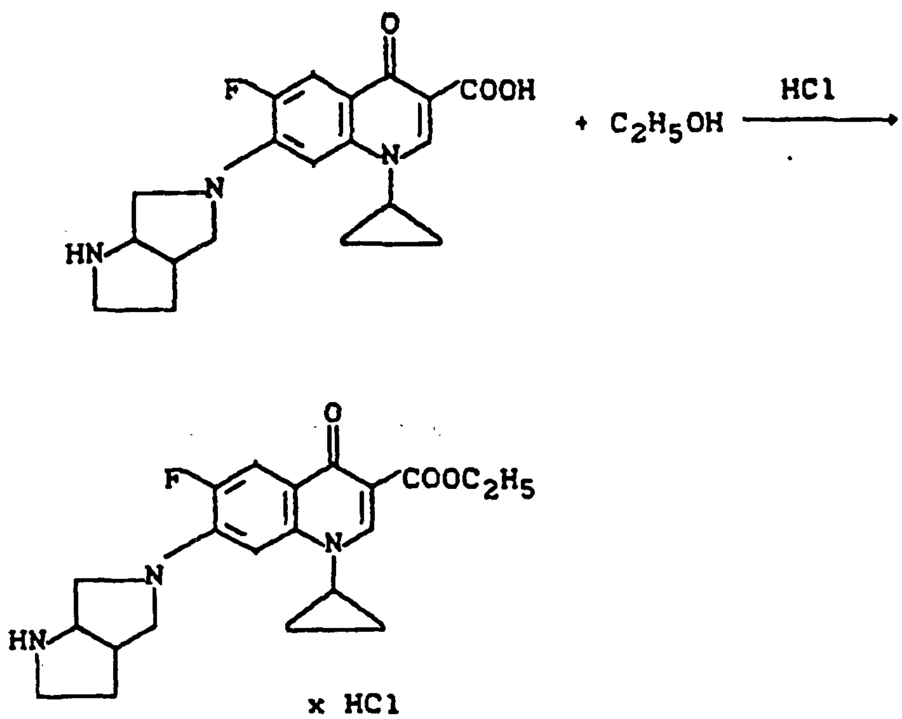


x HCl

Die als Ausgangsstoffe verwendeten Verbindungen der Formel (II) sind bekannt oder können nach bekannten

Methoden hergestellt werden. Als Beispiele seien genannt:

7-Chlor-1-cyclopropyl-6-fluor-1,4-dihydra-4-oxo-3-chinolincarbonsäure (Deutsche Patentanmeldung 3 142 854),

1-Cyclopropyl-6,7-difluor-1,4-dihydro-4-oxo-3-chinolincarbonsäure (Europäische Patentanmeldung 113 091),

6-Chlor-1-cyclopropyl-7,8-difluor-1,4-dihydro-4-oxo-3-chinolincarbonsäure (Deutsche Patentanmeldung 3 420 743),

8-Chlor-1-cyclopropyl-6,7-difluor-1,4-dihydro-4-oxo-3-chinolincarbonsäure (Deutsche Patentanmeldung 3 420 743),

1-Cyclopropyl-6,7,8-trifluor-1,4-dihydro-4-oxo-3-chinolincarbonsäure (Deutsche Patentanmeldung 3 318 145),

6,8-Dichlor-1-cyclopropyl-7-fluor-1,4-dihydro-4-oxo-3-chinolincarbonsäure (Deutsche Patentanmeldung 3 420 743),

1-Cyclopropyl-6,7-difluor-1,4-dihydro-8-methyl-4-oxo-3-chinolincarbonsäure,

1-Cyclopropyl-7-chlor-6-fluor-1,4-dihydro-8-nitro-4-oxo-3-chinolincarbonsäure,

6,7-Difluor-1-ethyl-1,4-dihydro-4-oxo-3-chinolincarbonsäure,

7-Chlor-6-fluor-1-ethyl-1,4-dihydro-4-oxo-3-chinolincarbonsäure,

7-Chlor-6-fluor-1,4-dihydro-1-(2-hydroxyethyl)-4-oxo-3-chinolincarbonsäure,

6,7-Difluor-1-(2-fluorethyl)-1,4-dihydro-4-oxo-3-chinolincarbonsäure,

8-Chlor-1-(2,4-difluorphenyl)-6,7-difluor-1,4-dihydro-4-oxo-3-chinolincarbonsäure (Europäische Patentanmeldung 235 762),

7-Chlor-6-fluor-1,4-dihydro-1-methoxy-4-oxo-3-chinolincarbonsäure,

7-Chlor-6-fluor-1,4-dihydro-1-methylamino-4-oxo-3-chinolincarbonsäure,

6,7-Difluor-1,4-dihydro-4-oxo-1-phenyl-3-chinolincarbonsäure,

7-Chlor-1-cyclopropyl-6-fluor-1,4-dihydro-4-oxo-1,8-naphthyridin-3-carbonsäure,

6,7-Dichlor-1-cyclopropyl-1,4-dihydro-4-oxo-1,8-naphthyridin-3-carbonsäure,

1-Cyclopropyl-6,7,8-trifluor-1,4-dihydro-4-oxo-3-chinolincarbonsäureethylester (Deutsche Patentanmeldung 3 318 145),

9,10-Difluor-2,3-dihydro-3-methyl-7-oxo-7H-pyrido-[1,2,3-de][1,4]benzoxacin-6-carbonsäure (Europäische Patentanmeldung 47 005),

8,9-Difluor-6,7-dihydro-5-methyl-1-oxo-1H,5H-benzo[i,j]-chinolicin-2-carbonsäure,

7-Chlar-6-fluor-1-phenyl-1,4-dihydro-4-oxo-1,8-naphthyridin-3-carbonsäure (Europäische Patentanmeldung 153 580),

7-Chlor-6-fluor-1-(4-fluorphenyl)-1,4-dihydro-4-oxo-1,8-naphthydrin-3-carbonsäure (Europäische Patentanmeldung 153 580),

6,7,8-Trifluor-1,4-dihydro-1-methylamino-4-oxo-3-chinolincarbonsäure (Deutsche Patentanmeldung 3 409 922),

1-Amino-6,7,8-trifluor-1,4-dihydro-4-oxo-3-chinolincarbonsäure (Deutsche Patentanmeldung 3 409 922),

6,7,8-Trifluor-1,4-dihydro-1-dimethylamino-4-oxo-3-chinolincarbonsäure (Deutsche Patentanmeldung 3 409 922),

7-Chlor-6-fluor-1,4-dihydro-8-nitro-4-oxo-1-phenyl-3-chinolincarbonsäure,

7-Chlor-6-fluor-1-(4-fluorphenyl)-1,4-dihydro-8-nitro-4-oxo-3-chinolincarbonsäure,

6,7-Difluor-1-(4-fluorphenyl)-1,4-dihydro-8-methyl-4-oxo-3-chinolincarbonsäure,

6-Chlor-7-fluor-1-(4-fluorphenyl)-1,4-dihydro-4-oxo-3-chinolincarbonsäure (Europäische Patentanmeldung 131 839),

5,6,7,8-Tetrafluor-1-(2,4-difluorphenyl)-1,4-dihydro-4-oxo-3-chinolincarbonsäure,

5,7-Dichlor-6-fluor-1-(2,4-difluorphenyl)-1,4-dihydro-4-oxo-3-chinolincarbansäure,

5,7-Dichlor-1-cyclopropyl-6-fluor-1,4-dihydro-4-oxo-3-chinolincarbonsäure,

6-Chlor-7-fluor-1-(2,4-difluorphenyl)-1,4-dihydro-4-oxo-3-chinolincarbonsäure (Europäische Patentanmeldung 131 839),

6,7,8-Trifluor-1-(4-fluorphenyl)-1,4-dihydro-4-oxo-3-chinolincarbonsäure (Europäische Patentanmeldung 154 780),

6,7,8-Trifluor-1-(2,4-difluorphenyl)-1,4-dihydro-4-oxo-3-chinolincarbonsäure (Europäische Patentanmeldung 154 780),

6,7,8-Trifluor-1,4-dihydro-4-oxo-1-phenyl-3-chinolincarbonsäure (Europäische Patentanmeldung 154 780),

7-Chlor-1-othyl-6-fluor-1,4-dihydro-4-oxo-1,8-naphthyridin-3-carbonsäure,

6,7-Difluor-1,4-dihydro-4-oxo-1-vinyl-3-chinolincarbonsäure,

1-Cyclopropyl-5,6,7,8-tetrafluor-1,4-dihydro-4-oxo-3-chinolincarbonsäure,

5-Amino-1-cyclopropyl-6,7,8-trifluor-1,4-dihydro-4-oxo-3-chinolincarbonsäure,

1-Cyclopropyl-6,7,8-trifluor-1,4-dihydro-5-hydroxy-4-oxo-3-chinolincarbonsäure,

1-Cyclopropyl-6,7-difluor-1,4-dihydro-8-methoxy-4-oxo-3-chinolincarbonsäure.

Die als Ausgangsbverbindungen verwendeten Verbindungen der Formel (III) sind zum Teil neu. Sie können nach folgenden Verfahren hergestellt werden.

1. Ausgehend von dem N-geschützten 3,4-Epoxypyrrolidin (1) (Deutsche Offenlegungsschrift 1 929 237, US-Patent 4 254 135), das gegebenenfalls noch eine oder zwei Methyl- oder Phenylreste tragen kann, werden die Ausgangsverbindungen der Formel (IIIa)-(IIIe) hergestellt.

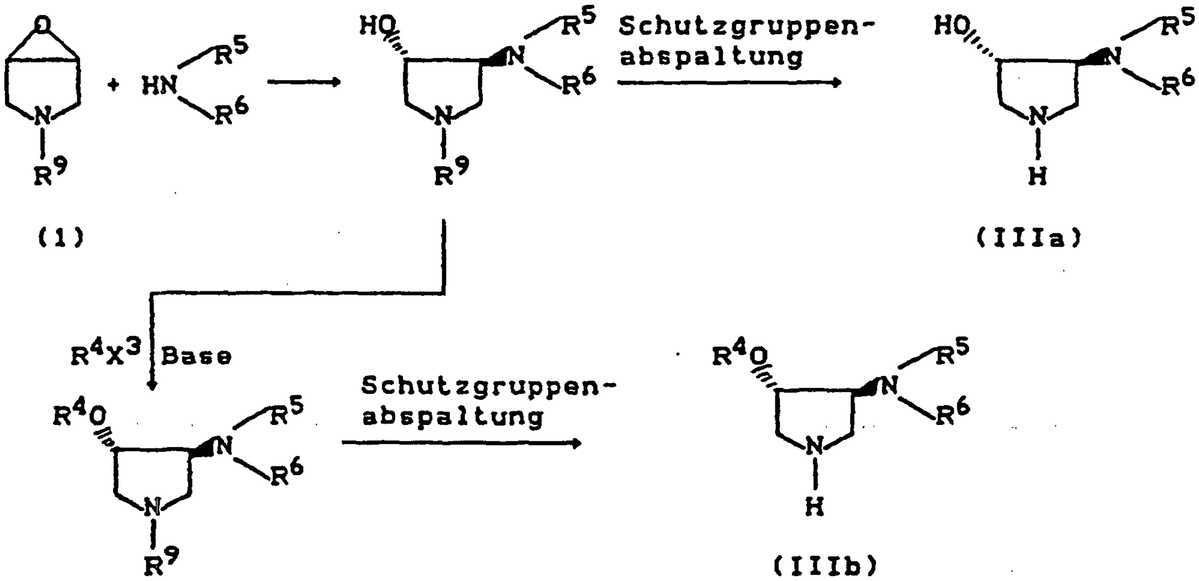


(1) (IIIa) (IIIb)

$R^9$ = Benzyl, Acyl, Alkoxycarbonyl, Benzyloxycarbonyl, Trialkylsilyl, Sulfonyl (Beispiele für Schutzgruppen),

$X^3$ = Abgangsgruppe wie Halogen, Alkyl- oder Arylsulfonyloxy

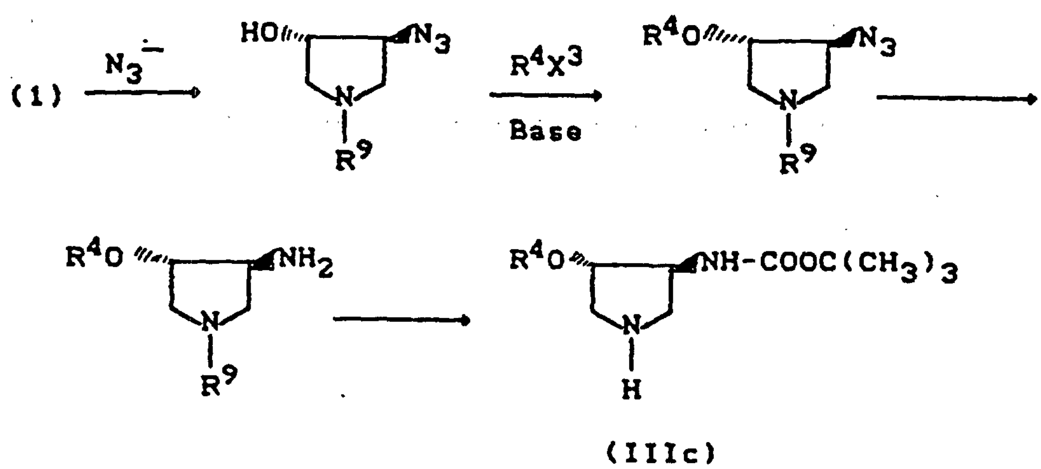


(IIIc)

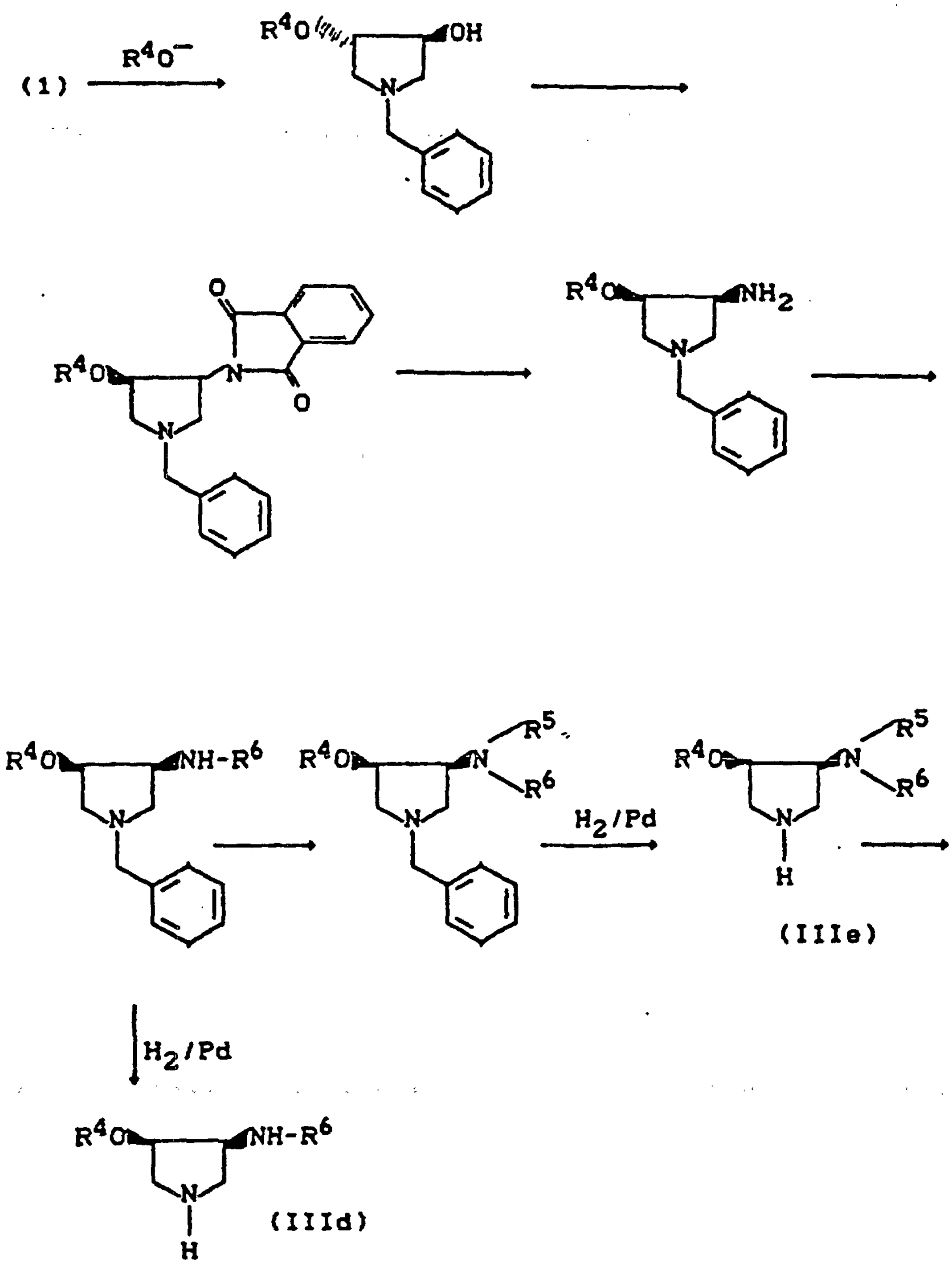


(IIIe)

(IIId)

2. Ausgangsverbindungen der Formel (IIIf) erhält man aus 2-(1,2-Dichlorethyl)-oxiran über die folgende Reaktionssequenz:

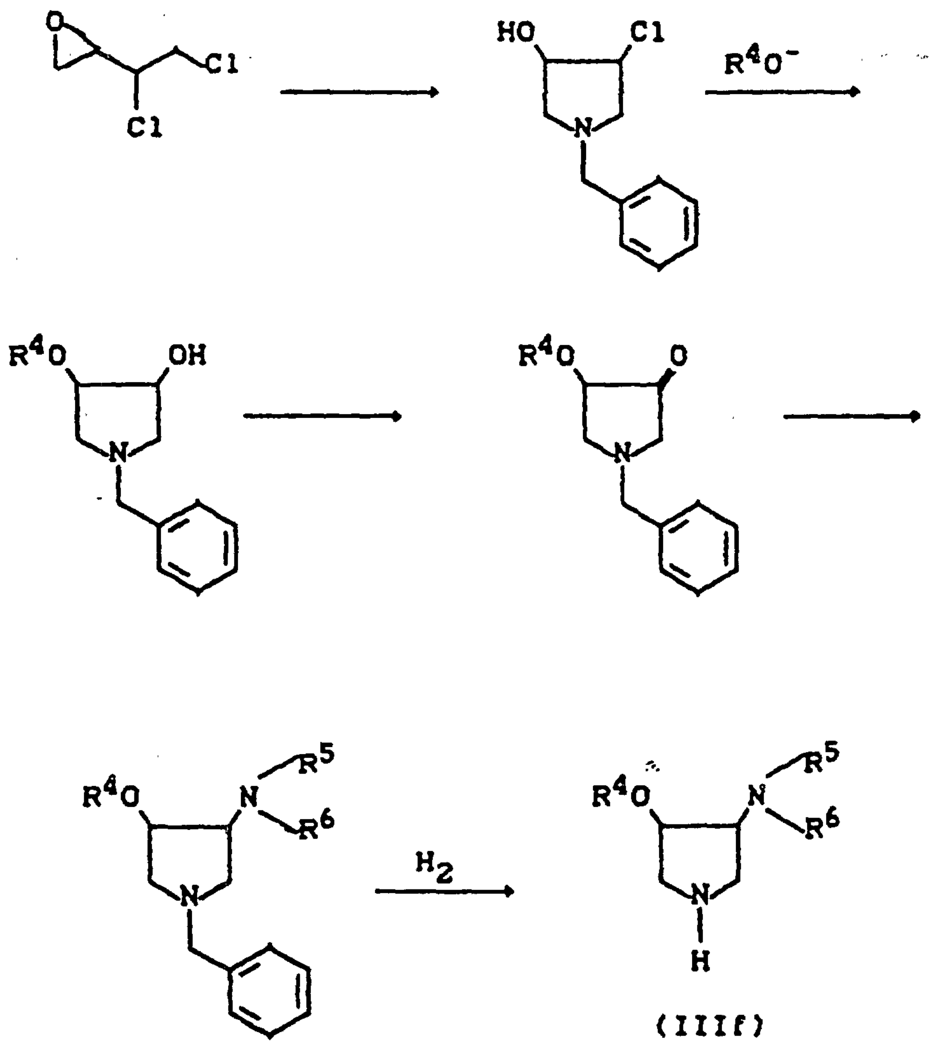


(IIIf)

3. Durch Addition von Aziden an gegebenenfalls mit einem oder zwei Methyl- beziehungsweise Phenylresten substituierte N-Benzylmaleinimide können Ausgangsverbindungen der Formel (III g) hergestellt werden:

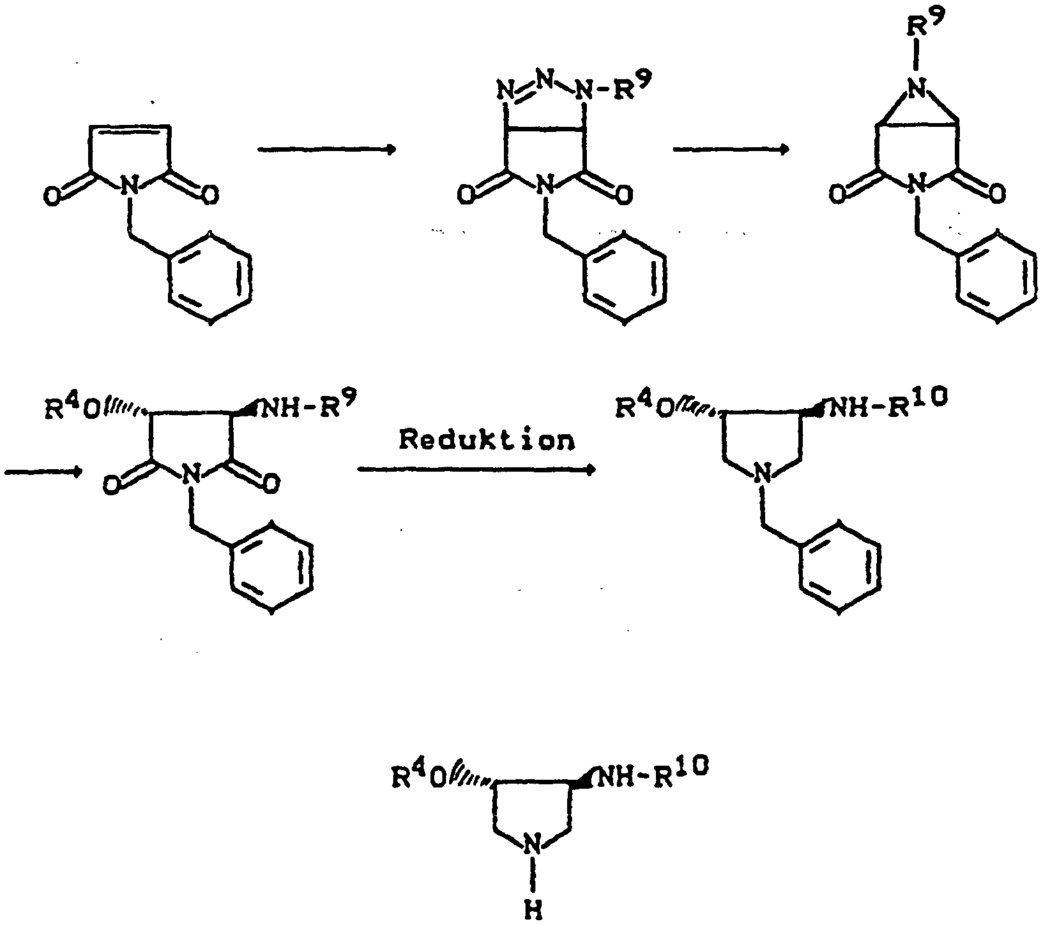


(IIIg)

$R^{10}$ = H, Alkyl, Benzyl.

4. Aus den 3,4-Epoxypyrrolidinen (1) erhält man über eine Cyclisierung mit Thionylchlorid die Ausgangsverbindungen der Formel (III h):

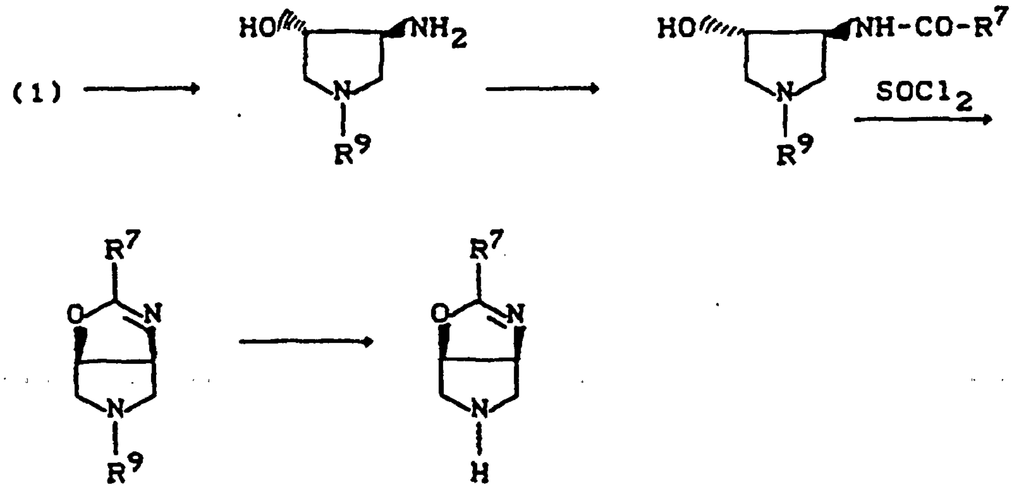


(III h)

5. Durch Umsetzen der 3,4-Epoxypyrrolidine (1) mit Ethanolaminen erhält man durch intramolekulare Veretherung die Ausgangsverbindungen der Formel (III i):

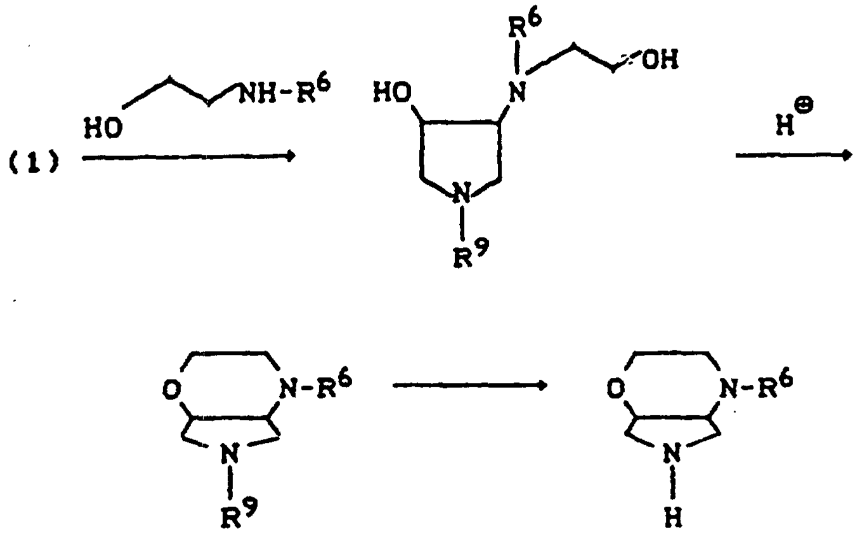


(III i).

6. Die Ausgangsverbindungen der Formel (III j) erhält man aus Aminoacetaldehyddimethylacetal über eine intramolekulare 1,3-dipolare Cycloaddition.

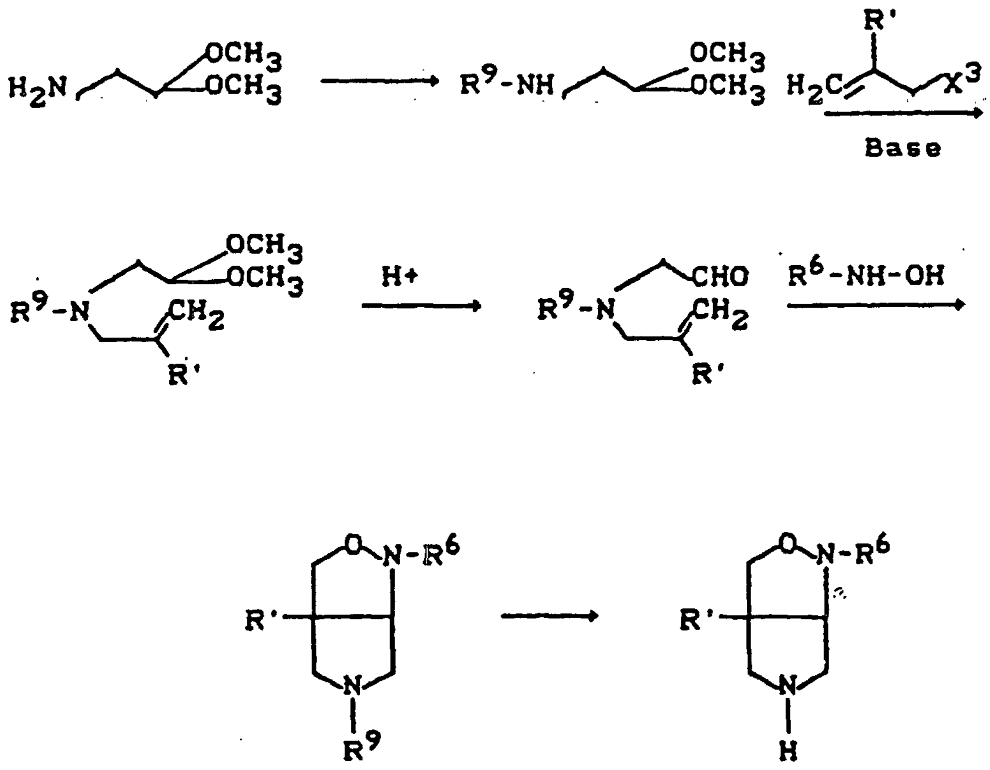


(III j)

7. Ausgehend von Pyridin-2,3-dicarbonsäure-N-benzylimid werden Ausgangsverbindungen (III k) beziehungsweise (III l) über die angegebenen Reaktionsschritte hergestellt.

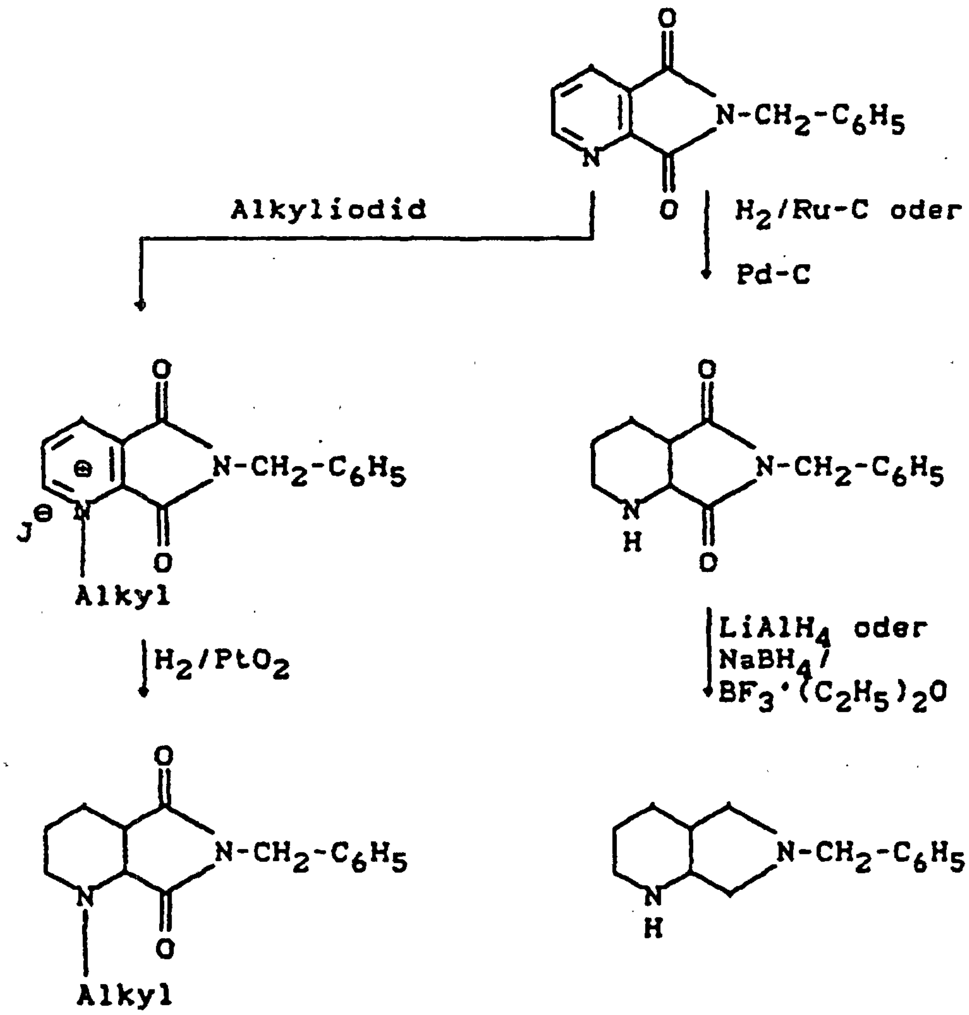

Alkyliodid
H2/Ru-C oder
Pd-C
H2/PtO2
LiAlH4 oder
NaBH4/
BF3·(C2H5)2O

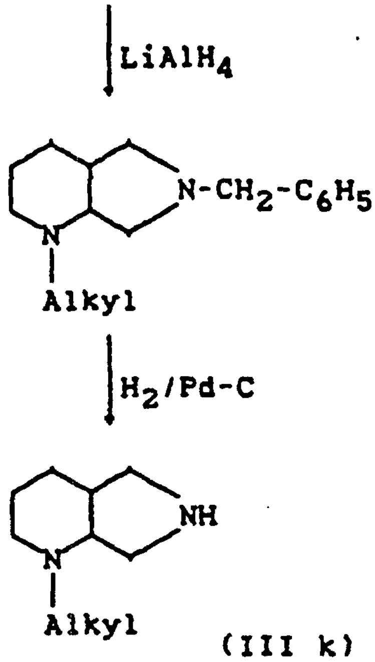


(III k)

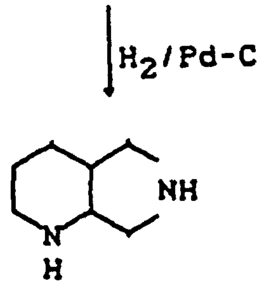


(III l)

8. N-Benzyl-maleinsäureimid addiert 2-Chlorethylamine zu den 3-(2-chlorethylamino)-succinimiden, die zu den Ausgangsverbindungen der Formel (III m) umgesetzt werden:

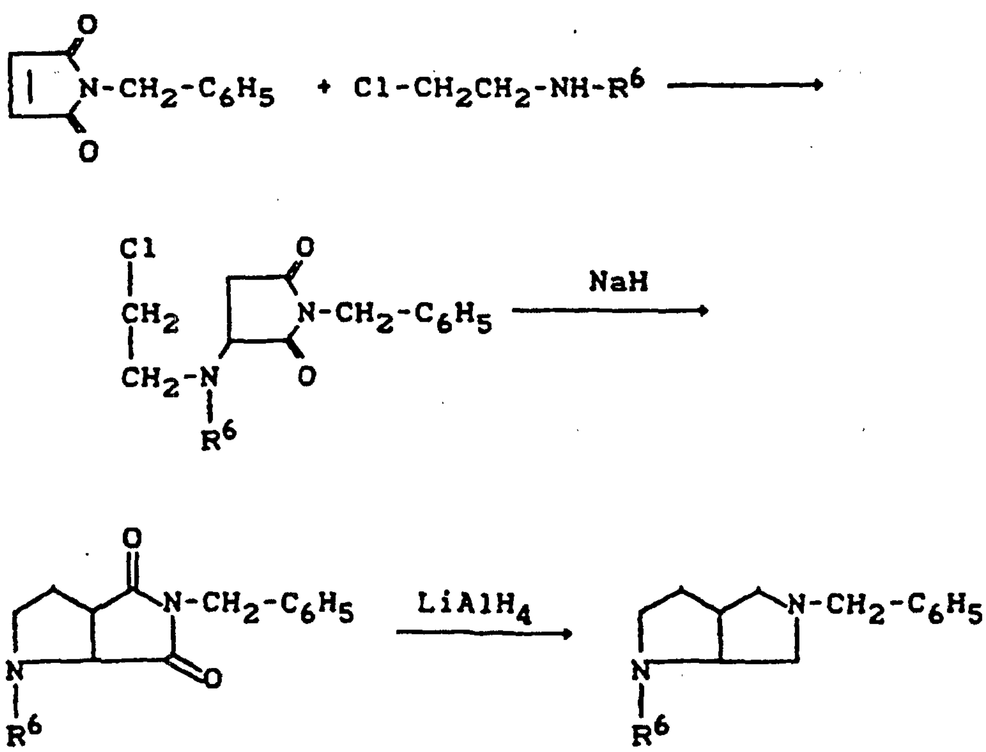

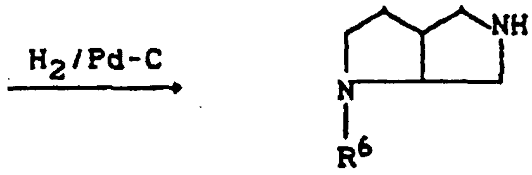


(III m)

9. 2-Methyl-2-propenal-dimethylhydrazon reagiert mit N-Benzylmaleinsäureimid zu einem Cycloaddukt, welches nach der angegebenen Reaktionsfolge in die Ausgangsverbindung (III n) überführt werden kann.

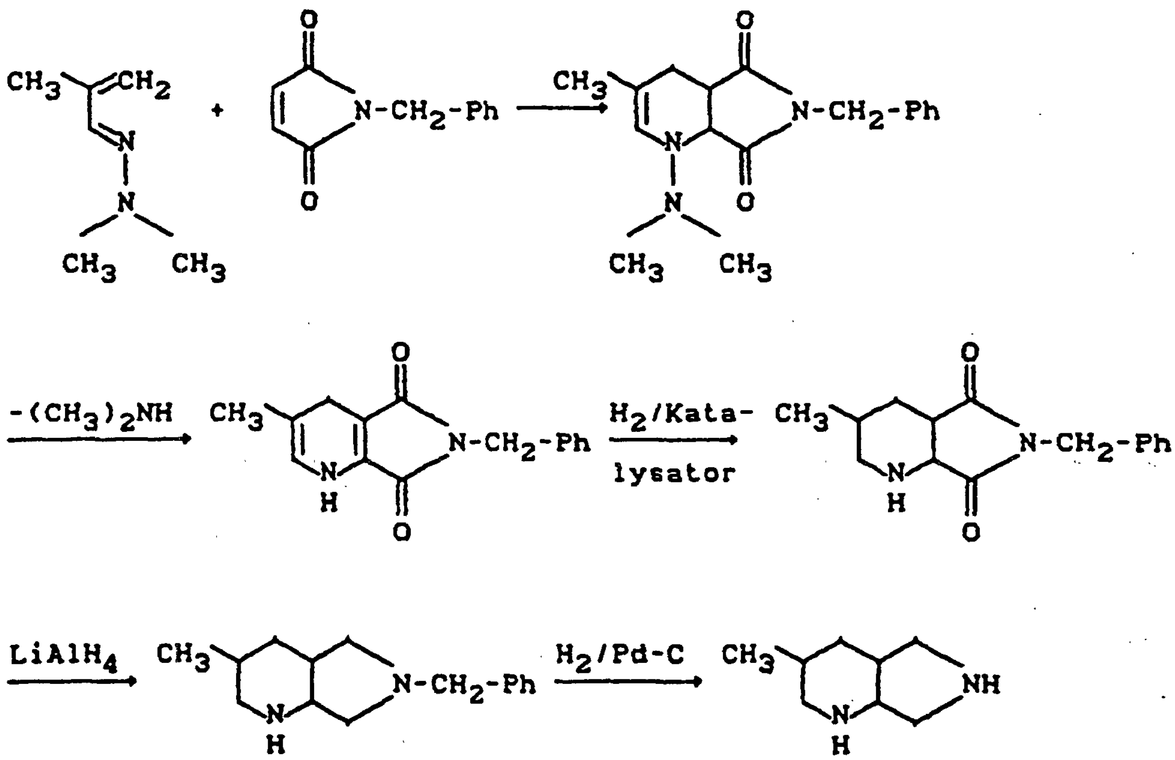


(III n)

10. Ausgangsverbindungen der Formeln (IIIo), (IIIp) oder (IIIq) können ausgehend von N-geschützten 2,5-Dihydropyrrolen (3-Pyrrolinen) durch Addition Sulfensäurechloriden auf folgenden Wege erhalten werden:

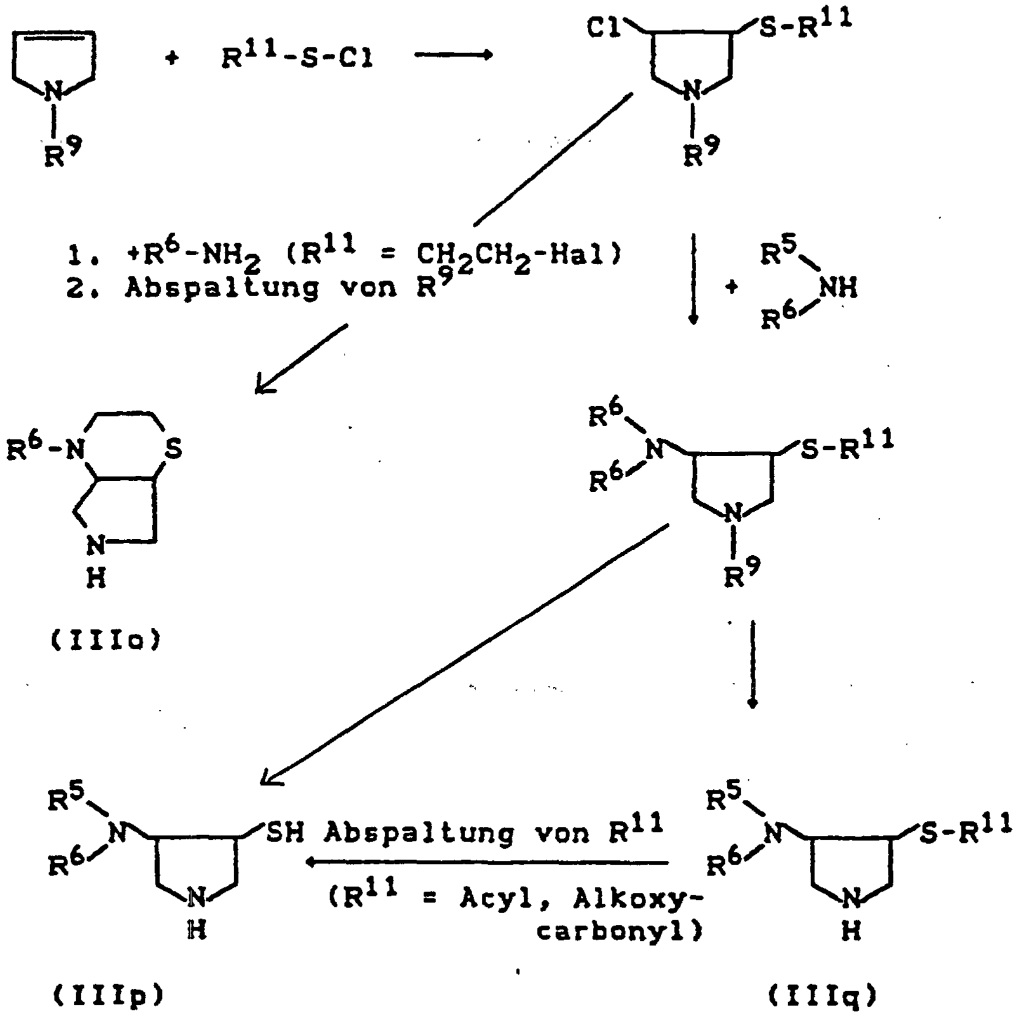


(IIIo)

(IIIp)

(IIIq)

$R^{11}$= gegebenenfalls durch Halogen substituiertes $C_1$-$C_4$-Alkyl, gegebenenfalls durch Halogen, Nitro, Alkyl und Alkoxy substituiertes Phenyl, sowie Acyl, Alkoxycarbonyl.

Nach diesem allgemeinen Formelschema lassen sich zum Beispiel die folgenden Ausgangsverbindungen herstellen. Sie können als Diastereomerengemische, in diastereomerenreiner und auch enantionmerenreiner Form hergestellt und eingesetzt werden.
4-Amino-3-hydroxypyrrolidin,
3-Hydroxy-4-methylaminopyrrolidin,
4-Dimethylamino-3-hydroxypyrrolidin,
4-Ethylamino-3-hydroxypyrrolidin,
3-Amino-4-methoxypyrrolidin,
4-Methoxy-3-methylaminopyrrolidin,
3-Dimethylamino-4-methoxypyrrolidin,
3-Ethylamino-4-methoxypyrrolidin,
3-Amino-4-ethoxypyrrolidin,
4-Ethoxy-3-methylaminopyrrolidin,
3-Dimethylamino-4-ethoxypyrrolidin,
4-Ethoxy-3-ethylaminopyrrolidin,
3-Hydroxy-4-hydroxyaminopyrrolidin,
3-Hydroxy-4-methoxyaminopyrrolidin,
3-Hydroxyamino-4-methoxypyrrolidin,
4-Methoxy-3-methoxyaminopyrrolidin,
3-Benzylamino-4-methoxypyrrolidin,

4-Methoxy-3-((5-methyl-2-oxo-1,3-dioxol-4-yl)-methylamino)-pyrrolidin,
3-Amino-4-methylmercaptopyrrolidin,
3-Acetoxy-4-dimethylaminopyrrolidin,
3-Acetamido-4-methoxypyrrolidin,
4-Methoxy-3-methoxycarbonylaminopyrrolidin, 3-Formamido-4-methoxypyrrolidin, 3-Amino-4-methoxy-2-methylpyrrolidin, 3-Amino-4-methoxy-5-methylpyrrolidin, 4-Methoxy-2-methyl-3-methylaminopyrrolidin, 4-Methoxy-5-methyl-3-methylaminopyrrolidin, 3-Amino-4-methoxy-2-phenylpyrrolidin, 4-Methoxy-3-methylamino-5-phenylpyrrolidin, 3-Methyl-2,7-diazabicyclo[3.3.0]octan, 4-Methyl-2,7-diazabicyclo[3.3.0]octan, 5-Methyl-2,7-diazabicyclo[3.3.0]octan, 3,5-Dimethyl-2,7-diazabicyclo[3.3.0]octan, 1,5-Dimethyl-2,7-diazabicyclo[3.3.0]octan, 2-Oxa-4,7-diazabicyclo[3.3.0]octan, 3,3-Dimethyl-2-oxa-4,7-diazabicyclo[3.3.0]octan, 3-Oxa-2,7-diazabicyclo[3.3.0]octan, 1,2-Dimethyl-3-oxa-2,7-diazabicyclo[3.3.0]octan, 2,5-Dimethyl-3-oxa-2,7-diazabicyclo[3.3.0]octan, 2,8-Dimethyl-3-oxa-2,7-diazabicyclo[3.3.0]octan, 5-Methyl-3-oxa-2,7-diazabicyclo[3.3.0]octan, 2-Oxa-4,7-diazabicyclo[3.3.0]oct-3-en, 3-Methyl-2-oxa-4,7-diazabicyclo[3.3.0]oct-3-en, 3-Phenyl-2-oxa-4,7-diazabicyclo[3.3.0]oct-3-en, 6-Methyl-2-oxa-4,7-diazabicyclo[3.3.0]oct-3-en, 8-Methyl-2-oxa-4,7-diazabicyclo[3.3.0]oct-3-en, 3-Methyl-2,8-diazabicyclo[4.3.0]nonan, 4-Methyl-2,8-diazabicyclo[4.3.0]nonan, 5-Methyl-2,8-diazabicyclo[4.3.0]nonan, 6-Methyl-2,8-diazabicyclo[4.3.0]nonan, 3-Methyl-2-oxa-5,8-diazabicyclo[4.3.0]nonan, 4-Methyl-2-oxa-5,8-diazabicyclo[4.3.0]nonan, 1-Methyl-2-oxa-5,8-diazebicyclo[4.3.0]nonan,
3,5-Dimethyl-2-oxa-5,8-diazabicyclo[4.3.0]nonan,
2-Thia-5,8-diazabicyclo[4.3.0]nonan,
5-Methyl-2-thia-5,8-diazabicyclo[4.3.0]nonan,
3,5-Dimethyl-2-thia-5,8-diazabicyclo[4.3.0]nonan,
3-Oxa-2,8-diazabicyclo[4.3.0]nonan,
2-Methyl-9-oxa-2,8-diazabicyclo[4.3.0]nonan,
4-Methyl-3-oxa-2,8-diazabicyclo[4.3.0]nonan,
2,5-Dimethyl-3-oxa-2,8-diazabicyclo[4.3.0]nonan,
3-Oxa-5,8-diazabicyclo[4.3.0]nonan,
5-Methyl-3-oxa-5,8-diazabicyclo[4.3.0]nonan,
1,5-Dimethyl-3-oxa-5,8-diazabicyclo[4.3.0]nonan,
4,4-Dimethyl-3-oxa-5,8-diazabicyclo[4.3.0]nonan.

Die Umsetzung von (II) mit (III) gemäß Methode A, bei der die Verbindungen (III) auch in Form ihrer Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5,4,0]undec-7-en (DBU) oder überschüssiges Amin (III).

Die Reaktionstemperaturen können in einem größeren Bereich variiert worden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol der Verbindung (III) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden (siehe J.F.W. HcOmie, Protective Groups in Organic Chemistry (1973), Seite 104).

Freie Aminofunktionen können während der Umsetzung durch eine geeignete Aminoschutzgruppe, zum Beispiel durch den Ethoxycarbonyl- oder den tert.-Butoxycarbonylrest, geschützt und nach Beendigung der Reaktion durch Behandlung mit einer geeigneten Säure wie Chlorwasserstoffsäure oder Trifluoresigsäure wieder freigesetzt werden (siehe Houben-Weyl, Methoden der oganischen Chemie, Band E4, Seite 144 (1983); J.F.W. McOmie, Protective Groups in Organic Chemistry (1973), Seite 43).

Die Umsetzung von (IV) mit (V) gemäß Methode B wird vorzugsweise in einem Verdünnungsmittel wie Dimethylsulfoxid, Dioxan, N,N-Dimethylformamid, N-Methylpyrrolidon, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders

geeignet seien im einzelnen genannt: Triethylamin, 1,4-Diazabicyclo-[2.2.2]octan (DABCO) oder 1,8-Diazabicyclo [5,4,0]undec-7-en (DBU).

Die Reaktionstemperaturen können in einem größeren Bereich variiert werden. Im allgemeinen arbeitet man zwischen etwa 70 und etwa 200°C, vorzugsweise zwischen 100 und 180°C.

Die Umsetzung kann bei Normaldruck, aber auch bei erhöhtem Druck durchgeführt werden. Im allgemeinen arbeitet man bei Drucken zwischen etwa 1 bar und etwa 100 bar, vorzugsweise zwischen 1 und 10 bar.

Bei der Durchführung des erfindungsgemäßen Verfahrens gemäß Methode B setzt man auf 1 Hol der Verbindung (IV) 1 bis 50 Mol, vorzugsweise 1 bis 30 Mol der Verbindung (V) ein.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200°C, vorzugsweise etwa 60° bis 120°C umgesetzt. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4 yl-methyl)-ester werden durch Umsetzung eines Alkalisalzes der zugrundeliegenden Carbonsäure mit 4-Brommethyl- oder 4-Chlormethyl-5-methyl-1,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0° bis 100°C, vorzugsweise 0° bis 50°C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können ebenfalls die in Tabelle 1 beispielhaft aufgeführten Verbindungen hergestellt werden, wobei diese Verbindungen sowohl als Diastereomerengemische als auch als diastereomerenreine oder enantiomerenreine Verbindungen vorliegen können.

**Tabelle 1**

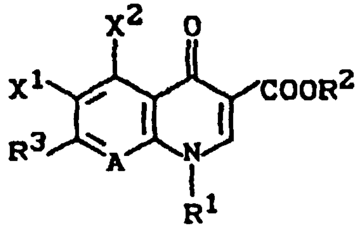

| $R^1$ | $R^2$ | $R^3$ | $X^1$ | $X^2$ | A |
|---|---|---|---|---|---|
| 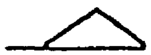 | H | 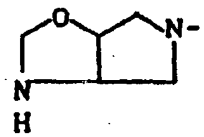 | F | H | CH |
| 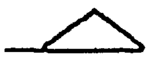 | $C_2H_5$ | 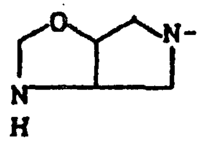 | F | H | CF |
| 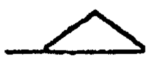 | H | 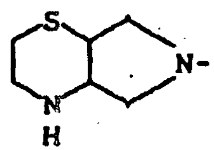 | F | H | CF |
| 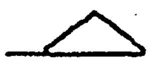 | H | 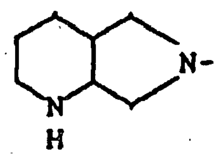 | F | H | $C-OCH_3$ |

**Tabelle 1** (Fortsetzung)

| $R^1$ | $R^2$ | $R^3$ | $X^1$ | $X^2$ | A |
|---|---|---|---|---|---|
| $C_2H_5$ | H | 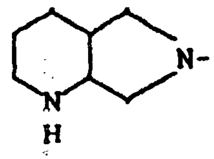 | F | H | CH |
| $C_2H_5$ | H | 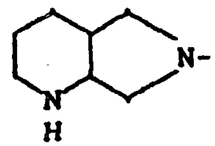 | F | $NH_2$ | CF |
| 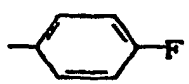 | H | 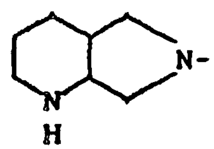 | F | H | CF |
| 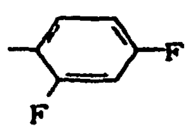 | H | 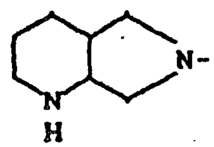 | F | H | CF |
| $-CH=CH_2$ | H | 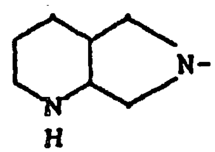 | F | H | CH |

**Tabelle 1** (Fortsetzung)

| $R^1$ | $R^2$ | $R^3$ | $X^1$ | $X^2$ | A |
|---|---|---|---|---|---|
| $HO-CH_2CH_2-$ | H | 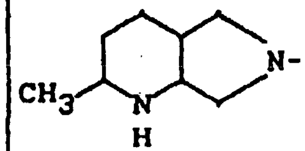 | F | H | CH |
| 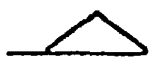 | H | 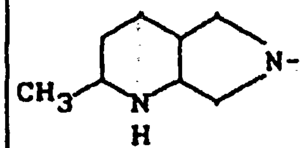 | F | F | CF |
| 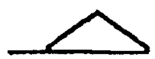 | H | 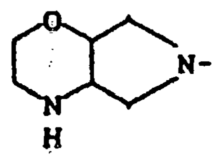 | F | F | CF |
| 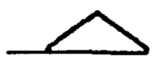 | $-C_2H_5$ | 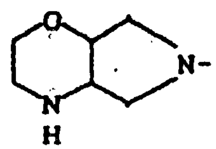 | F | H | CF |
| 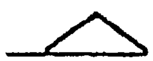 | 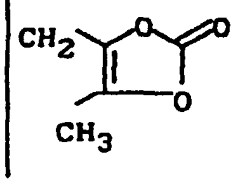 | 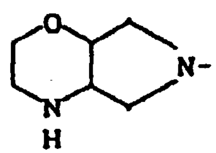 | F | H | CF |

**Tabelle 1** (Fortsetzung)

| $R^1$ | $R^2$ | $R^3$ | $X^1$ | $X^2$ | A |
|---|---|---|---|---|---|
| 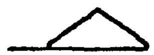 | H | 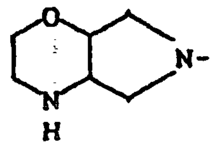 | F | $NH_2$ | CF |
| 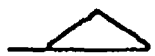 | H | 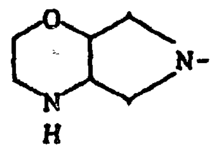 | F | OH | CF |
| 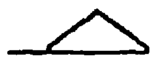 | H | 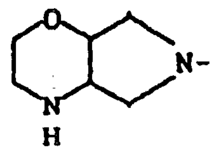 | F | H | CCl |
| 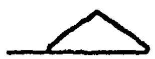 | H | 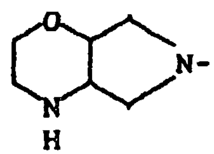 | F | H | CK |
| 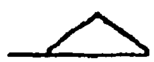 | H | 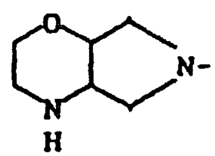 | F | H | N |

Tabelle 1 (Fortsetzung)

| $R^1$ | $R^2$ | $R^3$ | $X^1$ | $X^2$ | A |
|---|---|---|---|---|---|
| 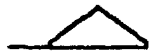 | H | 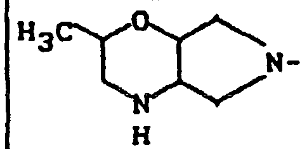 | F | H | CF |
| 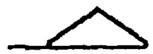 | H | 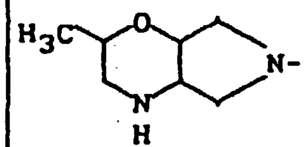 | F | F | CF |
| 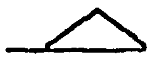 | H | 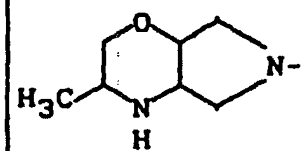 | F | H | CF |
| 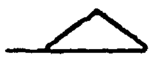 | H | 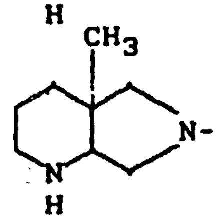 | F | F | CF |
| $F-CH_2CH_2$ | H | 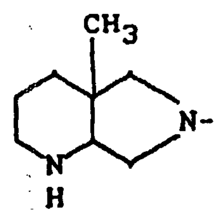 | F | H | CH |

**Tabelle 1** (Fortsetzung)

| $R^1$ | $R^2$ | $R^3$ | $X^1$ | $X^2$ | A |
|---|---|---|---|---|---|
| 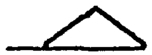 | H | 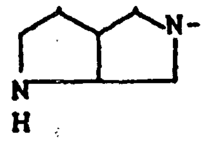 | F | $NH_2$ | CF |
| 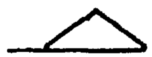 | H | 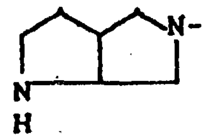 | F | H | N |
| 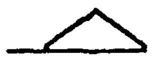 | H | 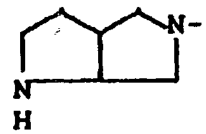 | F | H | CCl |
| $CH_3O$ | H | 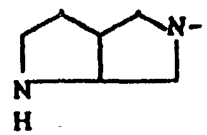 | F | H | CH |
| $CH_3-NH-$ | H | 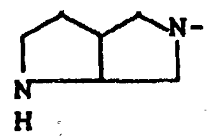 | F | H | CF |

**Tabelle 1** (Fortsetzung)

| $R^1$ | $R^2$ | $R^3$ | $X^1$ | $X^2$ | A |
|---|---|---|---|---|---|
| 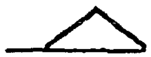 | H | 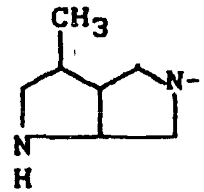 | F | H | CF |
| 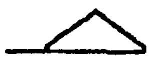 | H | 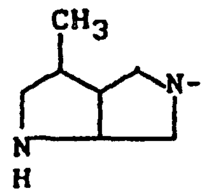 | F | H | CCl |
| 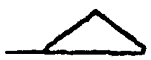 | H | 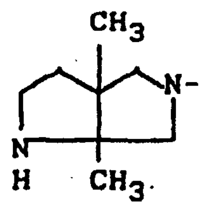 | F | H | CF |
| 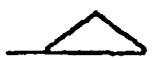 | H | 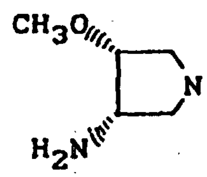 | F | H | $C-CH_3$ |

Table 1 (Fortsetzung)

| $R^1$ | $R^2$ | $R^3$ | $X^1$ | $X^2$ | A |
|---|---|---|---|---|---|
| 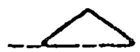 | H | 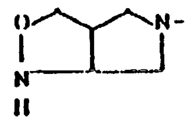 | F | H | CF |
| 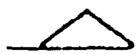 | H | 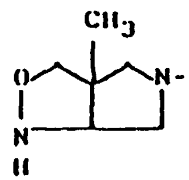 | F | H | CH |
| 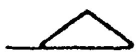 | H | 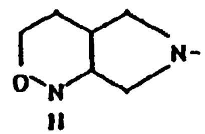 | F | H | CF |
| 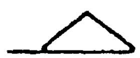 | H | 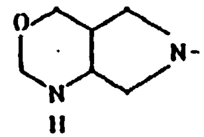 | F | H | CH |
| 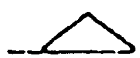 | H | 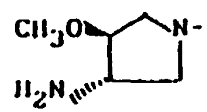 | F | H | CCl |
| 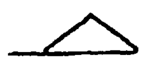 | H | 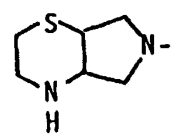 | F | H | Cl |

Tabelle 1 (Fortsetzung)

| $R^1$ | $R^2$ | $R^3$ | $X^1$ | $X^2$ | A |
|---|---|---|---|---|---|
| 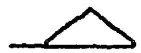 | H | 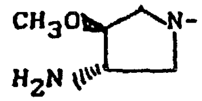 | F | H | N |
| 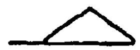 | H | 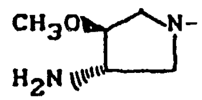 | F | $NH_2$ | CF |
| $-C_2H_5$ | H | 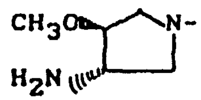 | F | H | CF |
| 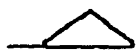 | $C_2H_5$ | 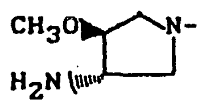 | F | H | CF |
| 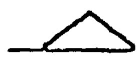 | H | 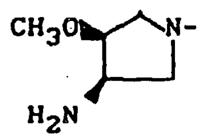 | F | H | N |
| 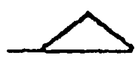 | H | 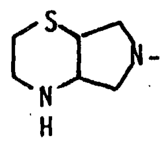 | F | H | CH |

Tabelle 1 (Fortsetzung)

| $R^1$ | $R^2$ | $R^3$ | $X^1$ | $X^2$ | A |
|---|---|---|---|---|---|
| 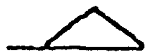 | $CH_3$ | 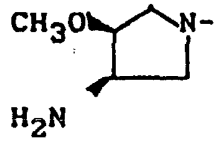 | F | H | CF |
| $-C_2H_5$ | H | 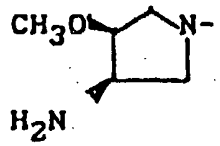 | F | H | N |
| 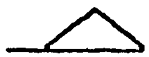 | H | 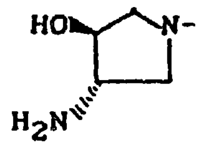 | F | H | CF |
| 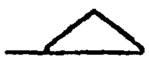 | H | 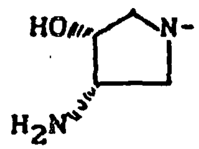 | F | H | CF |
| 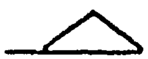 | H | 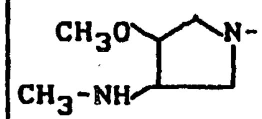 | F | H | CH |
| 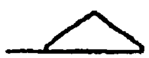 | H | 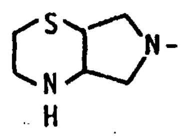 | F | H | N |

<u>Tabelle 1</u> (Fortsetzung)

| $R^1$ | $R^2$ | $R^3$ | $X^1$ | $X^2$ | A |
|---|---|---|---|---|---|
| 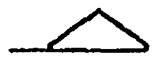 | H | 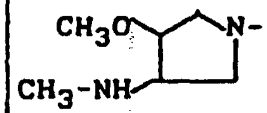 | F | H | $C-CH_3$ |
| 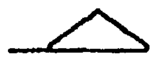 | H | 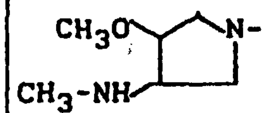 | F | H | CCl |
| 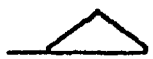 | H | 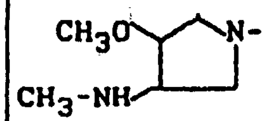 | F | H | N |
| 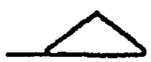 | H | 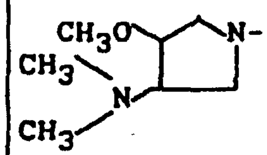 | F | H | CF |
| 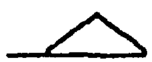 | H | 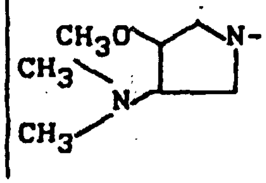 | F | $NH_2$ | CF |

**Tabelle 1 (Fortsetzung)**

| $R^1$ | $R^2$ | $R^3$ | $X^1$ | $X^2$ | A |
|---|---|---|---|---|---|
| $-C_2H_5$ | H | 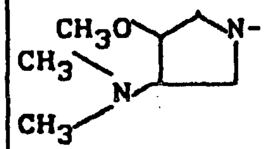 | F | H | CH |
| 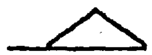 | H | 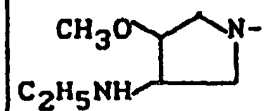 | F | H | CF |
| 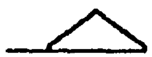 | H | 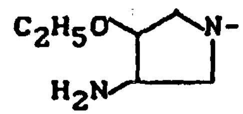 | F | H | CF |
| 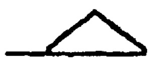 | H | 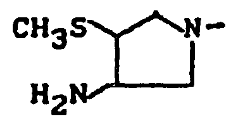 | F | H | CF |
| $-C_2H_5$ | H | 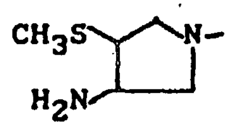 | F | H | CH |

<u>Tabelle 1</u> (Fortsetzung)

| $R^1$ | $R^2$ | $R^3$ | $X^1$ | $X^2$ | A |
|---|---|---|---|---|---|
| 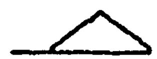 | H | 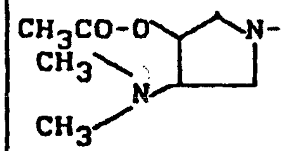 | F | H | CH |
| 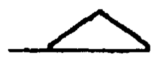 | H | 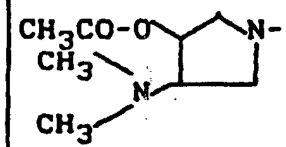 | F | H | CF |
| 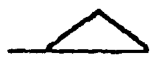 | H | 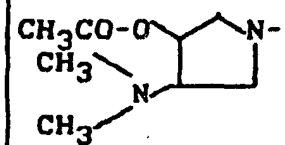 | F | H | CCl |
| 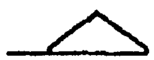 | H | 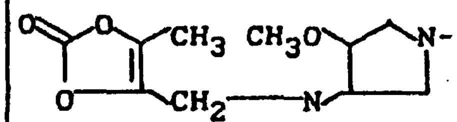 | F | H | CH |
| 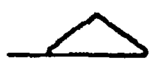 | H | 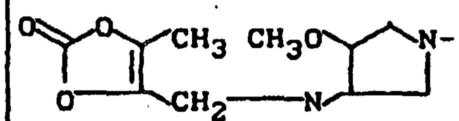 | F | H | CF |
| 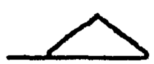 | H | 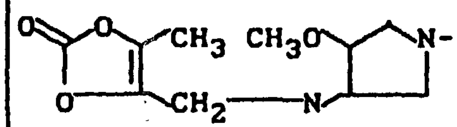 | F | H | CCl |

<u>Tabelle 1</u> (Fortsetzung)

| $R^1$ | $R^2$ | $R^3$ | $X^1$ | $X^2$ | A |
|---|---|---|---|---|---|
| 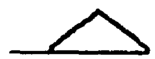 | H | 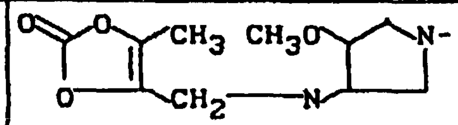 | F | H | N |
| 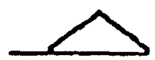 | H | 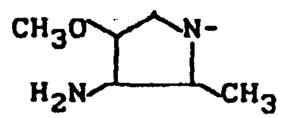 | F | H | CF |
| 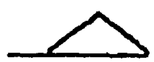 | H | 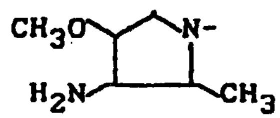 | F | H | CCl |
| 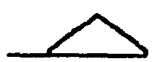 | H | 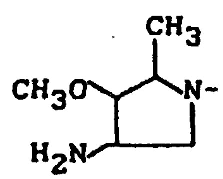 | F | H | CF |
| 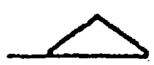 | H | 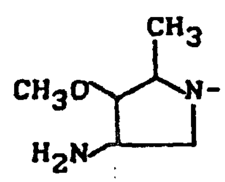 | F | H | CH |

**Tabelle 1** (Fortsetzung)

| $R^1$ | $R^2$ | $R^3$ | $X^1$ | $X^2$ | A |
|---|---|---|---|---|---|
| 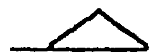 | H | 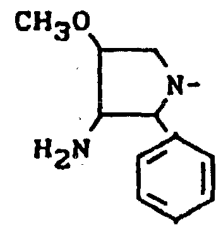 | F | H | CF |
| 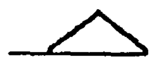 | H | 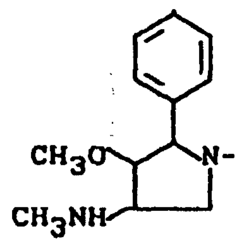 | F | H | CF |
| 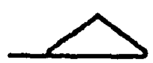 | H | 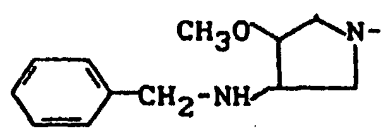 | F | H | CF |
| 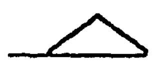 | H | 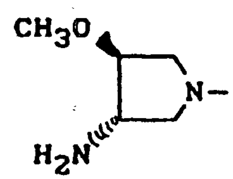 | F | H | $C-CH_3$ |

**Tabelle 1** (Fortsetzung)

| $R^1$ | $R^2$ | $R^3$ | $X^1$ | $X^2$ | A |
|---|---|---|---|---|---|
| 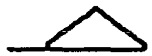 | H | 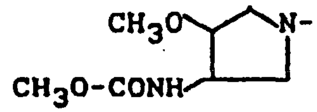 | F | H | CF |
| 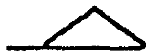 | H | 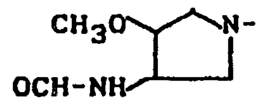 | F | H | CF |
| 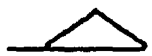 | H | 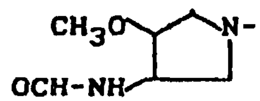 | F | H | CCl |
| 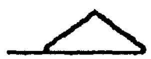 | H | 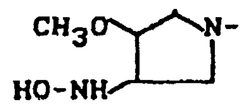 | F | H | CF |
| 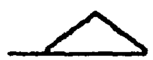 | H | 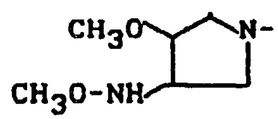 | F | H | CF |

Tabelle 1 (Fortsetzung)

| $R^1$ | $R^2$ | $R^3$ | $X^1$ | $X^2$ | A |
|---|---|---|---|---|---|
| 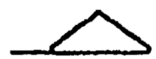 | H | 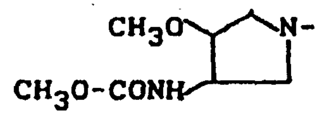 | F | H | CCl |
| 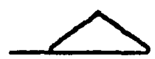 | H | 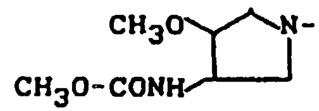 | F | H | CH |
| 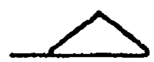 | H | 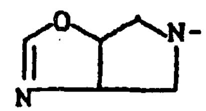 | F | H | CH |
| 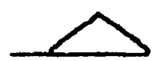 | H | 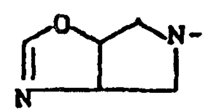 | F | H | C[illegible] |
| 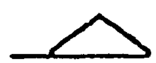 | H | 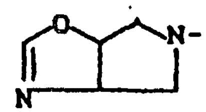 | F | H | CCl |

**Tabelle 1** (Fortsetzung)

| $R^1$ | $R^2$ | $R^3$ | $X^1$ | $X^2$ | A |
|---|---|---|---|---|---|
| 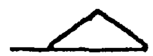 | H | 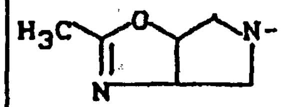 | F | H | CF |
| 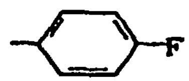 | H | 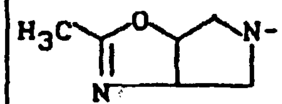 | F | H | CF |
| 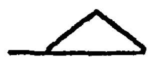 | H | 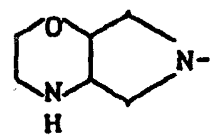 | F | H | $C-CH_3$ |
| 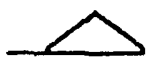 | H | 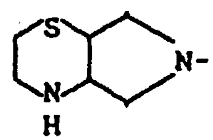 | F | H | $C-CH_3$ |
| 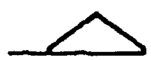 | H | 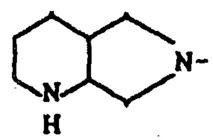 | F | H | $C-CH_3$ |

**Tabelle 1** (Fortsetzung)

| $R^1$ | $R^2$ | $R^3$ | $X^1$ | $X^2$ | A |
|---|---|---|---|---|---|
| 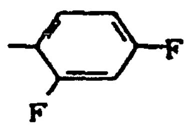 | H | 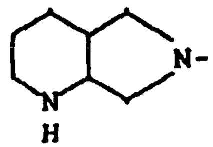 | F | H | N |
| 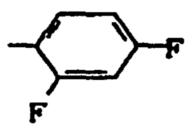 | H | 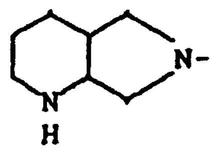 | F | H | CH |
| 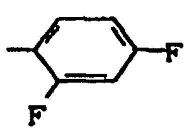 | H | 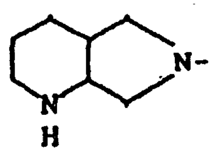 | F | H | CCl |
| 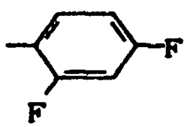 | $C_2H_5$ | 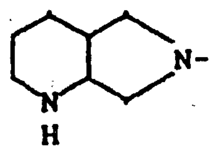 | F | F | CF |
| 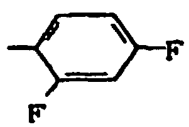 | H | 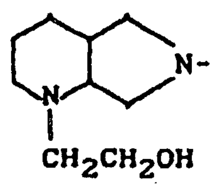 | F | $NH_2$ | CF |

**Tabelle 1** (Fortsetzung)

| $R^1$ | $R^2$ | $R^3$ | $X^1$ | $X^2$ | A |
|---|---|---|---|---|---|
| 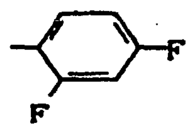 | H | 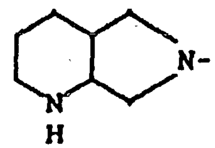 | F | Cl | CH |
| 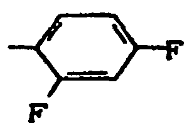 | H | 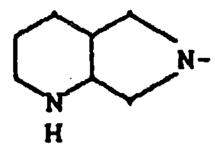 | F | Cl | CF |
| 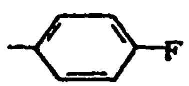 | H | 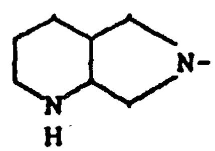 | F | H | N |
| 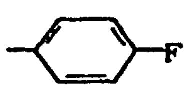 | H | 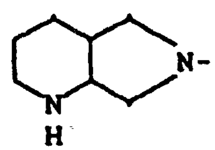 | F | H | CF |
| 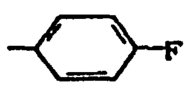 | H | 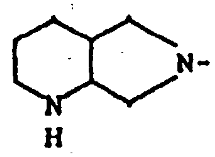 | F | H | CH |

**Tabelle 1 (Fortsetzung)**

| $R^1$ | $R^2$ | $R^3$ | $X^1$ | $X^2$ | A |
|---|---|---|---|---|---|
| 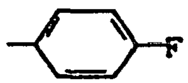 | H | 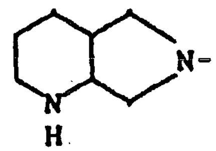 | F | H | CCl |
| 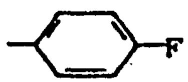 | H | 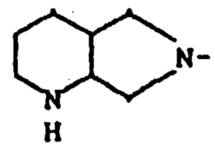 | F | F | CF |
| 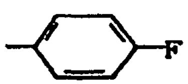 | H | 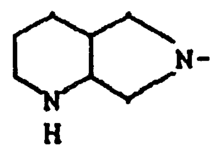 | F | $NH_2$ | CF |
| 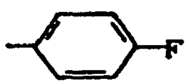 | H | 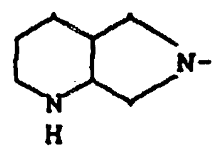 | F | Cl | CH |
| 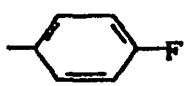 | H | 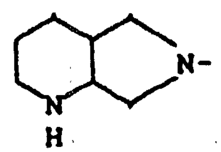 | F | Cl | CF |

**Tabelle 1 (Fortsetzung)**

| $R^1$ | $R^2$ | $R^3$ | $X^1$ | $X^2$ | A |
|---|---|---|---|---|---|
| 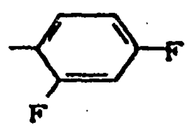 | H | 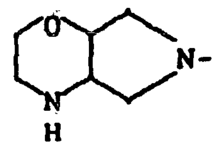 | F | H | N |
| 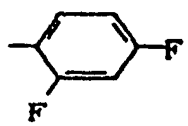 | H | 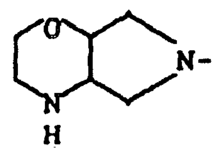 | F | N | CH |
| 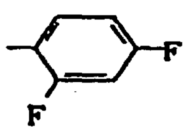 | H | 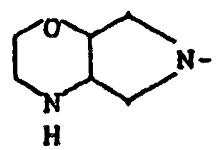 | F | H | CCl |
| 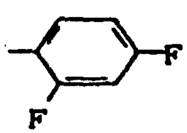 | H | 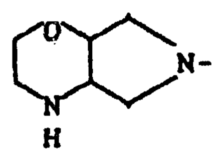 | F | F | CF |
| 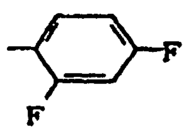 | H | 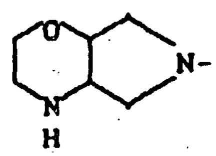 | F | $NH_2$ | CF |

**Tabelle 1** (Fortsetzung)

| $R^1$ | $R^2$ | $R^3$ | $X^1$ | $X^2$ | A |
|---|---|---|---|---|---|
| 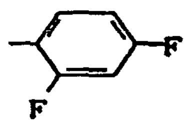 | H | 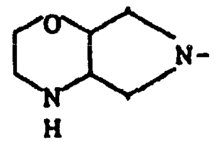 | F | Cl | CH |
| 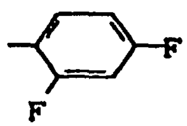 | H | 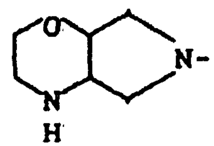 | F | Cl | CF |
| 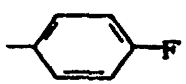 | H | 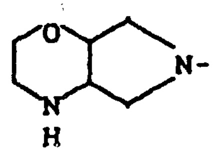 | F | H | N |
| 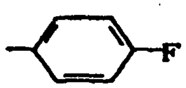 | H | 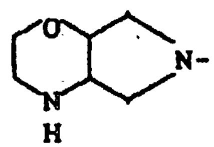 | F | H | CF |
| 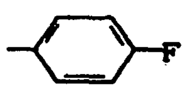 | H | 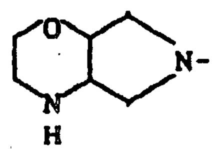 | F | H | CH |

**Tabelle 1** (Fortsetzung)

| $R^1$ | $R^2$ | $R^3$ | $X^1$ | $X^2$ | A |
|---|---|---|---|---|---|
| 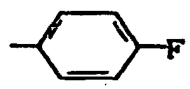 | H | 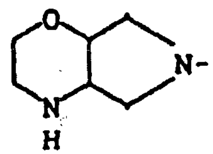 | F | H | CCl |
| 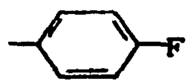 | H | 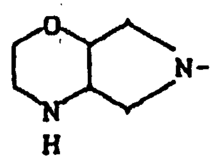 | F | F | CF |
| 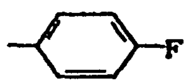 | H | 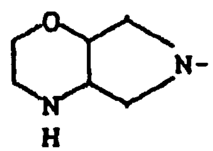 | F | $NH_2$ | CF |
| 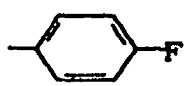 | H | 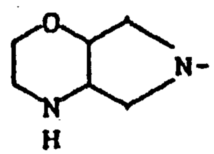 | F | Cl | CH |
| 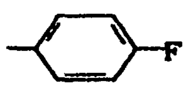 | H | 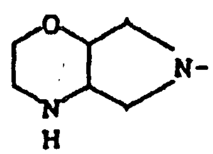 | F | Cl | CF |

**Tabelle 1** (Fortsetzung)

| $R^1$ | $R^2$ | $R^3$ | $X^1$ | $X^2$ | A |
|---|---|---|---|---|---|
| 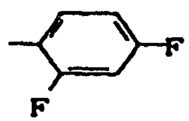 | H | 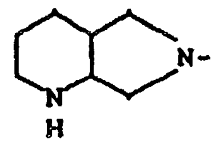 | F | H | CF |
| 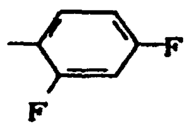 | H | 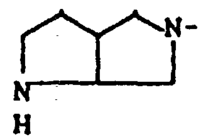 | F | H | CH |
| 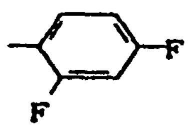 | H | 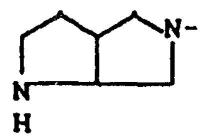 | F | H | CCl |
| 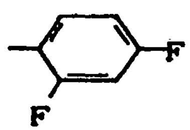 | H | 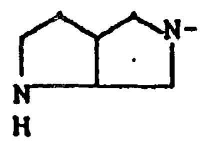 | F | F | CF |
| 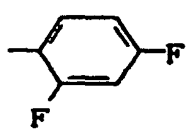 | H | 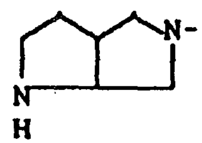 | F | $NH_2$ | CF |

**Tabelle 1** (Fortsetzung)

| $R^1$ | $R^2$ | $R^3$ | $X^1$ | $X^2$ | A |
|---|---|---|---|---|---|
| 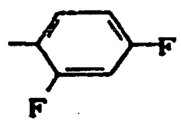 | H | 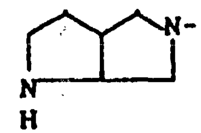 | F | Cl | Cl |
| 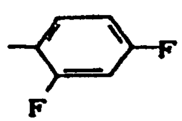 | H | 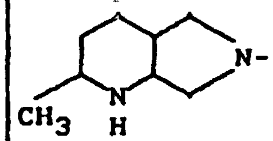 | F | H | CF |
| 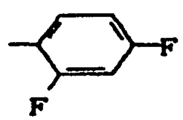 | H | 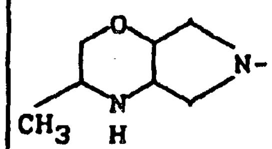 | F | H | N |
| 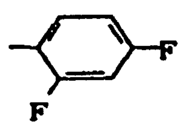 | H | 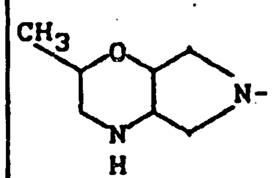 | F | H | N |
| 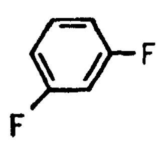 | H | 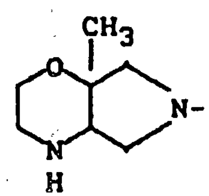 | F | H | N. |

**Tabelle 1** (Fortsetzung)

| $R^1$ | $R^2$ | $R^3$ | $X^1$ | $X^2$ | A |
|---|---|---|---|---|---|
| 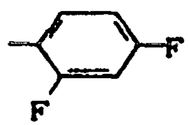 | H | 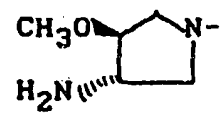 | F | H | N |
| 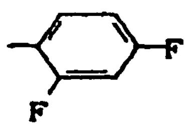 | H | 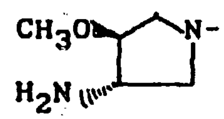 | F | H | CH |
| 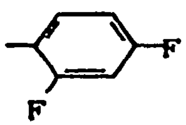 | H | 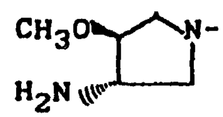 | F | H | CCl |
| 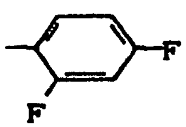 | H | 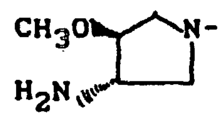 | F | F | CF |
| 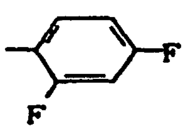 | H | 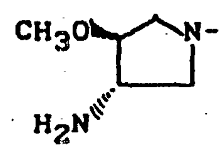 | F | $NH_2$ | CF |

**Tabelle 1** (Fortsetzung)

| $R^1$ | $R^2$ | $R^3$ | $X^1$ | $X^2$ | A |
|---|---|---|---|---|---|
| 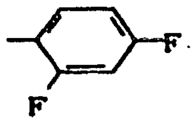 | H | 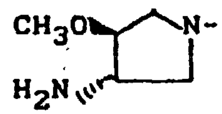 | F | Cl | CH |
| 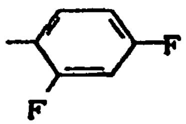 | H | 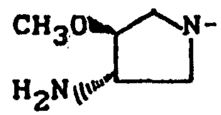 | F | Cl | CF |
| 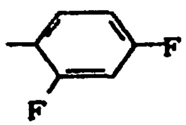 | H | 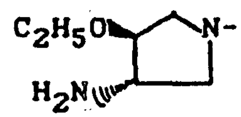 | F | H | N |
| 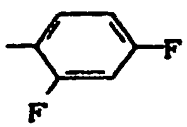 | H | 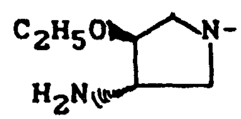 | F | H | CF |
| 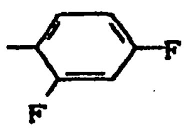 | H | 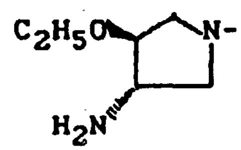 | F | H | CH |

**Tabelle 1** (Fortsetzung)

| $R^1$ | $R^2$ | $R^3$ | $X^1$ | $X^2$ | A |
|---|---|---|---|---|---|
| 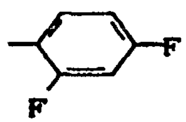 | H | 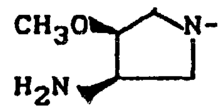 | F | H | N |
| 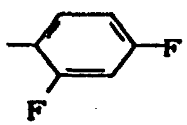 | H | 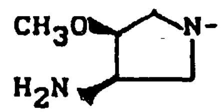 | F | H | CH |
| 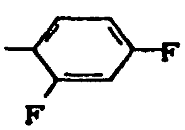 | H | 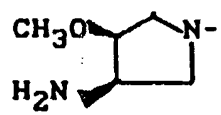 | F | H | CCl |
| 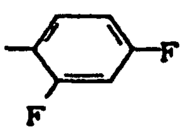 | H | 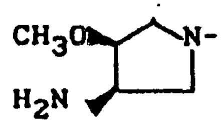 | F | F | CF |
| 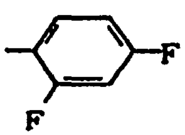 | H | 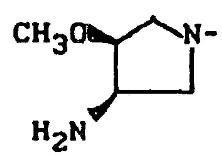 | F | $NH_2$ | CF |

**Tabelle 1** (Fortsetzung)

| $R^1$ | $R^2$ | $R^3$ | $X^1$ | $X^2$ | A |
|---|---|---|---|---|---|
| 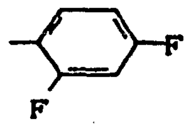 | H | 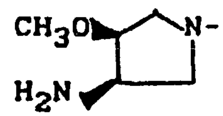 | F | Cl | CH |
| 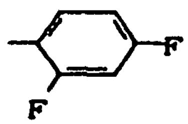 | H | 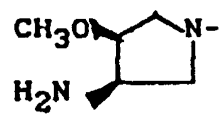 | F | Cl | CF |
| 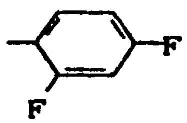 | H | 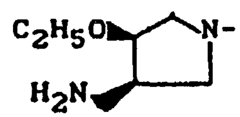 | F | H | N |
| 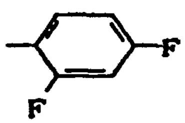 | H | 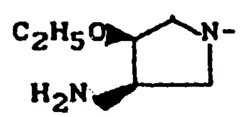 | F | H | CF |
| 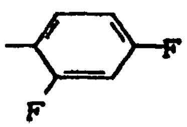 | H | 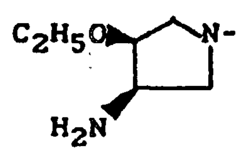 | F | H | CH |

| Beispiel für eine erfindungsgemäße Tablette | |
|---|---|
| Jede Tablette enthält: | |
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |
| Die Lackhülle enthält: | |
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob, freundii, Citrob. divernis), Salmonella und Shigella; ferner Klebsiellen (Klebs. pneumaniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Hischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis,

Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden,
wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkchol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcaronat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet worden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Ubernachtkulturen der Erreger verwandt, die zuvor so verdünnt wurden, daß jeder Impfpunkt ca. $10^4$ koloniebildende Partikel enthielt. Die beimpften Agarplatten wurden bei 37° C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Keimwachstum zu erkennen war.

In der nachstehenden Tabelle sind die MHK-Warte einiger der erfindungsgemäßen Verbindungen im Vergleich zu Ciprofloxacin angegeben.

MHK-Werte (mg/l) bestimmt durch Agar-Verdünnungstest (Denley Multipoint Inoculator; Iso-Sensitest-Agar)

| Teststamm | Beispiel 1 | 2 | 3 | 4 | 5 | 8 | 9 | 10 |
|---|---|---|---|---|---|---|---|---|
| Escherichia coli Neumann | ≤0,015 | ≤0,015 | ≤0,015 | ≤0,015 | ≤0,015 | ≤0,015 | 0,25 | 0,125 |
| Proteus mirabilis 8223 | 1 | 4 | 1 | 0,5 | 2 | 2 | 8 | 16 |
| Proteus vulgaris 1017 | ≤0,015 | 0,125 | ≤0,015 | ≤0,015 | 0,03 | 0,06 | 0,5 | 1 |
| Morganella morganii 932 | ≤0,015 | 0,03 | 0,03 | ≤0,015 | ≤0,015 | 0,06 | 0,5 | 0,5 |
| Providencia-stuartei 12052 | 1 | 4 | 2 | 0,5 | 4 | 4 | 32 | 64 |
| Staphylococcus aureus | | | | | | | | |
| FK 422 | 0,06 | 0,125 | 0,06 | ≤0,015 | 0,125 | 0,03 | 0,06 | 0,125 |
| 1756 | 0,06 | 0,125 | 0,06 | ≤0,015 | 0,125 | 0,03 | 0,06 | 0,125 |
| 133 | 0,06 | 0,125 | 0,03 | ≤0,015 | 0,125 | 0,03 | 0,06 | 0,125 |
| Enterococcus faecalis 27101 | 0,125 | - | 0,125 | 0,06 | 0,25 | 0,125 | 0,25 | 2 |
| 9790 | 0,125 | 0,5 | 0,25 | 0,06 | 0,25 | 0,125 | 0,25 | 2 |

MHK-Werte (mg/l) bestimmt durch Agar-Verdünnungstest
(Denley Multipoint Inoculator; Iso-Sensitest-Agar, Oxid)

| Teststamm | Beispiel 13 | 14 | 15 | 16 | 17 | 18 | Ciprofloxacin |
|---|---|---|---|---|---|---|---|
| Escherichia coli Neumann | 0,06 | 0,06 | ≤0,015 | 0,06 | 0,125 | 0,03 | ≤0,015 |
| Proteus mirabilis 8223 | 1 | 4 | 0,5 | 4 | 8 | 1 | 1 |
| Proteus vulgaris 1017 | 0,03 | 0,5 | 0,03 | 0,06 | 0,5 | 0,06 | ≤0,015 |
| Morganella morganii 932 | 0,125 | 0,25 | 0,03 | 0,06 | 0,5 | 0,06 | ≤0,015 |
| Providencia-stuartei 12052 | 2 | 4 | 1 | 32 | 8 | 4 | 4 |
| Staphylococcus aureus | | | | | | | |
| FK 422 | 0,06 | 0,25 | 0,03 | 0,125 | 0,5 | 0,125 | 0,25 |
| 1756 | 0,06 | 0,25 | 0,03 | 0,125 | 0,5 | 0,125 | 0,25 |
| 133 | 0,06 | 0,25 | 0,03 | 0,125 | 0,5 | 0,125 | 0,25 |
| Enterococcus faecalis 27101 | 0,125 | 0,25 | 0,03 | 0,5 | 1 | 0,25 | 0,05 |
| 9790 | 0,25 | 0,5 | - | 0,5 | 2 | 0,5 | 0,25 |

Die folgenden Beispiele erläutern die Erfindung:

Herstellung der Zwischenprodukte:

Beispiel A

N-(cis-4-Mothoxy-pyrrolidin-3-yl)-carbamidsäure-tert.butylester

a) trans-1-Benzyl-3-hydroxy-4-methoxypyrrolidin

Man erhitzt 34,9 g (0,2 mol) 3-Benzyl-6-oxa-3-azabicyclo-[3.1.0]hexan (US-Patent 4 254 135) mit 3,6 g (20 mmol) Natriummethylatlösung (30%) in 200 ml absolutem Methanol im Autoklaven 10 Stunden auf 120°C. Nach dem Abkühlen neutralisiert man mit 1,2 g (20 mmol) Essigsäure und entfernt das Lösungsmittel am Rotationsverdampfer. Der Rückstand wird in Tetrahydrofuran aufgenommen und das Natriumacetat abfiltriert. Das Filtrat wird eingeengt und der Rückstand destilliert.
Ausbeute: 40,9 g (91% der Theorie)
Siedepunkt: 112-116°C/ 0,1 mbar
Gehalt: 92%ig

b) cis-3-Amino-1-benzyl-4-methoxy-pyrrolidin

Man legt 5,6 g (25 mmol) trans-1-Benzyl-3-hydroxy-4-methoxy-pyrrolidin und 8,6 g (33 mmol) Triphenylphosphin in 40 ml absolutem Tetrahydrofuran vor und tropft bei 0°C eine Lösung von 6 g (34 mmol) Azodicarbonsäurediethylester in 40 ml absolutem Tetrahydrofuran hinzu. Anschließend gibt man bei 0°C 3,9 g (27 mmol) Phthalimid in kleinen Portionen innerhalb einer Stunde hinzu. Man rührt über Macht bei Raumtemperatur und engt ein. Den Rückstand löst man in 80 ml Essigester und setzt 80 ml Petrolether hinzu. Man läßt über Nacht auskristallisieren und filtriert die Kristalle (Triphenylphosphinoxid und Hydrazindicarbonsäurediethylester) ab. Das Filtrat wird eingeengt und der Rückstand mit 60 ml konzentrierter Salzsäure über Nacht unter Rückfluß erhitzt. Man dekantiert von ungelösten Rückständen und engt die Lösung ein. Der Rückstand wird in wenig Wasser aufgenommen, die Lösung mit festem Kaliumcarbonat alkalisch gestellt und fünfmal mit 50 ml Chloroform extrahiert. Man trocknet über Kaliumcarbonat, engt ein und destilliert den Rückstand.
Ausbeute: 3,4 g (65,9% der Theorie)
Siedepunkt: 95°C/0,2 mbar

c) N-(cis-1-Benzyl-4-methoxypyrrolidin-3-yl)-carbamidsäure-tert.-butylester

Zu einer Lösung von 0,65 g NaOH in 8 ml Wasser gibt man 3 g (14,5 mmol) cis-3-Amino-1-benzyl-4-methoxypyrrolidin und 11 ml tert.-Butanol. Dazu tropft man 3,5 g (16 mmol) Dikohlensäuredi-tert.-butylester. Man rührt über Nacht bei Raumtemperatur, saugt anorganische Salze ab und extrahiert das Filtrat mit Chloroform. Man trocknet über Kaliumcarbonat, engt ein und destilliert den Rückstand.
Ausbeute: 3,8 g (85,5% der Theorie)
Siedepunkt: 130-140° C/0,05 mbar

d) N-(cis-4-Methoxypyrrolidin-3-yl)-carbamidsäuretert.-butylester

Man hydriert 3,5 g (11,4 mmol) N-(cis-1-Benzyl-4-methoxypyrrolidin-3-yl)-carbamidsäure-tert.-butylester in 100 ml Methanol bei 100°C und 100 bar an 2 g Palladium-Aktivkohle (10% Pd). Man filtriert den Katalysator ab, engt das Filtrat ein und destilliert den Rückstand.
Ausbeute: 1,9 g (81,6% der Theorie)
Siedepunkt: 84° C/0,1 mbar

Beispiel B

N-(trans-4-Methoxy-pyrrolidin-3-yl)-carbamidsäure-tert.butylester

a) trans-3-Amino-1-benzyl-4-methoxy-pyrrolidin

Man löst 27 g (0,41 mol) Natriumazid in 50 ml Wasser und setzt 17,5 g (0,1 mol) 3-Benzyl-6-oxa-3-azabicyclo-[3.1.0]hexan in 300 ml Dioxan hinzu. Man erhitzt 72 Stunden unter Rückfluß, engt ein, löst anorganische Salze in Wasser und extrahiert mit Chloroform. Man trocknet über Kaliumcarbonat und engt ein. Der Rückstand wird in 50 ml absolutem Tetrahydrofuran gelöst und zu 4 g Natriumhydrid (80% in Paraffinöl) in 200 ml absolutem Tetrahydrofuran getropft. Man erhitzt eine Stunde unter Rückfluß und tropft dann 15 g (0,1 mol) Methyliodid hinzu. Anschließend erhitzt man über Nacht unter Rückfluß, engt ein, nimmt in Wasser auf und extrahiert mit Chloroform. Man trocknet über Kaliumcarbonat, engt ein und destilliert. Man erhält 13,1 g eines nach Gaschromatogramm 73%igen Materials. Hiervon tropft man 12,7 g in 40 ml absolutem Tetrahydrofuran zu einer Suspension von 4 g Lithiumaluminiumhydrid in 150 ml absolutem Tetrahydrofuran und erhitzt 2 Stunden unter Rückfluß. Man zersetzt überschüssiges Lithiumaluminiumhydrid durch vorsichtiges Zutropfen von je 4 ml Wasser, 15%iger Kalilauge und wieder 4 ml Wasser. Die anorganischen Salze werden abgesaugt und mehrfach mit Chloroform gewaschen. Die organischen Phasen werden über Kaliumcarbonat getrocknet, eingeengt und der Rückstand destilliert.
Ausbeute: 9 g (32,8% der Theorie)
Siedepunkt: 91° C/0,07 mbar

Das Produkt hat einen gaschromatographisch (Flächenmethode) ermittelten Gehalt von 75%.

b) H-(trans-1-Benzyl-4-methoxypyrrolidin-3-yl)carbamidsäure-tert.-butylester

Zu einer Lösung von 1,3 g NaOH in 15 ml Wasser gibt man 8,2 g (30 mmol) trans-3-Amino-1-benzyl-4-methoxy-pyrrolidin und 21 ml tert.-Butanol. Dazu tropft man 7,1 g (31 mmol) Dikohlensäuredi-tert.-butylester und rührt anschließend über Nacht bei Raumtemperatur. Man saugt von anorganischen Salzen ab, extrahiert das Filtrat mit Chloroform, trocknet über Kaliumcarbonat, engt ein und destilliert den Rückstand.
Ausbeute: 7,7 g (84,4% der Theorie)
Siedepunkt: 148° C/0,1 mbar
Schmelzpunkt: 88-90° C

c) N-(trans-4-Methoxypyrrolidin-3-yl)-carbamidsäuretert.-butylester

Man hydriert 6,7 g (22 mmol) N-(trans-1-Benzyl-4-methoxypyrrolidin-3-yl)-carbamidsäure-tert.-butylester in 150 ml Methanol bei 100 bar und 100°C an 2 g Palladium-Aktivkohle (10% Pd). Man saugt den Katalysator ab, engt das Filtrat ein und destilliert den Rückstand.
Ausbeute: 2,2 g (46% der Theorie)
Siedepunkt: 94°C/0,05 mbar

Beispiel C

trans-3-Amino-4-hydroxy-pyrrolidin

a) trans-3-Amino-1-benzyl-4-hydroxy-pyrrolidin

Man erhitzt 8,9 g (50 mmol) 3-Benzyl-6-oxa-3-azabicyclo-[3.1.0]hexan in 75 ml Ammoniaklösung (25%ig) 8 Stunden im Autoklaven auf 120°C. Die Lösung wird eingeengt und der Rückstand destilliert.
Ausbeute: 6 g (62,4% der Theorie)
Siedepunkt: 130-140°C/0,1 mbar
Schmelzpunkt: 82-84°C

b) trans-3-Amino-4-hydroxy-pyrrolidin

Man hydriert 5,2 g (27 mmol) trans-3-Amino-1-benzyl-4-hydroxy-pyrrolidin in 40 ml Methanol bei 100°C und 100 bar an 1 g Palladium-Aktivkohle (10% Pd). Man saugt den Katalysator ab, engt das Filtrat ein und destilliert den Rückstand.
Ausbeute: 1 g (36,3% der Theorie)
Siedepunkt: 110° C/0,3 mbar

Beispiel D

trans-4-Hydroxy-3-(2-hydroxyethylamino)-pyrrolidin

a) trans-1-Benzyl-4-hydroxy-3-(2-hydroxyethylamino)-pyrrolidin

Man erhitzt 40 g (0,22 mol) 3-Benzyl-6-oxa-3-azabicyclo-[3.1.0]hexan mit 42 g (0,68 mol) 2-Aminoethanol in 450 ml Wasser über Nacht unter Rückfluß. Man extrahiert die Lösung einmal mit tert.-Butylmethylether und engt die wäßrige Phase ein. Der Rückstand wird destilliert.
Ausbeute: 34,1 g (65,6% der Theorie)
Siedepunkt: 190°C/0,1 mbar

b) trans-4-Hydroxy-3-(2-hydroxyethylamino)-pyrrolidin

Man hydriert analog Beispiel C b) trans-1-Benzyl-4-hydroxy-3-(2-hydroxyethylamino)-pyrrolidin und erhält das Reaktionsprodukt als Öl.

Beispiel E

trans-4-Hydroxy-3-(2-hydroxyethyl-methyl-amino)-pyrrolidin

a) trans-1-Benzyl-4-hydroxy-3-(2-hydroxyethyl-methylamino)-pyrrolidin

Man setzt 17,5 g (0,1 mol) 3-Benzyl-6-oxa-3-azabicyclo-[3.1.0]hexan mit 17 g (0,1 mol) Methylaminoethanol analog Beispiel D a) in 200 ml Wasser um.
Ausbeute: 18,2 g (73% der Theorie)
Siedepunkt: 180-190°C/0,1 mbar

b) trans-4-Hydroxy-3-(2-hydroxyethyl-methyl-amino)-pyrrolidin

Analog Beispiel C b) hydriert man trans-1-Benzyl-4-hydroxy-3-(2-hydroxyethyl-methyl-amino)-pyrrolidin und erhält das Reaktionsprodukt als ölige Verbindung.

Beispiel F

2-Oxa-5,8-diazabicyclo[4.3.0]nonan-Dihydrochlorid

a) 8-Benzyl-2-oxa-5,8-diazabicyclo[4.3.0]nonan

Man erhitzt 15,6 g (66 mmol) 1-Benzyl-4-hydroxy-3-(2-hydroxyethylamino)-pyrrolidin in einem Gemisch aus 60 ml konzentrierter Schwefelsäure und 20 ml Wasser 6 Stunden unter Rückfluß. Man stellt mit konzentrierter Natronlauge alkalisch, saugt ausgeschiedenes Natriumsulfat ab und extrahiert das Filtrat mit Chloroform. Man trocknet über Kaliumcarbonat, engt ein und destilliert den Rückstand.
Ausbeute: 4,1 g (28,5% der Theorie)
Siedepunkt: 122-128°C (0,08 mbar)

b) 2-Oxa-5,8-diazabicyclo[4.3.0]nonan-Dihydrochlorid

Man hydriert eine Lösung von 4 g (18,2 mmol) 8-Benzyl-2-oxa-5,8-diazabicyclo[4.3.0]nonan in 100 ml Methanol und 3,5 ml konzentrierter Salzsäure an 2 g Palladium-Aktivkohle (10% Pd) bei 80°C und 100 bar. Der Katalysator wird abfiltriert und mit Wasser gewaschen. Die Filtrate werden eingeengt und durch Verreiben mit wenig Methanol kristallisiert. Man saugt ab, wäscht die Kristalle mit Aceton und trocknet an der Luft.
Ausbeute: 1,85 g (51% der Theorie)
Schmelzpunkt: 280°C unter Zersetzung

c) 2-Oxa-5,8-diazabicyclo[4.3.0]nonan

Man hydriert 7,2 g (33 mmol) 8-Benzyl-2-oxa-5,8-diazabicyclo[4.3.0]nonan in 400 ml Methanol mit 2,5 g Palladium-Aktivkohle (10 % Pd) bei 50 bar und 100°C. Man saugt den Katalysator ab, engt das Filtrat ein und destilliert den Rückstand.
Ausbeute: 3,1 g (73,4 % der Theorie); cis-trans Isomerengemisch 1:7
Siedepunkt: 58°C/0,1 mbar.

d) trans-2-Oxa-5,8-diazabicyclo[4.3.0]nonan

Analog Beispiel D a) wird 3-Benzyl-6-oxa-3-azabicyclo-[3.1.0]hexan mit 2-(Benzylamino)-ethanol zu trans-1-Benzyl-3-[N-benzyl-N-(2-hydroxyethyl)-amino]-4-hydroxypyrrolidin umgesetzt und anschließend analog Beispiel F a) zu 5,8-Dibenzyl-2-oxa-5,8-diazabicyclo[4.3.0]nonan umgesetzt und chromatographisch gereinigt (Kieselgel, Cyclohexan/tert.-Butylmethylether/Essigsäureethylester 1:1:1).
Die hydrogenolytische Debenzylierung erfolgt analog Beispiel F c) zu trans-2-Oxa-5,8-diazabicyclo[4.3.0]nonan, Siedepunkt: 60°C/0,1 mbar.

Beispiel G

5-Methyl-2-oxa-5,8-diazabicyclo[4.3.0]nonan-Dihydrochlorid

a) 8-Benzyl-5-methyl-2-oxa-5,8-diazabicyclo[4.3.0] nonan

Wie in Beispiel F a) setzt man 18 g (71,9 mmol) 1-Benzyl-4-hydroxy-3-(2-hydroxyethyl-methy-amino)-pyrrolidin in 60 ml konzentrierter Schwefelsäure und 30 ml Wasser um.
Ausbeute: 10 g (60% der Theorie)
Siedepunkt: 122°C/0,08 mbar

b) 5-Methyl-2-oxa-5,8-diazabicyclo[4.3.0]nonan-Dihydrochlorid

Man hydriert eine Lösung von 9,4 g (40 mmol) 8-Benzyl-5methyl-2-oxa-5,8-diazabicyclo[4.3.0]nonan in 150 ml Methanol und 7,4 ml konzentrierter Salzsäure an 3 g Palladium-Aktivkohle (10% Pd) bei 80°C und 100 bar. Der Katalysator wird abgesaugt und das Filtrat eingeengt. Der Rückstand wird mit Butanol/Aceton 1:1 verrieben, die Kristalle abgesaugt und im Exsikkator über $P_4O_{10}$ getrocknet. Das Produkt ist sehr hygroskopisch.
Ausbeute: 8,2 g (95% der Theorie)
Massenspektrum: $^{m}/_{e}$ 142 ($M^+$), 112 ($M^+$-$CH_2O$), 100 ($M^+$-$CH_2$-N=$CH_2$), 82 ($C_4H_4NO^+$), 68 ($C_4H_6N^+$)

Beispiel H

2-Methyl-3-oxa-2,7-diazabicyclo[3.3.0]octan

a) N-(2,2-Dimethoxyethyl)-carbamidsäureethylester

Zu 214 g (2 mol) Aminoacetaldehyddimethylacetal in 1 l Toluol und 90 g NaOH in 500 ml Wasser tropft man 214 g (2 mol) Chlorameisensäureethylester bei 10°C. Man rührt noch zwei Stunden bei Raumtemperatur, trennt die wäßrige Phase ab, sättigt sie mit Kochsalz und extrahiert mit Toluol. Die Toluollösungen werden über Magnesiumsulfat getrocknet, eingeengt und destilliert.
Ausbeute: 338 g (95,4% der Theorie)
Siedepunkt: 60°C/0,03 mbar

b) N-Allyl-N-(2,2-dimethoxyethyl)-carbamidsäureethylester

Man legt 20 g Natriumhydrid (80% im Paraffinöl) in 500 ml Toluol vor und tropft bei 80°C 89 g (0,5 mol) N-(2,2-Dimethoxyethyl)-carbamidsäureethylester hinzu. Man rührt eine Stunde bei 80°C und tropft dann 73 g (0,6 mol) Allylbromid innerhalb von drei Stunden hinzu. Man rührt über Nacht bei 80°C, bringt die Salze mit Wasser in Lösung und trennt die organische Phase ab. Die wäßrige Phase wird mit Toluol extrahiert, die organischen Phasen über Kaliumcarbonat getrocknet, eingeengt und der Rückstand destilliert.
Ausbeute: 68 g (62,6% der Theorie)
Siedepunkt: 65°C/0,09 mbar

c) N-Allyl-N (2-oxoethyl)-carbamidsäureethylester

Man erhitzt 68 g (0,313 mol) N-Allyl-N-(2,2-dimethoxyethyl)-carbamidsäureethylester mit 150 ml Ameisensäure eine Stunde auf 100°C. Man gießt auf Eis, extrahiert mehrfach mit Methylenchlorid, wäscht die organischen Phasen mit Natriumhydrogencarbonatlösung, trocknet über Magnesiumsulfat, engt ein und destilliert.
Ausbeute: 46,7 g (87,2% der Theorie)
Siedepunkt: 58°C/0,09 mbar

d) 2-Methyl-3-oxa-2,7-diazabicyclo[3.3.0]octan-7-carbonsäureethylester

Man löst 10 g (0,12 mol) Methylhydroxylamin-Hydrochlorid in 50 ml Methanol, kühlt im Eisbad und tropft 22 g (0,12 mol) 30%ige Natriummethylatlösung in Methanol hinzu. Man saugt von Kochsalz ab und wäscht das Salz mit 80 ml Toluol. Die Lösung von Methylhydroxylamin tropft man innerhalb einer Stunde zu 20 g (0,117 mol) N-Allyl-N-(2-(oxoethyl)-carbamidsäureethylester, der in 160 ml Toluol am Wasserabscheider unter Rückfluß erhitzt wird. Man erhitzt über Nacht unter Rückfluß und extrahiert das Produkt zweimal mit je 80 ml 10%iger Salzsäure. Die salzsauren Lösungen werden mit Kaliumcarbonat gesättigt und sechsmal mit je 200 ml Chloroform extrahiert. Man trocknet über $K_2CO_3$, engt ein und destilliert den Rückstand.
Ausbeute: 18,6 g (79,5% der Theorie)
Siedepunkt: 93°C/0,09 mbar

e) 2-Methyl-3-oxa-2,7-diazabicyclo[3.3.0]octan

Man erhitzt 13 g (65 mmol) 2-Methyl-3-oxa-2,7-diazabicyclo[3.3.0]octan-7-carbonsäureesthylester in 300 ml Wasser mit 41 g $Ba(OH)_2 \cdot 8H_2O$ über Nacht unter Rückfluß. Man setzt Kaliumcarbonat hinzu, saugt ausgefallenes Bariumcarbonat ab und extrahiert das Filtrat zehnmal mit je 100 ml Chloroform. Man trocknet über Kaliumcarbonat, engt ein und destilliert den Rückstand.
Ausbeute: 5,4 g (65% der Theorie)
Siedepunkt: 80°C/10 mbar

Beispiel I

1-Methyl-octahydropyrrolo[3,4-b]pyrrol (2-Methyl-2,7-diazabicyclo[3.3.0]octan)

a) 1-Benzyl-3-(2-chlorethyl-methyl-amino)-pyrrolidin-2,5-dion

74,8 g (0,4 mol) N-Benzylmaleinimid [Arch. Pharm. 308, 489 (1975)] und 52,0 g (0,4 mol) 2-Chlorekhyl-methylamin-Hydrochlorid worden in 400 ml Dioxan vorgelegt und 40,4 g (0,4 mol) Triethylamin bei 20°C zugetropft. Anschließend wird 5 Stunden unter Rücktluß gekocht. Dann wird der Ansatz auf 2 l Eiswasser gegossen, mit 3 mal 400 ml Chloroform extrahiert, der Extrakt mit Wasser gewaschen, über Natriumsulfat getrocknet und am Rotationsverdampfer eingeengt. Bei der Chromatographie des Rückstands (101,1 g) auf Kieselgel mit Essigester:Petrolether (1:2) werden 56,8 g (51% der Theorie) eines Öls erhalten.
$R_F$-Wert: 0,33 (Kieselgel, Essigester/Petrolether = 1:2)

b) 5-Benzyl-4,6-dioxo-1-methyl-octahydropyrrolo [3,4-b]pyrrol

7,2 g (0,24 mol) einer 80%igen Natriumhydrid-Suspension in Mineralöl werden in 150 ml absolutem Dimethylformamid (über Calciumhydrid getrocknet) suspendiert und 62 g (0,22 mol) 1-Benzyl-3-(2-chlorethyl-methylamino)-pyrrolidin-2,5-dion als Lösung in 50 ml absolutem Dimethylformamid bei Raumtemperatur zugetropft. Dabei erfolgt eine exotherme Reaktion unter Aufschäumen. Es wird mit weiteren 50 ml absolutem Dimethylformamid verdünnt, 1 Stunde bei Raumtemperatur nachgerührt, dann auf Eiswasser gegossen und mit Dichlormethan extrahiert. Der Extrakt wird mit Wasser gewaschen, mit Natriumsulfat getrocknet und am Rotationsverdampfer eingeengt. Der Rückstand wird auf Kieselgel mit Essigester:Petrolether (1:2) und später (1:1) chromatographiert. Dabei werden zunächst 16,4 g Edukt wiedergewonnen und anschließend 17,2 g (44% der Theorie, bezogen auf umgesetztes Edukt) öliges Produkt isoliert.
$R_f$-Wert = 0,26
(Kieselgel, Essigester:Petrolether = 1:1).

c) 5-Benzyl-1-methyl-octahydropyrrolo[3,4-b]pyrrol

1,52 g (40 mmol) Lithiumaluminiumhydrid werden in 30 ml wasserfreiem Tetrahydrofuran vorgelegt und 4,9 g (20 mmol) 5-Benzyl-4,6-dioxo-1-methyl-octahydropyrrolo-[3,4-b]pyrrol als Lösung in 15 ml wasserfreiem Tetrahydrofuran zugetropft. Dann wird 3 Stunden bei Siedetemperatur nachgerührt. Nacheinander werden zu dem Ansatz 1,5 ml Wasser, 1,5 ml 15%ige Kalilauge und 4,5 ml Wasser getropft, dann wird der Niederschlag abgesaugt und mit Tetrahydrofuran gewaschen. Das Filtrat wird am Rotationsverdampfer eingeengt und der Rückstand destilliert. Es werden 3,1 g (72% der Theorie) eines farblosen Destillats vom Siedepunkt 80°C/0,07 mbar erhalten.

d) 1-Methyl-octahydropyrrolo[3,4-b]pyrrol

6,49 g (30 mmol) 5-Benzyl-1-methyl-octahydropyrrolo-[3,4-b]-pyrrol werden in 100 ml absolutem Ether gelöst und 5,2 g über Phosphorpentoxid getrockneter Chlorwasserstoff eingeleitet. Die entstandene Hydrochlorid-Suspension wird im Vakuum eingeengt und der Rückstand in 100 ml Methanol aufgenommen. Dann wird mit 2 g Pd-C (5 %) 4 Stunden bei 80°C und 50 bar hydriert. Der Katalysator wird anschließend abfiltriert, das Filtrat eingeengt und der Rückstand mit 30 ml 40%iger Natronlauge und 50 ml Ether versetzt. Die etherische Phase wird abgetrennt und die wäßrige Phase mit 2 x 50 ml Ether extrahiert. Die vereinigten organischen Phasen werden über Natriumsulfat getrocknet, eingeengt und destilliert. Es werden 1,3 g (34% der Theorie) eines farblosen Öls vom Siedepunkt 65-66°C/12 mbar erhalten.
Reinheit: >99%

Beispiel J

Octahydropyrrolo[3,4-b]pyrrol (2,7-Diazabicyclo[3.3.0]-octan)

a) 1-Benzyl-3-(2-chlorethylamino)-pyrrolidin-2,5-dion

Nach der Arbeitsvorschrift des Beispiels Ia werden 74,8 g (0,4 mol) N-Benzylmaleinimid mit 58 g (0,5 mol) 2-Chlorethylamin-Hydrochlorid und 50,5 g (0,5 mol) Triethylamin umgesetzt. Nach der chromatographischen Aufarbeitung werden 81,6 g (77% der Theorie) eines Öls mit einem $R_F$-Wert von 0,24 (auf Kieselgel mit Essigester:Petrolether = 1:1) erhalten.

b) 5-Benzyl-4,6-dioxo-octahydropyrrolo[3,4-b]pyrrol

Nach der Arbeitsvorschrift des Beispiele Ib werden 17,4 g (0,58 mol) Natriumhydridsuspension mit 119 g (0,45 mol) 1-Benzyl-3-(2-chlorethylamino)-pyrrolidin-2,5-dion in 550 ml absolutem Dimethylformamid umgesetzt. Nach dem Stehen über Nacht wird wäßrig aufgearbeitet. Bei der chromatographischen Reinigung werden Verunreinigunden zunächst mit Essigester und dann das Produkt mit Essigester:Methanol (3:1) ($R_F$-Wert 0.55) eluiert. Es werden 57,7 g Produkt (56% der Theorie) isoliert.

c) 5-Benzyl-octahydropyrrolo[3,4-b]pyrrol

Nach der Arbeitsvorschrift des Beispiels Ic werden 57,7 g (0,25 mol) rohes 5-Benzyl-4,6-dioxo-octahydropyrrolo[3,4-b]pyrrol mit 21,4 g (0,56 mol) Lithiumaluminiumhydrid durch 10-stündiges Kochen in 700 ml abs. Tetrahydrofuran reduziert. Bei der destillativen Aufarbeitung werden 21,0 g (41,1% der Theorie) eines Öls vom Siedepunkt 95°C/ 0,1 mbar erhalten.

d) Octahydropyrrolo[3,4-b]pyrrol

21,0 g (0,104 mol) 5-Benzyl-octahydropyrrolo[3,4-b]pyrrol werden in 180 ml eisgekühltem Methanol vorgelegt und mit 17,3 ml (0,208 mol) konzentrierter Salzsäure versetzt. Dann wird mit 2 g Pd-C (5 %) 4 Stunden bei 90°C und 100 bar hydriert. Der Katalysator wird abfiltriert, das Filtrat mit 37,4 g (0,208 mol) 30%iger Natriummethylat-Lösung versetzt, erneut filtriert und das Filtrat eingeengt. Der Rückstand wird über eine kleine Vigreux-Kolonne destilliert. Es werden 5,6 g eines farblosen Öls (48% der Theorie) vom Siedepunkt 93-95°C/30 mbar erhalten, welches an der Luft raucht und in der Vorlage langsam erstarrt (Schmelzpunkt 40°C).

Beispiel K

Octahydropyrrolo[3,4-b]pyridin (2,8-Diazabicyclo[4.3.0] nonan

a) 6-Benzyl-5,7-dioxo-octahydropyrrolo[3,4-b]pyridin

47,6 g (0,2 mol) Pyridin-2,3-dicarbonsäure-N-benzylimid (Brit. Pat. 1 086 637; Chem. Abstr. 68, 95695w) werden in 400 ml Glykolmonomethylether über 15 g Ruthenium auf Aktivkohle (5%) bei 90°C und 100 bar hydriert, bis die berechnete Wasserstoffmenge aufgenommen worden ist. Dann wird der Katalysator abfiltriert und das Filtrat am Rotationsverdampfer eingeengt. Es werden 44 g eines öligen Rohproduktes erhalten.
Die entsprechende Hydrierung mit Palladium/Aktivkohle (5 %ig) liefert in quantitativer Ausbeute ein Reinprodukt vom Schmelzpunkt 67-69°C.

b) 6-Benzyl-octahydropyrrolo[3,4-b]pyridin

Nach der Arbeitsvorschrift des Beispiels Ic werden 44 g (ca. 0,18 mol) rohes oder reines 6-Benzyl-5,7-dioxooctahydropyrrolo[3,4-b]pyridin mit 15,2 g (0,40 mol) Lithiumaluminiumhydrid in 390 ml absolutem Tetrahydrofuran innerhalb von 10 Stunden reduziert. Bei der Destillation werden 24,4 g eines farblosen Öls mit einem Siedepunkt von 93-95°C/0,06 mbar erhalten.

c) Octahydropyrrolo[3,4-b]pyridin

69 g (0,32 mol) 6-Benzyl-octahydropyrrolo[3,4-b]pyridin werden in 450 ml Methanol bei 90° C/90 bar über 7 g Palladium auf Aktivkohle (5 %ig) innerhalb von 3 Stunden hydriert. Der Katalysator wird anschließend abfiltriert, das Filtrat eingeengt und der Rückstand destilliert.
Es werden 33,8 g (84 % der Theorie) eines farblosen Festkörpers mit einem Schmelzpunkt von 65-67°C und einem Siedepunkt von 78° C/9 mbar erhalten.

Beispiel L

1-Methyl-octahydropyrrolo[3,4-b]pyridin (2-Methyl-2,8-diazabicyclo[4.3.0]nonan)

a) 1-Methyl-pyridinium-2,3-dicarbonsäure-N-benzylimid-iodid

190,5 g (0,8 mol) Pyridin-2,3-dicarbonsäure-N-benzylimid werden unter Erwärmen in 800 ml Nitromethan gelöst und 136 g (0,96 mol) Methyliodid zugetropft. Anschliessend wird 8 Stunden unter Rückflußkühlung (Kühlwasser 0°C) gekocht. Nach dem Erkalten wird der Feststoff abgesaugt und mit Dichlormethan gewaschen. Es werden 123 g dunkelrote Kristalle mit einem Schmelzpunkt 162-165°C (Zersetzung) erhalten.

b) 6-Benzyl-1-methyl-5,7-dioxo-octahydropyrrolo-[3,4-b]pyridin

38 g (0,1 mol) 1-Methyl-pyridinium-2,3-dicarbonsäure-N-benzylimid-iodid werden bei 30°C und 70 bar über 1 g Platinoxid in 450 ml Glykolmonmethylether bis zur Beendigung der Wasserstoffaufnahme (51 Stunden) hydriert. Der Katalysator wird dann abfiltriert, das Filtrat eingsengt, der Rückstand in 300 ml Chloroform aufgenommen und die Lösung 2 x mit je 300 ml 10%iger Sodalösung sowie 300 ml Wasser gewaschen. Nach dem Trocknen über Natriumsulfat wird eingeengt. Es bleiben 27 g eines öligen Rückstands zurück.

c) 6-Benzyl-1-methyl-octahydropyrrolo[3,4-b]pyridin

Nach der Arbeitsvorschrift des Beispiels Ic werden 19,2 g (0,08 mol) rohes 6-Benzyl-1-methyl-5,7-dioxooctahydropyrrolo[3,4-b]pyridin mit 6,1 g (0,16 mol) Lithiumaluminiumhydrid im absolutem Tetrahydrofuran reduziert.
Ausbeute: 9,5 g (52% der Theorie),
Siedepunkt: 93-96° C/0,1 mbar.

d) 1-Methyl-octahydropyrrolo[3,4-b]pyridin

Nach der Arbeitsvorschrift des Beispiels Id werden 11,7 g (54 mmol) 6-Benzyl-1-methyl-octahydropyrrolo-[3,4-b]pyridin als Dihydrochlorid in 100 ml Methanol über Palladium auf Aktivkohle hydriert. Bei der destillativen

Aufarbeitung werden 2,6 g (34% der Theorie) eines farblosen Öls vom Siedepunkt 83-85°/12 mbar erhalten.

Beispiel M

trans-4-Methoxy-3-methylamino-pyrrolidin-Dihydrochlorid

a) trans-1-Benzyl-3-benzylmethylamino-4-hydroxypyrrolidin

Man erhitzt 19,4 g (0,1 mol) 90 %iges 3-Benzyl-6-oxa-3-azabicyclo[3.1.0]hexan mit 14,5 g (0,12 mol) Benzylmethylamin in 100 ml Dioxan und 200 ml Wasser über Nacht unter Rückfluß. Man extrahiert mit $CHCl_3$, trocknet die Extrakte mit $K_2CO_3$, engt ein und destilliert bis 160°C (Ölbadtemperatur) an. Rohausbeute: 18,3 g
Gehalt: 100 % (gaschromatographisch bestimmt)

b) trans-1-Benzyl-3-benzylmethylamino-4-methoxypyrrolidin

Man tropft 17,3 g (58 mmol) rohes trans-1-Benzyl-3-benzylmethylamino-4-hydroxy-pyrrolidin in 80 ml absolutem Tetrahydrofuran zu 2,8 g (93,3 mmol) 80 %igem Natriumhydrid in 40 ml absolutem Tetrahydrofuran und erhitzt gleichzeitig unter Rückfluß. Nach dem Ende der Wasserstoffentwicklung tropft man 8,7 g (61 mmol) Methyliodid hinzu und erhitzt dann über Nacht unter Rückfluß. Man gießt auf Eiswasser, extrahiert mit Toluol, trocknet die Extrakte mit $K_2CO_3$, engt ein und destilliert.
Ausbeute: 9,7 g (52 % der Theorie)
Siedepunkt: 140-150° C/0,1 mbar

c) trans-4-Methoxy-3-methylamino-pyrrolidin-Dihydrochlorid

Man löst 9,3 g (29 mmol) trans-1-Benzyl-3-benzylmethylamino-4-methoxy-pyrrolidin in 100 ml Methanol, fügt 4,8 ml konzentrierte Salzsäure hinzu und hydriert an 4 g 10 %iger Pd-Aktivkohle bei 90°C und 100 bar. Man saugt den Katalysator ab, engt das Filtrat ein und kristallisiert den Rückstand aus Isopropanol/Methanol um.
Ausbeute: 3,7 g (62,8 % der Theorie)
Schmelzpunkt: 157-162°C

Beispiel N

2,5-Dimethyl-3-oxa-2,7-diazabicyclo[3.3.0]octan

a) N-(2-Methylprop-2-enyl)-N-(2,2-dimethoxyethyl)-urethan

Zu 20 g Natriumhydrid (80 %ig) in 500 ml absolutem Toluol tropft man bei 90°C 89 g (0,5 mol) N-(2,2-Dimethoxyethyl)-urethan. Wenn kein Wasserstoff mehr entsteht tropft man 54 g (0,6 mol) Methallylchlorid hinzu und rührt über Nacht bei 90°C. Das ausgeschiedene Kochsalz wird mit wenig Wasser gelöst, die organische Phase abgetrennt, über $K_2CO_3$ getrocknet, eingeengt und destilliert.
Ausbeute: 71,3 g (61,7 % der Theorie)
Siedepunkt: 60°C/0,08 mbar

b) N-(2-Methylprop-2-enyl)-N-(2-oxoethyl)-urethan

Man erhitzt 11,5 g (50 mmol) N-(2-Methylprop-2-enyl)-N-(2,2-dimethoxyethyl)-urethan und 1,25 g (5 mmol) Pyridinium-p-toluolsulfonat in 100 ml Aceton und 10 ml Wasser zwei Tage unter Rückfluß. Man engt ein und destilliert den Rückstand.
Ausbeute: 5,3 g (61,2 % der Theorie)
Siedepunkt: 73°C/0,1 mbar

c) 2,5-Dimethyl-3-oxa-2,7-diazabicyclo[3.3.0]octan-7-carbonsäureethylester

Zu 10 g (0,12 mol) N-Methylhydroxylamin-Hydrochlorid in 26 ml Methanol tropft man 21,7 g 30 %ige Natriummethylatlösung. Man saugt das Kochsalz ab und wäscht mit 8 ml Methanol und 80 ml Toluol. Diese Lösung tropft man zu 19,2 g (0,11 mol) N-(2-Methylprop-2-enyl)-N-(2-oxoethyl)-urethan, das in 160 ml Toluol am Wasserabscheider unter Rückfluß erhitzt wird. Man erhitzt über Nacht unter Rückfluß, extrahiert das Produkt mit 160 ml 10 %iger Salzsäure,

stellt die salzsaure Lösung mit Kaliumcarbonat alkalisch und extrahiert mit sechsmal 200 ml $CHCl_3$. Man trocknet die Extrakte über $K_2CO_3$, engt ein und destilliert.
Ausbeute: 13 g (55 % der Theorie)
Siedepunkt: 88-95° C/0,08 mbar

d) 2,5-Dimethyl-3-oxa-2,7-diazabicyclo[3.3.0]octan

Man erhitzt 13 g (60,6 mmol) 2,5-Dimethyl-3-oxa-2,7-diazabicyclo[3.3.0]octan-7-carbonsäureethylester mit 33 g $Ba(OH)_2 \cdot 8H_2O$ in 330 ml Wasser über Nacht unter Rückfluß. Man saugt $BaCO_3$ ab, setzt $K_2CO_3$ zum Fitrat, saugt erneut ab und extrahiert das Filtrat zehnmal mit je 100 ml $CNCl_3$. Man trocknet die Extrakte über $K_2CO_3$, engt ein und destilliert.
Ausbeute: 5,9 g (63,7 % der Theorie)
Siedepunkt: 64° C/5 mbar

Beispiel O

2,8-Dimethyl-3-oxa-2,7-diazabicyclo[3.3.0]octan

a) N-(1,1-Dimethoxyprop-2-yl)-urethan

Zu 86,2 g (0,72 mol) 2-Aminopropionaldehyddimethylacetal in 350 ml Toluol und 32 g (0,8 mol) NaOH in 300 ml Wasser tropft man unter Eiskühlung 80 g (0,73 mol) Chlorameisensäureethylester. Man rührt noch zwei Stunden bei Raumtemperatur, trennt die organische Phase ab, extrahiert die wäßrige Phase mit Toluol und trocknet die Toluallösungen über $K_2CO_3$. Man engt ein und destilliert.
Ausbeute: 132 g (95 % der Theorie)
Siedepunkt: 55°C/0,06 mbar

b) N-Allyl-N-(1,1-dimethoxyprop-2-yl)-urethan

Zu 25 g Natriumhydrid (80 %ig) in 700 ml absolutem Toluol tropft man bei 90°C 131 g (0,686 mol) N-(1,1-Dimethoxyprop-2-yl)-urethan. Nach Beendigung der Wasserstoffentwicklung tropft man bei 90°C 61,2 g (0,8 mol) Allylchlorid hinzu und rührt über Nacht bei 90°C. Ausgeschiedenes Kochsalz wird mit Wasser aufgelöst, die organische Phase abgetrennt, über $K_2CO_3$ getrocknet, eingeengt und destilliert.
Ausbeute: 78 g (31,7 % der Theorie)
Siedepunkt: 62-69°C/0,06 mbar.
Gehalt: 64,5 %ig (gaschchromatographisch bestimmt)

c) N-Allyl-N-(1-oxoprop-2-yl)-urethan

Man erhitzt 76,5 g (0,213 mol) 64,5 %iges N-Allyl-N-(1,1-dimethoxyprop-2-yl)-urethan in 180 ml Ameisensäure eine Stunde auf 100°C. Man gießt auf Eiswasser, extrahiert mit $CH_2Cl_2$, wäscht die Extrakte mit $NaHCO_3$-Lösung neutral, trocknet über $MgSO_4$, engt ein und destilliert.
Ausbeute: 36 g (80,9 % der Theorie)
Siedepunkt: 97-102°C/8 mbar
Gehalt: 88,8 %ig (gaschromatographisch bestimmt)

d) 2,8-Dimethyl-3-oxa-2,7-diazbicyclo[3.3.0]octan-7-carbonsäureethylester

Man stellt aus 16,4 g (0,2 mol) N-Methylhydroxylamin-Hydrochlorid in 33 ml absolutem Methanol und 36 g (0,2 mol) 30 %iger Natriummethylatlösung eine methanolische Methylhydroxylaminlösung her, verdünnt sie mit 130 ml Toluol und tropft sie zu 354 g (0,17 mol) N-Allyl-N-(1-oxoprop-2-yl)-urethan in 250 ml Toluol, welches am Wasserabscheider unter Rückfluß erhitzt wird. Man erhitzt über Nacht unter Rückfluß, extrahiert das Produkt mit verdünnter Salzsäure, stellt die salzsaure Lösung mit $K_2CO_3$ alkalisch und extrahiert mit $CHCl_3$. Man trocknet über $K_2CO_3$, engt ein und destilliert.
Ausbeute: 18,5 g (50,8 % der Theorie)
Siedepunkt: 95-105° C/0,1 mbar

e) 2,8-Dimethyl-3-oxa-2,7-diazabicyclo[3.3.0]octan

Man erhitzt 9,2 g (42,9 mmol) 2,8-Dimethyl-3-oxa-2,7-diazabicyclo[3.3.0]octan-7-carbonsäureethylester mit 23,5 g $Ba(OH)_2 \cdot 8H_2O$ in 235 ml Wasser über Nacht unter Rückfluß. Man saugt $BaCO_3$ ab, versetzt das Filtrat mit $K_2CO_3$ und saugt erneut ab. Das Filtrat wird zehnmal mit je 50 ml $CHCl_3$ extrahiert, die Extrakte über $K_2CO_3$ getrocknet, eingeengt und destilliert.
Ausbeute: 1,7 g
Siedepunkt: 87-92° C/10 mbar
Es handelt sich um ein Gemisch der möglichen Stereoisomeren im Verhältnis 3:1 ($^1$H-NMR).
Im Nachlauf konnten 4 g Ausgangsmaterial zurückgewonnen werden.

Beispiel P

2-Methyl-4-oxa-2,8-diazabicyclo[4.3.0]nonan

a) 4-Hydroxymethyl-3-methylaminopyrrolidin-1-carbonsäureethylester

Man hydriert 10 g (50 mmol) 2-Methyl-3-oxa-2,7-diazabicyclo[3.3.0]octan-7-carbonsäureethylester (Beispiel H d)) in 200 ml Ethanol an 3 g Pd-Aktivkohle (10 % Pd) bei 50°C und 50 bar. Der Katalysator wird abfiltriert, das Filtrat eingeengt und der Rückstand destilliert.
Ausbeute: 8,1 g (80 % der Theorie)
Siedepunkt: 135-140° C/0,1 mbar

b) 2-Methyl-4-oxa-2,8-diazabicyclo[4.3.0]nonan-8-carbonsäureethylester

Man löst 10,1 g (50 mmol) 4-Hydroxymethyl-3-methylamino-pyrrolidin-1-carbonsäureethylester und 8 g (0,1 mol) 37 %ige Formaldehydlösung in 100 ml Butanol und rührt über Nacht bei Raumtemperatur. Anschließend engt man ein und destilliert den Rückstand.
Ausbeute: 9,5 g (88,7 % der Theorie)
Siedepunkt: 110° C/0,1 mbar

c) 2-Methyl-4-oxa-2,8-diazabicyclo[4.3.0]nonan

Man erhitzt 9 g (42 mmol) 2-Methyl-4-oxa-2,8-diazabicyclo[4.3.0]nonan-8-carbonsäureethylester mit 28 g $Ba(OH)_2 \cdot 8H_2O$ in 280 ml Wasser über Macht unter Rückfluß. Man saugt $BaCO_3$ ab, engt ein und kocht den Rückstand mit Dioxan aus. Die Dioxanlösung wird eingeengt und der Rückstand destilliert.
Ausbeute: 1,3 g (21,8 % der Theorie)
Siedepunkt: 115°C/8 mbar.

d) 4-Hydroxymethyl-3-methylaminopyrrolidin

Man erhitzt 34 g (0,168 mol) 4-Hydroxymethyl-3-methylaminopyrrolidin-1-carbonsäureethylester mit 100 g $Ba(OH)_2 \cdot 8H_2O$ in 400 ml Wasser über Macht unter Rückfluß. Man saugt $BaCO_3$ ab, engt das Filtrat ein und kocht den Rückstand zehnmal mit je 100 ml Dioxan aus. Man filtriert die Dioxanlösungen, engt ein und destilliert.
Ausbeute: 13 g (60,3 % der Theorie)
Siedepunkt: 85-88°C/0,08 mbar

e) 2-Methyl-4-oxa-2,8-diazabicyclo[4.3.0]nonan

Zu 13 g (0,101 mol) 4-Hydroxymethyl-3-methylaminopyrrolidin in 100 ml n-Butanol tropft man bei Raumtemperatur 8,1 g (0,1 mol) 37 %ige Formaldehydlösung in 20 ml n-Butanol hinzu. Man rührt über Nacht bei Raumtemperatur, engt ein und destilliert.
Ausbeute: 8,7 g (61,2 % der Theorie)
Siedepunkt: 84°C/6 mbar

Beispiel Q

3-Oxa-2,7-diazabicyclo[3.3.0]octan

a) 2-(Tetrahydropyran-2-yl)-3-oxa-2,7-diazabicyclo[3.3.0]octan-7-carbonsäureethylester

Man erhitzt 18,1 g (0,106 mol) N-Allyl-N-(2-oxoethyl)-carbamidsäureathylester (Beispiel M c)) in 220 ml Toluol unter Rückfluß und tropft 14,2 g (0,12 mol) 5-Hydroxypentanaloxim (Acta Chim. Acad. Sci. Hung., 14, 333 (1958)) in 55 ml heißem Toluol gelöst hinzu. Man erhitzt über Nacht unter Rückfluß, engt ein und destilliert.
Ausbeute: 15,5 g (54 % der Theorie)
Siedepunkt: 160°C/0,01 mbar

b) 3-Oxa-2,7-diazabicyclo[3.3.0]octan-7-carbonsäureethylester

Man erhitzt 15 g (55,5 mmol) 2-(Tetrahydropyran-2-yl)-3-oxa-2,7-diazabicyclo[3.3.0]octan-7-carbonsäureethylester mit 8,25 g (56 mmol) 70 %iger Perchlorsäure in 100 ml Ethanol 30 Minuten unter Rückfluß. Man setzt 10,5 g (58 mmol) 30 %ige Natriummethylatlösung hinzu, engt ein, nimmt in Wasser auf, sättigt mit $K_2CO_3$ und extrahiert mit $CHCl_3$. Man trocknet über $K_2CO_3$, engt ein und destilliert.
Ausbeute: 7,6 g (73,5 % der Theorie)
Siedepunkt: 125-130°C/0,1 mbar

c) 3-Oxa-2,7-diazabicyclo[3.3.0]octan-7-carbonsäureethylester

Man erhitzt 8,5 g (50 mmol) N-(2-Oxoethyl)-N-allylcarbamidsäureethylester mit 5,5 g (50 mmol) o-Trimethylsilylhydroxylamin in 100 ml Xylol über Nacht unter Rückfluß. Man engt ein und destilliert.
Ausbeute: 6,8 g (73 % der Theorie)
Siedepunkt: 120-122°C/0,05 mbar

d) 3-Oxa-2,7-diazabicyclo[3.3.0]octan

Man erhält diese Substanz analog Beispiel N d) durch Verseifen von 3-Oxa-2,7-diazabicyclo[3.3.0]-octan-7-carbonsäureethylester mit $Ba(OH)_2 \cdot 8H_2O$.
Siedepunkt: 75°C/10 mbar.

Beispiel R

3-Methyl-2,7-diazabicyclo[3.3.0]octan

Analog Beispiel I erhält man 3-Methyl-2,7-diazabicyclo[3.3.0]octan.
Siedepunkt: 68-70°C/6 mbar.

Beispiel S

2,3-Dimethyl-2,7-diazabicyclo[3.3.0]octan

Analog Beispiel I erhält man 2,3-Dimethyl-2,7-diazabicyclo[3.3.0.]octan.
Siedepunkt: 72-74°C/10 mbar.

Beispiel T

1,2-Dimethyl-3-oxa-2,7-diazabicyclo[3.3.0]octan

a) N-Allyl-N-(2,2-dimethoxypropyl)-acetamid

Zu 29,6 g (0,987 mol) Natriumhydrid (80 %ig in Paraffinöl) in 750 ml absolutem Toluol tropft man bei 80°C 119 g (74 mol) 2,2-Dimethoxypropylacetamid. Anschließend rührt man eine Stunde und tropft dann 100 g (0,83 mol) Allylbromid bei 80°C hinzu. Man rührt über Nacht bei 80°C, kühlt ab und löst die Salze mit Wasser. Man trennt die wäßrige Phase ab und extrahiert sie zweimal mit je 100 ml

Toluol. Man trocknet die Toluollösungen über $K_2CO_3$, engt ein und destilliert.
Ausbeute: 112 g (75,6 % der Theorie)
Siedepunkt: 70°C/0,08 mbar.

b) N-Allyl-N-(2-oxopropyl)-acetamid

Man erhitzt 85,5 g (0,425 mol) N-Allyl-N-(2,2-dimethoxypropyl)-acetamid mit 212 ml Ameisensäure eine Stunde unter Rückfluß. Man gießt auf 500 g Eis, extrahiert mehrfach mit Methylenchlorid, wäscht die organischen Phasen mit Natriumhydrogencarbonatlösung, trocknet über Magnesiumsulfat, engt ein und destilliert.
Ausbeute: 50 g (75,8 % der Theorie)
Siedepunkt: 79°C/0,25 mbar.

c) 7-Acetyl-1,2-dimethyl-3-oxa-2,7-diazabicyclo-[3.3.0]octan

Man löst 15,5 g (0,1 mol) N-Allyl-N-(2-oxopropyl)-acetamid in 100 ml Dioxan und setzt 9 g wasserfreies Natriumacetat sowie 9 g (0,108 mol) N-Methylhydroxylaminhydrochlorid in 10 ml Wasser hinzu. Man erhitzt über Nacht unter Rückfluß, kühlt ab, saugt Salze ab und wäscht sie mit Dioxan. Das Filtrat wird eingeengt, der Rückstand in 100 ml Wasser aufgenommen und mit $K_2CO_3$ versetzt. Man extrahiert mit $CHCl_3$, trocknet über $K_2CO_3$, engt ein und destilliert.
Ausbeute: 15,9 g (86,3 % der Theorie)
Siedepunkt: 75°C/0,1 mbar.

d) 1,2-Dimethyl-3-oxa-2,7-diazabicyclo[3.3.0]octan

Man erhitzt 11,8 g (64 mmol) 7-Acetyl-1,2-dimethyl-3-oxa-2,7-diazabicyclo[3.3.0]octan mit 12 g NaOH in 36 ml Wasser über Nacht unter Rückfluß. Man sättigt mit $K_2CO_3$, extrahiert mehrfach mit $CHCl_3$, trocknet über $K_2CO_3$, engt ein und destilliert.
Ausbeute: 4,7 g (51,6 % der Theorie)
Siedepunkt: 40°C/0,2 mbar.

Beispiel U

2,4-Dimethyl-3-oxa-2,7-diazabicyclo[3.3.0]octan

a) N-(But-2-enyl)-N-(2,2-dimethoxyethyl)-carbamidsäureethylester

Zu 17,5 g (0,58 mol) NaH (80 %ig in Paraffinöl) in 500 ml absolutem Toluol tropft man bei 80°C 89 g (0,5 mol) N-(2,2-Dimethoxyethyl)-carbamidsäureethylester. Anschließend rührt man eine Stunde und tropft dann bei 80°C 80 g (0,59 mol) 1-Brom-2-buten hinzu. Man rührt über Nacht bei 80°C, kühlt ab, bringt die Salze mit Wasser in Lösung, trennt die wäßrige Phase ab und extrahiert sie mit Toluol. Die Toluollösungen werden über $K_2CO_3$ getrocknet, eingeengt und destilliert.
Ausbeute: 90 g (77,8 % der Theorie)
Siedepunkt: 65°C/0,1 mbar.

b) N-(But-2-enyl)-N-(2-oxoethyl)-carbamidsäureethylester

Man erhitzt 90 g (0,39 mol) N-(But-2-enyl)-N-(2,2-dimethoxyethyl)-carbamidsäureethylester mit 200 ml Ameisensäure eine Stunde unter Rückfluß. Man gießt auf 500 g Eis, extrahiert mit Methylenchlorid, wäscht die organischen Phasen mit Natriumhydrogencarbonatlösung, trocknet über Magnesiumsulfat, engt ein und destilliert.
Ausbeute: 33,6 g (46,5 % der Theorie)
Siedepunkt: 65° C/0,1 mbar.

c) 2,4-Dimethyl-3-oxa-2,7-diazabicyclo[3.3.0]octan-7-carbonsäureethylester

Man löst 18,4 g (0,1 mol) N-(But-2-enyl)-N-(2-oxoethyl)-carbamidsäureethylester in 100 ml Dioxan und setzt 9 g wasserfreies Natriumacetat sowie 9 g (0,108 mol) N-Methylhydroxylaminhydrochlorid in 10 ml Wasser hinzu. Man erhitzt über Nacht unter Rückfluß, kühlt ab, saugt Salze ab und wäscht sie mit Dioxan. Das Filtrat wird eingeengt, der Rückstand in 100 ml Wasser aufgenommen und mit $K_2CO_3$ versetzt. Man extrahiert mit $CHCl_3$, trocknet über $K_2CO_3$,

engt ein und destilliert.
Ausbeute: 15,0 g (70 % der Theorie)
Siedepunkt: 74-87° C/0,1 mbar.

d) 2,4-Dimethyl-3-oxa-2,7-diazabicyclo[3.3.0]octan

Man erhitzt 13,2 g (61,6 mmol) 2,4-Dimethyl-3-oxa-2,7-diazabicyclo[3.3.0]octan-7-carbonsäureethylester mit 39 g $Ba(OH)_2 \cdot 8H_2O$ in 200 ml Wasser über Nacht unter Rückfluß. Man versetzt mit $K_2CO_3$, saugt $BaCO_3$ ab und extrahiert das Filtrat mehrfach mit $CHCl_3$. Man trocknet über $K_2CO_3$, engt ein und destilliert.
Ausbeute: 4,8 g (54,8 % der Theorie)
Siedepunkt: 74°C/8 mbar.

Beispiel V

2,7-Diazabicyclo[3.3.0]octan-2-carbonsäureethylester

Analog Beispiel Oa) wird 7-Benzyl-2,7-diazabicyclo[3.3.0]octan (Beispiel Jc) mit Chlorameisensäureethylester zu 7-Henzyl-2,7-diazabicyclo[3.3.0]octan-2-carbonsäureethylester umgesetzt und dieser anschließend analog Beispiel Jd) hydrogenolytisch debenzyliert. Es wird ein farbloses Öl vom Siedepunkt 90°C/0,1 mbar erhalten.

Beispiel W

2-Phenyl-2,7-diazabicyclo[3.3.0]octan

Die Herstellung erfolgt analog Beispiel I);
Siedepunkt: 103° C/0,08 mbar.

Beispiel X

4-Oxa-2,8-diazabicyclo[4.3.0]nonan

a) 3-Amino-4-hydroxymethyl-pyrrolidin-1-carbonsäureethylester

Analog Beispiel Pa) wird 3-Oxa-2,7-diazabicyclo[3.3.0]octan-7-carbonsäureethylester (Beispiel Qc) hydriert.
Siedepunkt: 163-168°C/0,8 mbar

b) 3-Amino-4-hydroxymethyl-pyrrolidin

Analog Beispiel Pd) wird 3-Amino-4-hydroxymethylpyrrolidin-1-carbonsäureethylester verseift.
Siedepunkt: 78°C/0,06 mbar

c) 4-Oxa-2,8-diazabicyclo[4.3.0]nonan

Analog Beispiel Pe) wird 3-Amino-4-hydroxymethylpyrrolidin mit Formaldehydlösung umgesetzt.
Siedepunkt: 50-60°C/0,07 mbar

Beispiel Y

trans-3-Ethylamino-4-methylthio-pyrrolidin

a) 1-Benzoyl-trans-3-ethylamino-4-methylthio-pyrrolidin

8,65 g (50 mmol) 1-Benzoyl-2,5-dihydropyrrol [Chem. Ber. 22, 2521 (1889)] werden in 30 ml Dichlormethan vorgelegt, und bei 0°C 4,94 g (60 mmol) Methansulfonsäurechlorid in 20 ml Dichlormethan zugetropft. Es wird 16 Stunden bei 20-25°C nachgerührt, bei 8 mbar eingeengt und der Rückstand in 50 ml Tetrahydrofuran gelöst. Dann werden 18 g (0,2 mol) 50 %ige wäßrige Ethylamin-Lösung zugesetzt. Der Ansatz wird 18 Stunden unter Rückflußkühlung gekocht, auf Wasser gegossen und mit Dichlormethan extrahiert. Beim Einengen werden 11,1 g Rohprodukt erhalten, das mit Essigsäureethylester/Ethanol 5:1 auf Kieselgel (RF-Wert 0,34) chromatographiert wird.

Ausbeute: 7,4 g (56 % der Theorie).

b) trans-3-Ethylamino-4-methylthio-pyrrolidin

6,0 g (22 mmol) 1-Benzoyl-trans-3-ethylamino-4-methylthio-pyrrolidin werden mit 22 ml 5 n NaOH 24 h bei 100°C kräftig gerührt, bis der Ansatz homogen ist. Dann wird mit 3 x 80 ml Ether extrahiert, der Extrakt über Natriumsulfat getrocknet und am Rotationsverdampfer eingeengt. Das Rohprodukt wird über eine Mikro-Einstichkolonne destilliert.
Ausbeute: 1,56 g (44 % der Theorie) farblose Flüssigkeit,
Siedepunkt: 52° C/0,1 mbar

Beispiel Z

trans-3-Amino-4-methylthio-pyrrolidin

In Analogie zu Beispiel Y läßt man 1-Benzoyl-2,5-dihydropyrrol mit Methylsulfenylchlorid zu 1-Benzoyl-3-chlor-4-methylthio-pyrrolidin reagieren, setzt dieses als Rohprodukt mit Ammoniak zu 3-Amino-1-benzoyl-4-methylthio-pyrrolidin um und entfernt den Benzoylrest mit Natronlauge.
Ausbeute über 3 Stufen: 47 % der Theorie,
Siedepunkt: 108-110°C/11 mbar.

Beispiel ZA

4-Methyl-2,8-diazabicyclo[4.3.0]nonan

a) 5-Methyl-1,4-dihydropyridin-2,3-dicarbonsäure-N-benzylimid

33 g (0,29 mol) 2-Methyl-2-propenal-dimethylhydrazon und 55 g (0,29 mol) N-Benzylmaleinimid werden in 225 ml Acetonitril 3 Stunden bei 60°C gerührt. Dann wird das Lösungsmittel am Rotationsverdampfer entfernt, der Rückstand in 600 ml Toluol aufgenommen und unter Zusatz von 150 g Kieselgel 1 Stunde unter Rückflußkühlung gekocht. Dann wird heiß filtriert und das Kieselgel mehrmals mit Ethanol ausgekocht. Die vereinigten organischen Phasen werden am Rotationsverdampfer eingeengt. Es werden 17,5 g (24 % der Theorie) rote Kristalle vom Schmelzpunkt 184-186°C erhalten.

b) 5-Methyl-hexahydropyridin-2,3-dicarbonsäure-N-benzylimid

17,5 g (70 mmol) 5-Methyl-1,4-dihydropyridin-2,3-dicarbonsäure-N-benzylimid werden in 150 ml Tetrahydrofuran bei 70°C und 100 bar über Palladium auf Aktivkohle hydriert. Dann wird der Katalysator abfiltriert und das Filtrat eingeengt. Der ölig-feste Rückstand (13,0 g) wird als Rohprodukt in die nächste Stufe eingesetzt.

c) 8-Benzyl-4-methyl-2,8-diazabicyclo[4.3.0]nonan

13,0 g rohes 5-Methyl-hexahydropyridin-2,3-dicarbonsäure-N-benzylimid werden als Lösung in 50 ml absolutem Tetrahydrofuran zu vorgelegten 4,6 g (0,12 mol) Lithiumaluminiumhydrid in 100 ml absolutem Tetrahydrofuran getropft. Dann wird 17 Stunden unter Rückflußkühlung gekocht. Nacheinander werden 4,6 g Wasser in 14 ml Tetrahydrofuran, 4,6 g 10%ige Natronlauge sowie 13,8 g Wasser zugetropft. Die Salze werden abgesaugt, das Filtrat eingeengt und der Rückstand destilliert.
Ausbeute: 8,7 g (54 % bezogen auf 5-Methyl-1,4-dihydropyridin-2,3-dicarbonsäure-N-benzylimid)
Siedepunkt: 95-98°C/0,1 mbar.

d) 4-Methyl-2,8-diazabicyclo[4.3.0]nonan

8,0 g (35 mmol) 8-Benzyl-4-methyl-2,8-diazabicyclo-[4.3.0]nonan werden in 60 ml Methanol gelöst und bei 100°C und 100 bar über Palladium auf Aktivkohle hydriert. Dann wird der Katalysator abfiltriert, das Filtrat eingeengt und der Rückstand destilliert.
Ausbeute: 3,3 g (67 % der Theorie),
Siedepunkt: 88-89°C/11 mbar.

Das $^1$H-NMR-Spektrum weist die Verbindung als Gemisch zweier Stereoisomere im Verhältnis 7:2 aus.

Beispiel AA

5,6,7,8-Tetrafluor-1-(2,4-difluorphenyl)-1,4-dihydro-4-oxo-3-chinolincarbonsäure

a) 2-(2,3,4,5,6-Pentafluorbenzoyl)-3-(2,4-difluorphenylamino)-acrylsäureethylester

Zu einer Lösung von 115 g 3-Ethoxy-2-(2,3,4,5,6-pentafluorbenzoyl)-acrylsäureethylester in 380 ml Ethanol gibt man unter Eiskühlung und Rühren tropfenweise 44,3 g 2,4-Difluoranilin. Man rührt 1 Stunde bei Raumtemperatur, versetzt unter Eiskühlung mit 380 ml Wasser, saugt den Niederschlag ab, wäscht mit Ethanol/$H_2O$ (1:1) und trocknet. Man erhält 135,4 g der Titelverbindung vom Schmelzpunkt 97-99°C.

b) 5,6,7,8-Tetrafluor-1-(2,4-difluorphenyl)-1,4-dihydro-4-oxo-3-chinolincarbonsäureethylester

Ein Gemisch von 135,4 g 2-(2,3,4,5,6-Pentafluorbenzoyl)-3-(2,4-difluorphenylamino)-acrylsäureethylester, 20,6 g Natriumfluorid und 300 ml wasserfreiem Dimethylformamid wird 3 Stunden auf 140-150°C erhitzt. Die Suspension wird heiß auf 2 kg Eis gegossen, der Niederschlag abgesaugt, mit Wasser gewaschen und getrocknet. Man erhält 122 g der Titelverbindung vom Schmelzpunkt 160-162°C.

c) 5,6,7,8-Tetrafluor-1-(2,4-difluorphenyl)-1,4-dihydro-4-oxo-3-chinolincarbonsäure

Zu einem Gemisch von 28,5 ml konz. Schwefelsäure, 250 ml Eisessig und 200 ml Wasser gibt man 40,1 g 5,6,7,8-Tetrafluor-1-(2,4-difluorphenyl)-1,4-dihydro-4-oxo-3-chinolincarbonsäureethylester und erhitzt 2 Stunden unter Rückfluß. Man gießt die heiße Lösung auf 2 kg Eis, saugt den Niederschlag ab, wäscht mit Wasser und trocknet. Es werden 34,5 g der Titelverbindung vom Schmelzpunkt 250-252°C erhalten.

Beispiel AB

5,7-Dichlor-1-cyclopropyl-6-fluor-1,4-dihydro-4-oxo-3-chinolincarbonsäure

a) (2,4-Dichlor-3,6-difluorbenzoyl)-essigsäureethylester

2,1 g Magnesiumspäne werden in 5 ml wasserfreiem Ethanol suspendiert. Man versetzt mit 0,5 ml Tetrachlorkohlenstoff und tropft, wenn die Reaktion in Gang gekommen ist, ein Gemisch von 14 g Malonsäureethylester, 10 ml abs. Ethanol und 41 ml Toluol zu. Dann wird noch 1,5 Stunden auf 70°C erhitzt, mit Aceton/Trockeneis auf -5°C bis -10°C gekühlt und bei dieser Temperatur eine Lösung von 21,5 g 2,4-Dichlor-3,6-difluorbenzoylchlorid in 30 ml Toluol langsam zugetropft. Man rührt 1 Stunde bei 0°C, läßt über Nacht auf Raumtemperatur kommen und läßt unter Eiskühlung ein Gemisch von 35 ml Eiswasser und 5 ml konzentrierter Schwefelsäure zulaufen. Die Phasen werden getrennt und zweimal mit Toluol nachextrahiert. Die vereinigten Toluollösungen werden einmal mit gesättigter Kochsalzlösung gewaschen, mit $Na_2SO_4$ getrocknet und das Lösungsmittel im Vakuum abgezogen. Man erhält 34,7 g (2,4-Dichlor-3,6-difluorbenzoyl)-malonsäurediethylester als Rohprodukt. Eine Emulsion von 34,7 g rohem (2,4-Dichlor-3,6-difluorbenzoyl)-malonsäurediethylester in 40 ml Wasser wird mit 0,04 g p-Toluolsulfonsäure versetzt. Man erhitzt unter gutem Rühren 3 Stunden zum Sieden, extrahiert die erkaltete Emulsion mehrmals mit Methylenchlorid, wäscht die vereinigten $CH_2Cl_2$-Lösungen einmal mit gesättigter Kochsalzlösung, trocknet mit $Na_2SO_4$ und destilliert das Lösungsmittel im Vakuum ab. Die Fraktionierung des Rückstandes (33,9 g) im Vakuum liefert 13,9 g (2,4-Dichlor-3,6-difluorbenzoyl)-essigsäureethylester vom Siedepunkt 110-115° C/0,05 mbar; $n_D^{25}$: 1,5241.

b) 2-(2,4-Dichlor-3,6-difluorbenzoyl)-3-ethoxy-acrylsäureethylester

13,7 g (2,4-Dichlor-3,6-difluorbenzoyl)-essigsäureethylester werden mit 10,25 g Orthoameisensäuretriethylester und 11,8 g Essigsäureanhydrid 2 Stunden unter Rückfluß erhitzt. Anschließend wird bis 140°C Badtemperatur im Vakuum eingeengt und 15,7 g 2-(2,4-Dichlor-3,6-difluorbenzoyl)-3-ethoxy-acrylsäureethylester als Öl erhalten; $n_D^{25}$: 1,5302.

c) 2-(2,4-Dichlor-3,6-difluorbenzoyl)-3-cyclopropylamino-acrylsäureethylester

15,6 g (2,4-Dichlor-3,6-difluorbenzoyl)-3-ethoxyacrylsäureethylester werden in 50 ml Ethanol gelöst und unter Kühlung 2,75 g Cyclopropylamin zugetropft. Man rührt 1 Stunde bei Raumtemperatur, versetzt unter Eiskühlung mit 50 ml Wasser, saugt ab, wäscht mit Ethanol/$H_2O$ (1:1) nach und trocknet. Es werden 14,1 g 2-(2,4-Dichlor-3,6-difluorbenzoyl)-3-cyclopropylamino-acrylsäureethylester vom Schmelzpunkt 106-107°C erhalten.

d) 5,7-Dichlor-1-cyclopropyl-6-fluor-1,4-dihydro-4-oxo-3-chinolincarbonsäureethylester

6 g 2-(2,4-Dichlor-3,6-difluorbonzoyl)-3-cyclopropylamino-acrylsäureethylester werden in 100 ml Dimethylformamid mit 2,75 g Kaliumcarbonat 2,5 Stunden auf 150°C erhitzt. Die Mischung wird auf 600 ml Eiswasser gegossen, der Niederschlag abgesaugt, mit Wasser gewaschen und getrocknet. Es werden 5,2 g 5,7-Dichlor-1-cyclopropyl-6-fluor-1,4-dihydro-4-oxo-3-chinolincarbonsäureethylester vom Schmelzpunkt 227-229°C erhalten.

e) 5,7-Dichlor-1-cyclopropyl-6-fluor-1,4-dihydro-4-oxo-3-chinolincarbonsäure

5,2 g 5,7-Dichlor-1-cyclopropyl-6-fluor-1,4-dihydro-4-oxo-3-chinolincarbonsäureesthylester werden in einer Mischung aus 38 ml Essigsäure, 30 ml Wasser und 4,3 ml konzentrierter Schwefelsäure 2,5 Stunden unter Rückfluß erhitzt. Nach dem Abkühlen wird auf 250 ml Eiswasser gegossen, der Niederschlag abgesaugt, mit Wasser gewaschen und getrocknet. Es werden 4,8 g 5,7-Dichlor-1-cyclopropyl-6-fluor-1,4-dihydro-4-oxo-3-chinolincarbonsäure vom Schmelzpunkt 277-278°C erhalten.

Beispiel AC

5,7-Dichlor-6-fluor-1-(2,4-difluorphenyl)-1,4-dihydro-4-oxo-3-chinolincarbonsäure

a) 2-(2,4-Dichlor-3,6-difluorbenzoyl)-3-(2,4-difluorphenylamino)-acrylsäureethylester

35,3 g 2-(2,4-Dichlor-3,6-difluorbenzoyl)-3-ethoxyacrylsäureethylester werden in 120 ml Ethanol gelöst und unter Eiskühlung 12,9 g 2,4-Difluoranilin zugetropft. Man rührt 1,5 Stunden bei Raumtemperatur, versetzt unter Kühlung mit 120 ml Wasser, saugt ab, wäscht mit Ethanol/$H_2O$ (1:1) nach und trocknet. Es werden 40,5 g 2-(2,4-Dichlor-3,6-difluorbenzoyl)-3-(2,4-difluorphenylamino)-acrylsäureethylester erhalten. Schmelzpunkt: 84-86°C.

b) 5,7-Dichlor-6-fluor-1-(2,4-difluorphenyl)-1,4-dihydro-4-oxo-3-chinolincarbonsäureethylester

43,6 g 2-(2,4-Dichlor-3,6-difluorbenzoyl)-3-(2,4-difluorphenylamino)-acrylsäureethylester werden in 260 ml Dimethylformamid mit 15,2 g Kaliumcarbonat 2,5 Stunden auf 150°C erhitzt. Die Mischung wird auf 1 Liter Eiswasser gegossen, der Niederschlag abgesaugt, mit Waser gewaschen und getrocknet. Es werden 38,6 g 5,7-Dichlor-6-fluor-1-(2,4-difluorphenyl)-1,4-dihydro-4-oxo-3-chinolincarbonsäureethylester erhalten.

c) 5,7-Dichlor-6-fluor-1-(2,4-difluorphenyl)-1,4-dihydro-4-oxo-3-chinolincarbonsäure

41,6 g 5,7-Dichlor-6-fluor-1-(2,4-difluorphenyl)-1,4-dihydro-4-oxo-3-chinolincarbonsäureethylester werden mit 250 ml Essigsäure, 200 ml Wasser und 28,5 ml konzentrierter Schwefelsäure 3 Stunden unter Rückfluß erhitzt. Nach dem Abkühlen wird auf 2 Liter Eiswasser gegossen, der Niederschlag abgesaugt, mit Wasser gewaschen und getrocknet. Es werden 35,5 g 5,7-Dichlor-6-fluor-1-(2,4-difluorphenyl)-1,4-dihydro-4-oxo-3-chinolincarbonsäure erhalten. Schmelzpunkt: 244-246°C.

Beispiel 1

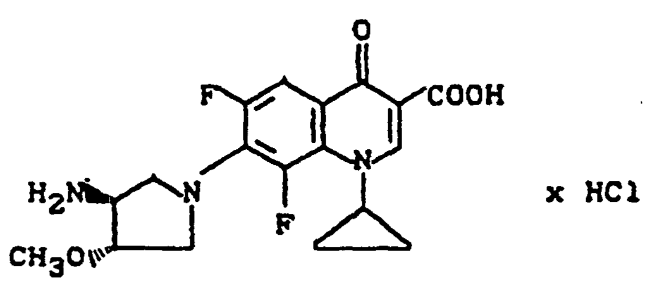

A. 855 mg (3 mmol) 1-Cyclopropyl-6,7,8-trifluor-1,4-dihydro-4-oxo-3-chinolincarbonsäure werden in einer Mischung aus 9 ml Acetonitril und 4,5 ml Dimethylformamid in Gegenwart von 330 mg (3,3 mmol) 1,4-Diazabicyclo [2.2.2]octan und 750 mg trans-3-tert.-Butoxycarbonyl-amino-4-methoxy-pyrrolidin 1 Stunde unter Rückfluß erhitzt. Die Mischung wird eingedampft, mit Wasser verrührt und getrocknet.
Ausbeute: 1,3 g (90,5% der Theorie) 7-(trans-3-tert.-Butoxycarbonylamino-4-methoxy-1-pyrrolidinyl)-1-cyclopropyl-6,8-difluor-1,4-dihydro-4-oxo-3-chinolincarbonsäure.
Schmelzpunkt: 222-224°C (unter Zersetzung) (aus Glykolmonomethylether).

B. 1,2 g (3,5 mmol) des Produktes aus Stufe A werden in 10 ml 3n-Salzsäure eingetragen, bis zur Lösung gerührt und eingeengt. Der Rückstand wird mit Ethanol verrieben, abgesaugt und bei 60° im Hochvakuum getrocknet.
Ausbeute: 0,73 g (70% der Theorie) 7-(trans-3-Amino-4-methoxy-1-pyrrolidinyl)-1-cyclopropyl-6,8-difluor-4-oxo-3-chinolincarbonsäure-Hydrochlorid.
Schmelzpunkt: 279° C (unter Zersetzung).

Beispiel 2

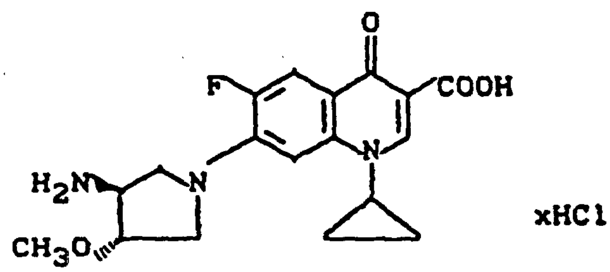

Analog Beispiel 1 setzt man 1-Cyclopropyl-6,7-difluor-1,4-dihydro-4-oxo-3-chinolincarbonsäure um und erhält:

A. 7-(trans-3-tert.-Butoxycarbonylamino-4-methoxy-1-pyrrolidinyl)-1-cyclopropyl-6-fluor-1,4-dihydro-4-oxo-3-chinolincarbonsäure, Schmelzpunkt: 247-249°C (unter Zersetzung).

B. 7-(trans-3-Amino-4-methoxy-1-pyrrolidinyl)-1-cyclopropyl-6-fluor-4-oxo-3-chinolincarbonsäure-Hydrochlorid, Schmelzpunkt: ab 293°C (unter Zersetzung).

Beispiel 3

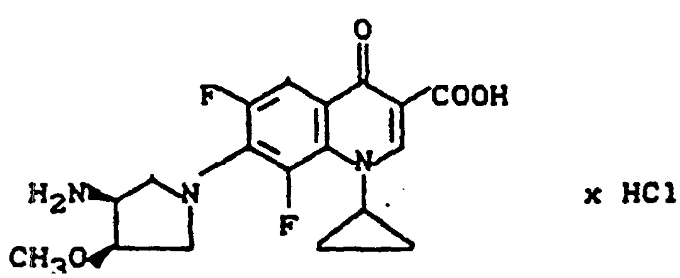

Analog Beispiel 1 wird mit cis-3-tert.-Butoxycarbonylamino-4-methoxy-pyrrolidin umgesetzt zu:

A. 7-(cis-3-tert.-Butoxycarbonylamino-4-methoxy-1-pyrrolidinyl)-1-cyclopropyl-6,8-difluor-1,4-dihydro-4-oxo-3-chinolincarbonsäure, Schmelzpunkt: 230-231°C (unter Zersetzung).

B. 7-(cis-3-Amino-4-methoxy-1-pyrrolidinyl)-1-cyclopropyl-6,8-difluor-4-oxo-3-chinolincarbonsäure-Hydrochlorid, Schmelzpunkt 201-203°C (unter Zersetzung).

Beispiel 4

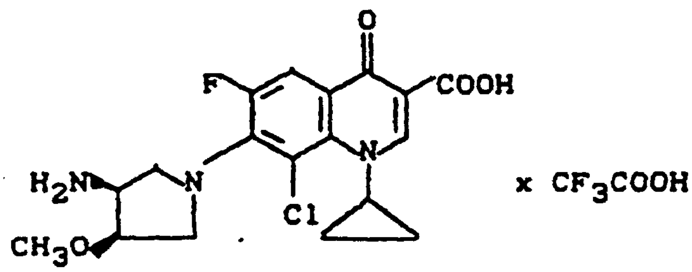

A. 1,5 g (5 mmol) 8-Chlor-1-cyclopropyl-6,7-difluor-1,4-dihydro-4-oxo-3-chinolincarbonsäure werden in einer Mischung aus 10 ml Acetonitril und 5 ml Dimethylformamid mit 550 mg (5 mmol) 1,4-Diazabicyclo[2.2.2]octan und 1,2 g (5,6 mmol) cis-3-tert.-Butoxycarbonylamino-4-mothoxy-pyrrolidin 2 Stunden unter Rückfluß erhitzt. Man läßt abkühlen, saugt den ausgefallenen Niederschlag ab, wäscht gut mit Wasser nach und trocknet bei 100°C im Vakuum.
Ausbeute: 2,0 g (80,7%) 7-(cis-3-tert.-Butoxycarbonylamino-4-methoxy-1-pyrrolidinyl)-8-chlor-1-cyclopropyl-6-fluor-1,4-dihydro-4-oxo-3-chinolincarbonsäure, Schmelzpunkt: 222-225°C (unter Zersetzung).

B. 1,9 g (3,8 mmol) des Produktes aus Stufe A werden in 10 ml Trifluoressigsäure 20 Minuten bei Raumtemperatur gerührt, die Lösung eingeengt, das zurückbleibende Öl zweimal mit Dichlormethan abgedampft und der Rückstand mit Ether verrührt. Der ausgefallene Niederschlag wird abgesaugt, mit Ether gewaschen und bei 60°C im Vakuum getrocknet.
Ausbeute: 1,9 g (97% der Theorie) 7-(cis-3-Amino-4-methoxy-1-pyrrolidinyl)-8-chlor-1-cyclopropyl-6-fluor-1,4-dihydro-4-oxo-3-chinolincarbonsäure-Trifluoracetat, Schmelzpunkt 235-239°C (unter Zersetzung).

Beispiel 5

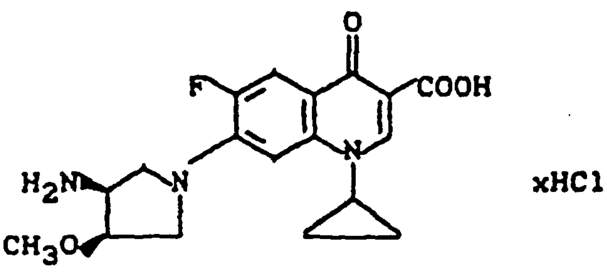

Analog Beispiel 1 wird cis-3-tert.-Butoxycarbonylamino-4-methoxy-pyrrolidin mit 1-Cyclopropyl-6,7-difluor-1,4-dihydro-4-oxo-3-chinolincarbonsäure umgesetzt zu:

A. 7-(cis-3-tert.-Butoxycarbonylamino-4-methoxy-1-pyrrolidinyl)-1-cyclopropyl-6-fluor-1,4-dihydro-4-oxo-3-chinolincarbonsäure, Schmelzpunkt 232-233°C (unter Zersetzung).

B. 7-(cis-3-Amino-4-methoxy-1-pyrrolidinyl)-1-cyclopropyl-6-fluor-1,4-dihydro-4-oxo-3-chinolincarbonsäure-Hydrochlorid, Schmelzpunkt 252-256°C (unter Zersetzung) (vorher Sintern).

Beispiel 6

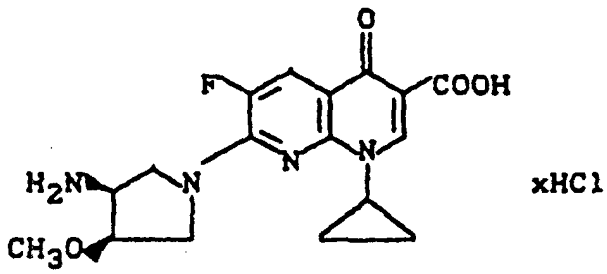

Analog Beispiel 1 wird cis-3-tert.-Butoxycarbonylamino-4-methoxypyrrolidin mit 7-Chlor-1-cyclopropyl-6-fluor-1,4-dihydro-4-oxo-1,8-naphthyridin-3-carbonsäure umgesetzt. zu:

A. 7-(cis-tert.-Butoxycarbonylamino-4-methoxy-1-pyrrolidinyl)-1-cyclopropyl-6-fluor-1,4-dihydro-4-oxo-1,8-naphthyridin-3-carbonsäure, Schmelzpunkt 214-216°C (unter Zersetzung).

B. 7-(cis-3-Amino-4-methoxy-1-pyrrolidinyl)-1-cyclopropyl-6-fluor-1,4-dihydro-4-oxo-1,8-naphthyridin-3-carbonsäure-Hydrochlorid, Schmelzpunkt 205-210° (unter Zersetzung).

Massenspektrum: $^{m}/_{e}$ 362 ($M^+$), 330 ($M^+$-32), 318 ($M^+$-$CO_2$), 286, 260, 41 ($C_3H_5$), 36 (HCl).

Beispiel 7

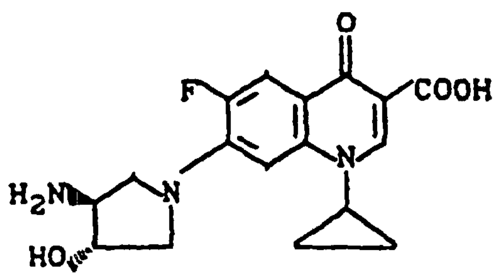

1,33 g (5 mmol) 1-Cyclopropyl-6,7-difluor-1,4-dihydro-4-oxo-3-chinolincarbonsäure werden in einer Mischung aus 30 ml Acetonitril und 5 ml Dimethylformamid mit 1,1 g (10 mmol) 1,4-Diazabicyclo[2.2.2]octan und 0,55 g (5,4 mmol) trans-3-Amino-4-hydroxy-pyrrolidin versetzt und 1 Stunde unter Rückfluß erhitzt. Die Suspension wird eingeengt, der Rückstand mit Wasser versetzt, das ungelöste Produkt abgesaugt und aus Dimethylformamid umkristallisiert. Ausbeute: 1,2 g (73% der Theorie) 7-(trans-3-Amino-4-hydroxy-1-pyrrolidinyl)-1-cyclopropyl-6-fluor-1,4-dihydro-4-oxo-3-chinolincarbonsäure,
Schmelzpunkt: 274-278°C (unter Zersetzung).

Beispiel 8

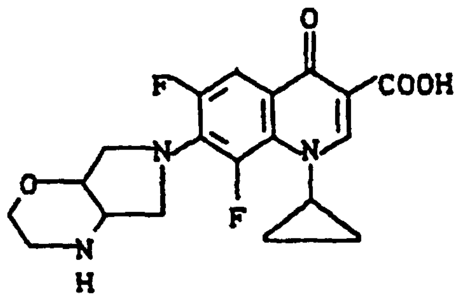

850 mg (3 mmol) 1-Cyclopropyl-6,7,8-trifluor-1,4-dihydro-4-oxo-3-chinolincarbonsäure werden in 9 ml Pyridin mit 630 mg (3,1 mmol) 2-Oxa-5,8-diazabicyclo[4.3.0]nonan-Dihydrochlorid und 500 mg (4,5 mmol) 1,4-Diazabicyclo [2.2.2]octan 1 Stunde unter Rückfluß erhitzt. Die Mischung wird eingeengt, der Rückstand mit Wasser verrührt, der Niederschlag abgesaugt, mit Wasser gewaschen, getrocknet und aus Glykolmonomethylether umkristallisiert.
Ausbeute: 840 mg (72% der Theorie) 1-Cyclopropyl-6,8-difluor-1,4-dihydro-7-(2-oxa-5,8-diazabicyclo[4.3.0]non-8-yl)-4-oxo-3-chinolincarbonsäure,
Schmelzpunkt: 289-291°C (unter Zersetzung); Massenspektrum: m/e 391 ($M^+$), 347 ($M^+$-$CO_2$), 331, 306, 294, 262, 234, 98, 41 ($C_3H_5$).

Beispiel 9

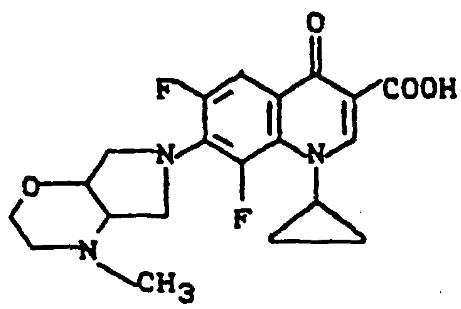

Analog Beispiel 8 setzt man mit 5-Methyl-2-oxa-5,8-diazabicyclo[4.3.0]nonan-Dihydrochlorid um und erhält: 1-Cyclopropyl-6,8-difluor-1,4-dihydro-7-(5-methyl-2-oxa-5,8-diazabicyclo[4.3.0]non-8-yl)-4-oxo-3-chinolincarbonsäure, Schmelzpunkt: ab 270°C (unter Zersetzung): Massenspektrum: $^m/e$ 405 ($M^+$), 361 ($M^+$-$CO_2$), 331, 112, (100%).

Beispiel 10

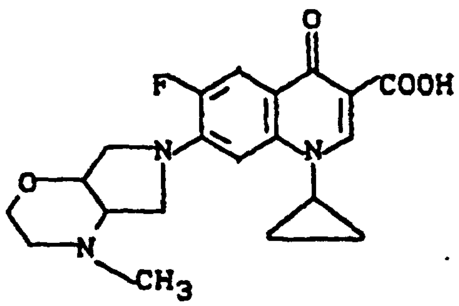

795 mg (3 mmol) 1-Cyclopropyl-6,7-difluor-1,4-dihydro-4-oxo-3-chinolincarbonsäure werden in einer Mischung aus 9 ml Acetonitril und 4,5 ml Dimethylformamid mit 890 mg (4,1 mmol) 5-Methyl-2-oxa-5,8-diazabicyclo[4.3.0] nonan-Dihydrochlorid und 860 mg (7,8 mmol) 1,4-Diazabicyclo-[2.2.2]octan 2 Stunden unter Rückfluß erhitzt. Die Mischung wird eingedampft, mit Wasser verrührt, das ungelöste Produkt wird abgesaugt, mit Wasser gewaschen, getrocknet und aus Dimethylformamid umkristallisiert. Ausbeute: 0,8 g (69% der Theorie) 1-Cyclopropyl-6-fluor-1,4-dihydro-7-(5-methyl-2-oxa-5,8-diazabicyclo[4.3.0]-non-8-yl)-4-oxo-3-chinolincarbonsäure, Schmelzpunkt 340°C (unter Zersetzung) (die Substanz wird bereits beim Aufheizen ab etwa 300° dunkel).
Massenspektrum: $^{m}/e$ ($M^+$), 343 ($M^+$-$CO_2$), 313, 244, 112, (100%).

Beispiel 11

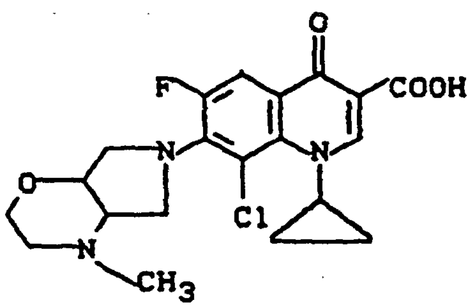

Analog Beispiel 10 setzt man mit 8-Chlor-1-cyclopropyl-6,7-difluor-1,4-dihydro-4-oxo-3-chinolincarbonsäure um und erhält 8-Chlor-1-cyclopropyl-6-fluor-1,4-dihydro-7-(5-methyl-2-oxa-5,8-diazabicyclo[4.3.0]non-8-yl)-4-oxo-3-chinolincarbonsäure, Schmelzpunkt 258-262°C (unter Zersetzung) (umkristallisiert aus Dimethylformamid).

Beispiel 12

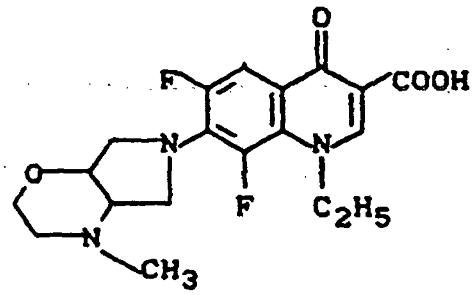

Analog Beispiel 10 setzt man mit 1-Ethyl-6,7,8-trifluor-1,4-dihydro-4-oxo-3-chinolincarbonsäure um und erhält 1-Ethyl-6,8-difluor-1,4-dihydro-7-(5-methyl-2-oxa-5,8-diazabicyclo[4.3.0]non-8-yl)-4-oxo-3-chinolincarbonsäure, Schmelzpunkt 279-281°C (unter Zersetzung),

Beispiel 13

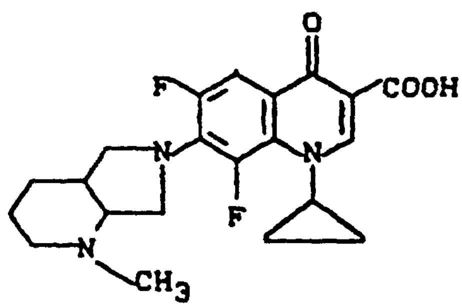

0,84 g (3 mmol) 1-Cyclopropyl-6,7,8-trifluor-1,4-dihydro-4-oxo-3-chinolincarbonsäure werden in einer Mischung aus 6 ml Acetonitril und 3 ml Dimethylformamid mit 0,66 g (6 mmol) 1,4-Diazabicyclo[2.2.2]octan und 0,49 g (3,5 mmol) 2-Methyl-2,8-diazabicyclo[4.3.0]nonan 2 Stunden unter Rückfluß erhitzt. Die Suspension wird eingeengt, mit 20 ml Wasser verrührt, mit 2n-Salzsäure auf pH 7 eingestellt, der Niederschlag wird abgesaugt, mit Wasser gewaschen, getrocknet und aus Glykolmonomethylether umkristallisiert.
Ausbeute: 0,7 g (58% der Theorie) 1-Cyclopropyl-6,8-difluor-1,4-dihydro-7-(2-methyl-2,8-diazabicyclo[4.3.0]-non-8-yl)-4-oxo-3-chinolincarbonsäure, Schmelzpunkt 204-207°.

Beispiel 14

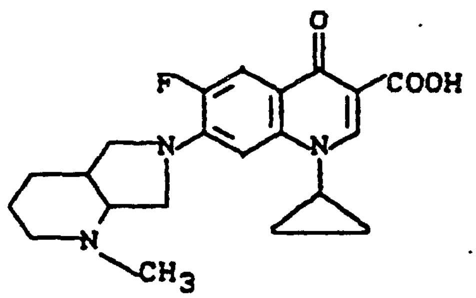

Analog Beispiel 13 erhält man mit 1-Cyclopropyl-6,7-difluor-1,4-dihydro-4-oxo-3-chinolincarbonsäure 1-Cyclopropyl-6-fluor-1,4-dihydro-7-(2-methyl-2,8-diazabicyclo[4.3.0]non-8-yl)-4-oxo-3-chinolincarbonsäure, Schmelzpunkt 234-236°.

Beispiel 15

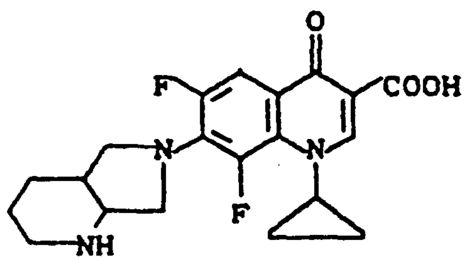

A. Man setzt analog Beispiel 13 1-Cyclopropyl-6,7,8-trifluor-1,4-dihydro-4-oxo-3-chinolincarbonsäure mit 2,8-Diazabicyclo[4.3.0]nonan um und erhält 1-Cyclopropyl-7-(2,8-diazabicyclo[4.3.0]non-8-yl)-6,8-difluor-1,4-dihydro-4-oxo-3-chinolincarbonsäure, Schmelzpunkt 265-267° (unter Zersetzung) (umkristallisiert aus Dimethylformamid).

B. Führt man die Umsetzung des Beispiels 15 A) in einer Mischung aus Acetonitril/1-Methyl-2-pyrrolidinon durch und kristallisiert das Rohprodukt aus Dimethylformamid um, dann erhält man 1-Cyclopropyl-7-(2,8-diazabicyclo[4.3.0]non-8-yl)-6,8-difluor-1,4-dihydro-4-oxo-3-chinolincarbonsäure vom Schmelzpunkt 269-271°C (unter Zersetzung). Das Produkt ist nach chromatographischem und spektroskopischem Vergleich identisch mit dem nach Verfahren A) hergestelltem Produkt.

C. 65 g (167 mmol) des Betains (Stufe A) worden in 330 ml halbkonzentrierter Salzsäure durch Erwärmen gelöst, die Lösung wird eingeengt und der Rückstand mit 300 ml Ethanol verrührt. Der ungelöste Niederschlag wird abgesaugt, mit Ethanol gewaschen und bei 100°C in Vakuum getrocknet.

Ausbeute: 66,3 g (93 % der Theorie) 1-Cyclopropyl-7-(2,8-diazabicyclo[4.3.0]non-8-yl)-6,8-difluor-1,4-dihydro-4-oxo-3-chinolincarbonsäure-hydrochlorid, Schmelzpunkt: 303-305°C (unter Zersetzung).

Beispiel 16

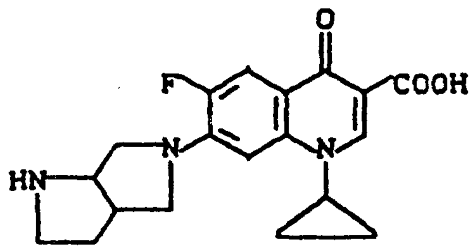

Analog Beispiel 13 erhält man mit 1-Cyclopropyl-6,7-difluor-1,4-dihydro-4-oxo-3-chinolincarbonsäure und 2,7-Diazabicyclo[3.3.0]octan 1-Cyclopropyl-7-(2,7-diazabicyclo[3.3.0]oct-7-yl)-6-fluor-1,4-dihydro-4-oxo-3-chinolincarbonsäure, Schmelzpunkt: 260-282° (unter Zersetzung).

Massenspektrum: $^{m}/e$ 357 ($M^+$), 313 (100%, $M^+$-$CO_2$), 269, 257, 244, 82, 28.

Beispiel 17

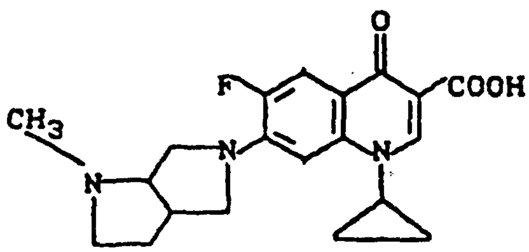

Analog Beispiel 13 erhält man mit 1-Cyclopropyl-6,7-difluor-1,4-dihydro-4-oxo-3-chinolincarbonsäure und 2-Methyl-2,7-diazabicyclo[3.3.0]octan 1-Cyclopropyl-6-fluor-1,4-dihydro-7-(2-methyl-2,7-diazabicyclo[3.3.0]-oct-7-yl)-4-oxo-3-chinolincarbonsäure, Schmelzpunkt: 206-208°C (unter Zersetzung).

Beispiel 18

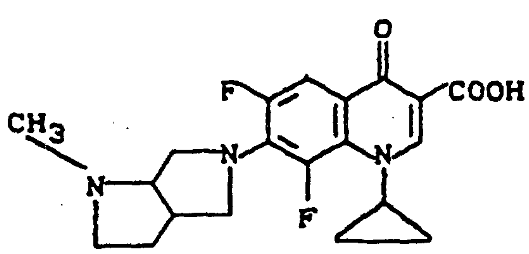

Analog Beispiel 13 erhält man mit 2-Methyl-2,7-diazabicyclo[3.3.0]octan 1-Cyclopropyl-6,8-difluor-1,4-dihydro-7-(2-methyl-2,7-diazabicyclo[3.3.0]oct-7-yl)-4-oxo-3-chinolincarbonsäure, Schmelzpunkt 198-200°C (unter Zersetzung).

Beispiek 19

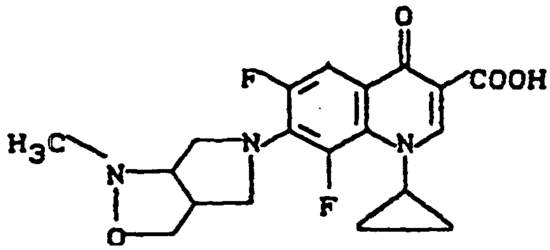

Eine Mischung aus 2,83 g (10 mmol) 1-Cyclopropyl-6,7,8-trifluor-1,4-dihydro-4-oxo-3-chinolincarbansäure, 1,1 g (10 mmol) 1,4-Diazabicyclo[2.2.2]octan und 1,4 g (11 mmol) 2-Methyl-3-oxa-2,7-diazabicyclo[3.3.0]octan in 20 ml Acetonitril und 10 ml 1-Methyl-2-pyrrolidinon wird 1 Stunde unter Rückfluß erhitzt. Man engt im Vakuum ein, verrührt den Rückstand mit Wasser (pH 7), saugt den Niederschlag ab, wäscht mit Wasser und trocknet bei 60° im Vakuum. Das Rohprodukt (3,7 g) wird aus Dimethylformamid umkristallisiert.

Ausbeute: 1,9 g (49% der Theorie) 1-Cyclopropyl-6,8-difluor-1,4-dihydro-7-(2-methyl-3-oxa-2,7-diazabicyclo [3.3.0]oct-7-yl)-4-oxo-3-chinolincarbonsäure, Schmelzpunkt 221-223°C (unter Zersetzung).

Beispiel 20

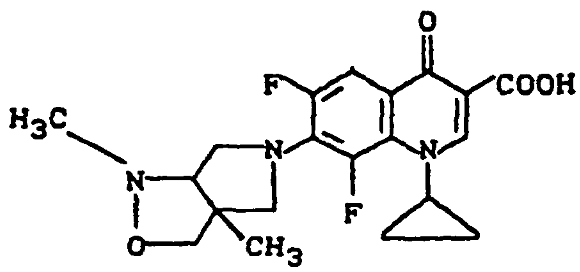

Analog Beispiel 19 wird mit 2,5-Dimethyl-3-oxa-2,7-diazabicyclo[3.3.0]octan zu 1-Cyclopropyl-6,8-difluor-1,4-dihydro-7-(2,5-dimethyl-3-oxa-2,7-diazabicyclo[3.3.0]-oct-7-yl)-4-oxo-3-chinolincarbonsäure vom Schmelzpunkt 237-238°C (unter Zersetzung) umgesetzt.

Beispiel 21

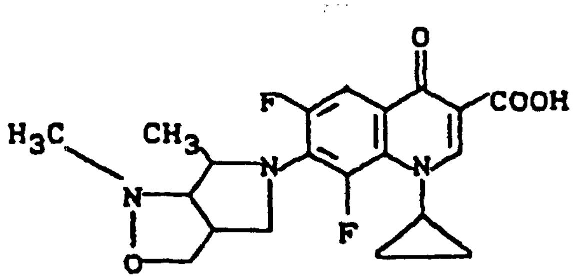

Analog Beispiel 19 wird mit 2,8-Dimethyl-3-oxa-2,7-diazabicyclo[3.3.0]octan zu 1-Cyclopropyl-6,8-difluor-1,4-dihydro-7-(2,8-dimethyl-3-oxa-2,7-diazabicyclo[3.3.0]-oct-7-yl]-4-oxo-3-chinolincarbonsäure vom Schmelzpunkt.

197-199°C umgesetzt.

Beispiel 22

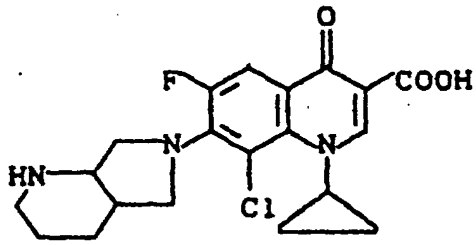

A. 3 g (10 mmol) 8-Chlor-1-cyclopropyl-6,7-difluor-1,4-dihydro-4-oxo-3-chinolincarbonsäure werden in einer Mischung aus 30 ml Acetonitril und 15 ml 1-Methyl-2-pyrrolidinon mit 1,4 g (11 mmol) 2,8-Diazabicyclo-[4.3.0]nonan und 1,65g(15 mmol) 1,4-Diazabicyclo[2.2.2]-octan 1 Stunde unter Rückfluß erhitzt. Die Suspension wird nach dem Abkühlen mit etwa 150 ml Wasser verrührt, der ungelöste Niederschlag abgesaugt, mit Wasser und Ethanol gewaschen und bei 80°C/12 mbar getrocknet. Das Rohprodukt wird aus 40 ml Glykolmonomethylether umkristallisiert.

Ausbeute: 2,3 g (57 % der Theorie) 8-Chlor-1-cyclopropyl-7-(2,8-diazabicyclo[4.3.0]non-8-yl)-6-fluor-1,4-dihydro-4-oxo-3-chinolincarbonsäure, Schmelzpunkt: 224-226°C (unter Zersetzung).

B. Man stellt analog Beispiel 22 A. das rohe Betain her, suspendiert dieses in 50 ml Wasser und bringt es durch Zugabe von 17 ml 1n-Salzsäure und Erwärmen in Lösung. Nach dem Abkühlen im Eisbad wird der ausgefallene Niederschlag abgesaugt, mit Ethanol gewaschen und bei 100°C im Vakuum getrocknet.

Ausbeute: 2,7 g (61 % der Theorie) 8-Chlor-1-cyclopropyl-7-(2,8-diazabicyclo[4.3.0]non-8-yl)-6-fluor-1,4-dihydro-4-oxo-3-chinolincarbonsäure-hydrochlorid,

Schmelzpunkt: ab 225°C Zersetzung.

Beispiel 23

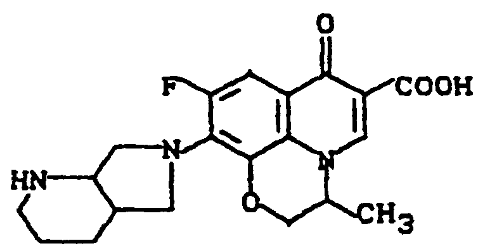

Analog Beispiel 22 wird die Umsetzung mit 9,10-Difluor-2,3-dihydro-3-methyl-7-oxo-7H-pyrido[1,2,3-de][1,4]benzoxacin-6-carbonsäure durchgeführt und das erhaltene Reaktionsprodukt durch Chromatographie an Kieselgel mit Dichlormethan/Methanol/17 %iger wäßriger Ammoniaklösung (30:8:1) als Laufmittel gereinigt. Man erhält 10-(2,8-Diazabicyclo[4,3,0]non-8-yl)-9-fluor-2,3-dihydro-3-methyl-7-oxo-7H-pyrido[1,2,3-de][1,4]benzoxacin-6-carbonsäure vom Schmelzpunkt 291-292°C (unter Zersetzung).

Beispiel 24

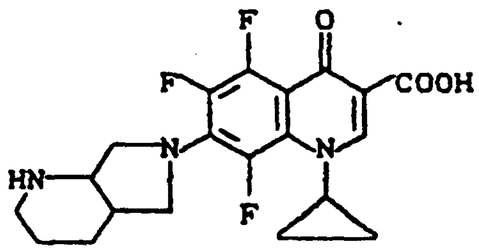

6 g (20 mmol) 1-Cyclopropyl-5,6,7,8-tetrafluor-1,4-dihydro-4-oxo-3-chinolincarbonsäure werden in 30 ml 1-Methyl-2-pyrrolidinon und 60 ml Acetonitril mit 2,2 g (20 mmol) 1,4-Diazabicyclo[2.2.2]octan und 2,7 g (21,4 mmol) 2,8-Diazabicyclo[4.3.0]nonan 1 Stunde unter Rückfluß erhitzt. Die Mischung wird im Vakuum weitgehend eingeengt, der Rückstand mit 200 ml Wasser verrührt, das ungelöste Kristallisat abgesaugt, mit Wasser gewaschen und getrocknet.
Ausbeute: 6,3 g (77,4 % der Theorie) 1-Cyclopropyl-7-(2,8-diazabicyclo[4.3.0]non-8-yl]-5,6,8-trifluor-1,4-dihydro-4-oxo-3-chinolincarbonsäure
Schmelzpunkt: 266-269°C (unter Zersetzung); nach Umkristallisation aus Dimethylformamid : Schmelzpunkt: 272-273°C (unter Zersetzung).

Beispiel 25

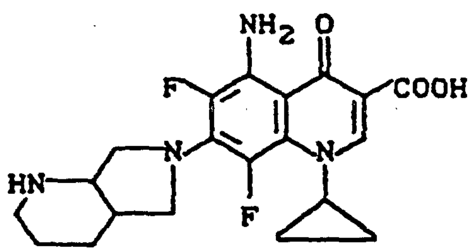

4,1 g (10 mmol) des Produktes aus Beispiel 24 werden in 40 ml Pyridin mit 20 ml gesättigter ethanolischer Ammoniak-Lösung versetzt und die Mischung im Autoklaven 12 Stunden auf 120°C erhitzt. Die Suspension wird eingedampft, der Rückstand mit Wasser verrührt und mit 2n-Salzsäure auf pH 7 eingestellt. Der ausgefallene Niederschlag wird abgesaugt und aus Glykolmonomethylether umkristallisiert.
Ausbeute: 0,7 g (17 % der Theorie) 5-Amino-1-cyclopropyl-7-(2,8-diazabicyclo[4.3.0]non-8-yl)-6,8-difluor-1,4-dihydro-4-oxo-3-chinolincarbonsäure, Schmelzpunkt: 275-277°C (unter Zersetzung).
Massenspektrum: $^{m}/e$ 404 ($M^+$), 384 ($M^+$-HF), 290, 249, 96 (100 %).

Beispiel 26

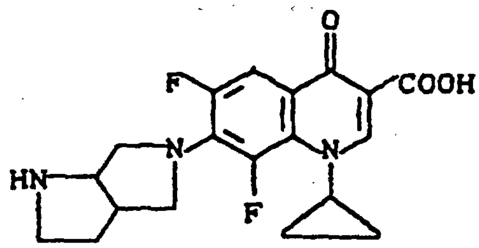

A. Analog Beispiel 13 erhält man mit 2,7-Diazabicyclo[3.3.0]octan 1-Cyclopropyl-7-(2,7-diazabicyclo-[3.3.0]oct-7-yl)-6,8-difluor-1,4-dihydro-4-oxo-3-chinolincarbonsäure, Schmelzpunkt: 277-260° (unter Zersetzung).

B. 370 mg des Betains werden in 13 ml halbkonzentrierter Salzsäure gelöst, die Lösung eingeengt und der Rückstand mit 10 ml Ethanol behandelt. Das ungelöste Produkt wird abgesaugt, mit Ethanol gewaschen und getrocknet. Ausbeute: 290 mg 1-Cyclopropyl-7-(2,7-diazabicyclo-[3.3.0]oct-7-yl)-6,8-difluor-1,4-dihydro-4-oxo-3-chinolincarbonsäure-hydrochlorid, Schmelzpunkt: 269-271°C (unter Zersetzung).

Beispiel 27

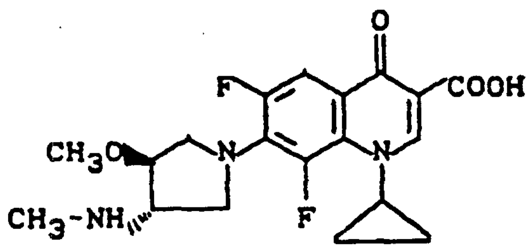

Analog Beispiel 8 wird mit trans-4-Methoxy-3-methylamino-pyrrolidin-Dihydrochlorid umgesetzt. Man erhält 1-Cyclopropyl-6,8-difluor-1,4-dihydro-7-(trans-4-methoxy-3-methylamino-1-pyrrolidinyl)-4-oxo-3-chinolincarbonsäure, Schmelzpunkt: 268-270°C (unter Zersetzung).

Beispiel 28

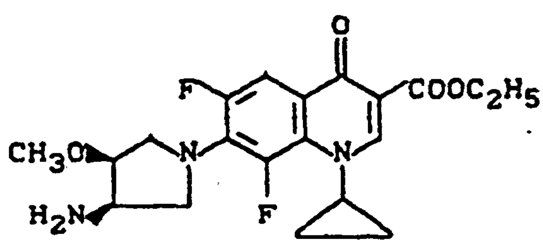

$xCF_3COOH$

A. 1,4 g (2,9 mmol) Produkt aus Beispiel 3 A) und 1,98 ml (1,7 g, 12 mmol) Dimethylformamid-diethylacetal werden in 15 ml absolutem Dimethylformamid 2 Stunden auf 120°C erhitzt. Danach engt man im Vakuum ein. Der verbleibende Rückstand wird mit Acetonitril verrührt. Der Niederschlag wird abgesaugt, mit wenig Acetonitril gewaschen und getrocknet.
Ausbeute: 0,8 g (54,4 % der Theorie) 7-(cis-3-tert.-Butoxycarbonylamino-4-methoxy-1-pyrrolidinyl)-1-cyclopropyl-6,8-difluor-1,4-dihydro-4-oxo-3-chinolincarbonsäureethylester,
Schmelzpunkt: 151-152°C.

B. 0,3 g (0,6 mmol) Produkt aus Beispiel 28 A) werden in 10 ml Trifluoressigsäure 10 Minuten bei 20°C gerührt. Anschließend wird die Trifluoressigsäure im Vakuum entfernt. Der Rückstand wird bei Zugabe von Diethylether fest. Der Feststoff wird isoliert, mit Diethylether gewaschen und getrocknet.
Ausbeute: 0,25 g (80,6 % der Theorie) 7-(cis-3-Amino-4-methoxy-1-pyrrolidinyl)-1-cyclopropyl-6,8-difluor-1,4-dihydro-4-oxo-3-chinolincarbonsäureethylester-trifluoracetat
Schmelzpunkt: 124-126°C.

Beispiel 29

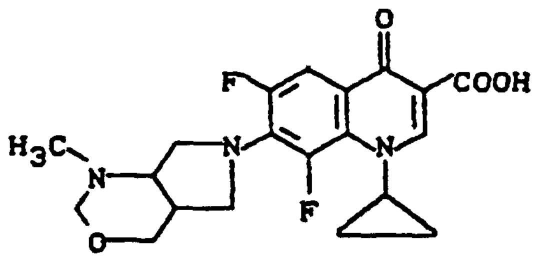

Analog Beispiel 13 erhält man mit 2-Methyl-4-oxo-2,8-diazabicyclo[4.3.0]nonan 1-Cyclopropyl-6,8-difluor-1,4-dihydro-7-(2-methyl-4-oxa-2,8-diazabicyclo[4.3.0]non-8-yl)-4-oxo-3-chinolincarbonsäure, Schmelzpunkt 258-260°C (unter Zersetzung).

Beispiel 30

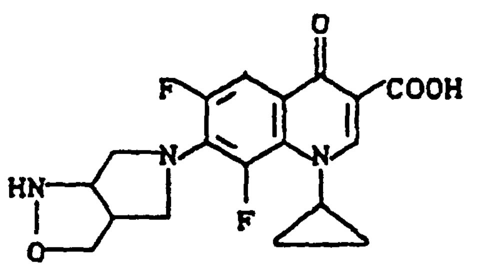

Analog Beispiel 19 erhält man mit 3-Oxa-2,7-diazabicyclo[3.3.0]octan 1-Cyclopropyl-6,8-difluor-1,4-dihydro-7-(3-oxa-2,7-diazabicyclo[3.3.0]octan-7-yl)-4-oxo-3-chinolincarbonsäure.

Beispiel 31

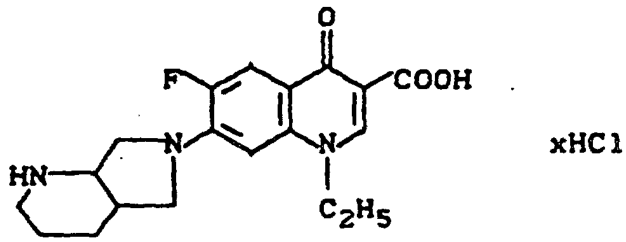

A. 2,53 g (10 mmol) 1-Ethyl-6,7-difluor-1,4-dihydro-4-oxo-3-chinolincarbonsäure werden in 30 ml Acetonitril und 15 ml Dimethylformamid mit 1,1 g (10 mmol) 1,4-Diazabicyclo[2.2.2]octan und 1,4 g (11 mmol) 2,8-Diazabicyclo [4.3.0]nonan versetzt und 1 Stunde unter Rückfluß erhitzt. Die Mischung wird eingeengt, mit Wasser ver rührt und der Niederschlag abgesaugt, mit Wasser gewaschen und getrocknet.
Ausbeute: 3,1 g (86 % der Theorie) 7-(2,8-Diazabicyclo[4.3.0]non-8-yl)-1-ethyl-6-fluor-4-oxo-3-chinolincarbonsäure, Schmelzpunkt: 259-261°C (unter Zersetzung).

B. 2,9 g (8 mmol) des Betains aus Stufe A werden in 20 ml halbkonzentrierter Salzsäure in der Wärme gelost, die Lösung heiß filtriert und aus dem Filtrat durch Zusatz von Ethanol das Hydrochlorid ausgefällt. Dieses wird abgesaugt, mit Ethanol gewaschen und bei 120°C/12 mbar getrocknet.
Ausbeute: 1,8 g (57 % der Theorie) 7-(2,8-Diazabicyclo-[4.3.0]non-8-yl)-1-ethyl-6-fluor-4-oxo-3-chinolincarbonsäure-Hydrochlorid, Schmelzpunkt unter Zersetzung: 299°C (bereits ab ca. 215°C beginnende Dunkelfärbung).

Beispiel 32

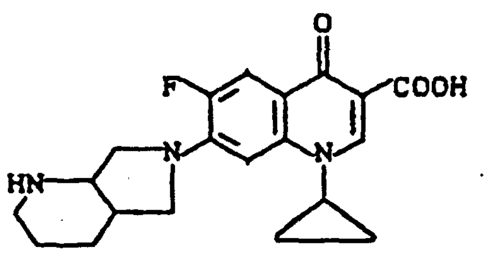

Analog Beispiel 31 erhält man mit 1-Cyclopropyl-6,7-difluor-1,4-dihydro-4-oxo-3-chinolincarbonsäure:

A. 1-Cyclopropyl-7-(2,8-diazabicyclo[4.3.0]non-8-yl)-6-fluor-4-oxo-3-chinolincarbonsäure, Schmelzpunkt: 249-257°C (unter Zersetzung)

B. 1-Cyclopropyl-7-(2,8-diazabicyclo[4.3.0]non-8-yl)-6-fluor-4-oxo-3-chinolincarbonsäure-Hydrochlorid, Schmelzpunkt unter Zersetzung: 320°C (bereits ab ca. 288°C beginnende Dunkelfärbung).

Beispiel 33

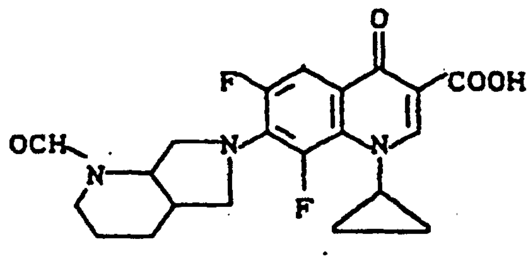

1,1 g (3 mmol) 1-Cyclopropyl-7-(2,8-diazabicyclo[4.3.0]-non-8-yl)-6,8-difluor-1,4-dihydro-4-oxo-3-chinolincarbonsäure werden in 10 ml Dimethylformamid und 1 ml Ameisensäure 4 Stunden unter Rückfluß erhitzt. Die Mischung wird eingedampft, der Rückstand mit 4 ml Wasser verrührt, der Niederschlag abgesaugt, getrocknet (Rohausbeute: 1 g, Gehalt: 99,5 %) und aus Dimethylformamid umkristallisiert,
Ausbeute: 0,8 g (64 % der Theorie) 1-Cyclopropyl-6,8-difluor-7-(2-formyl-2,8-diazabicyclo[4.3.0]non-8-yl)-1,4-dihydro-4-oxo-3-chinolincarbonsäure, Schmelzpunkt: 276-278°C.

Beispiel 34

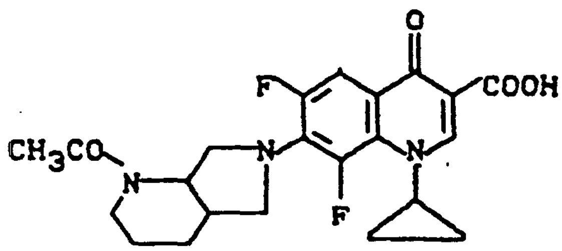

1,1 g (3 mmol) 1-Cyclopropyl-7-(2,8-diazabicyclo[4.3.0]-non-8-yl)-6,8-difluor-1,4-dihydro-4-oxo-3-chinolincarbonsäure werden in einer Mischung aus 8 ml Dioxan und einer Lösung von 120 mg Natriumhydroxid in 1 ml Wasser gelöst und unter Eiskühlung gleichzeitig mit 3 ml ln-Natronlauge und 260 mg Acetylchlorid versetzt. Man läßt 2 Stunden bei Raumtemperatur nachrühren, verdünnt mit 30 ml Wasser und saugt den ausgefallenen Niederschlag ab. Das Rohprodukt wird aus Glykolmonomethylether umkristallisiert.
Ausbeute: 0,6 g (46 % der Theorie) 7-(2-Acetyl-2,8-diazabicyclo[4.3.0]non-8-yl)-1-cyclopropyl-6,8-difluor-1,4-dihydro-4-oxo-3-chinolincarbonsäure, Schmelzpunkt: 261-263°C (unter Zersetzung)

Beispiel 35

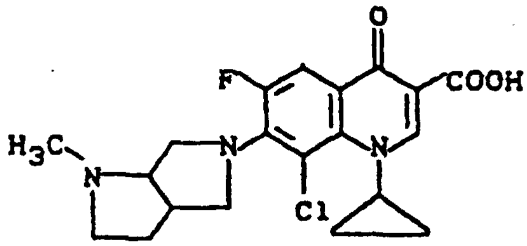

A. Analog Beispiel 13 erhält man mit 8-Chlor-1-cyclopropyl-6,7-difluor-1,4-dihydro-4-oxo-3-chinolincarbonsäure und 2-Methyl-2,7-diazabicyclo[3.3.0]octan 8-Chlor-1-cyclopropyl-6-fluor-1,4-dihydro-7-(2-methyl-2,7-diazabicyclo [3.3.0]oct-7-yl)-4-oxo-3-chinolincarbonsäure, Schmelzpunkt: 222-227°C (unter Zersetzung).

B. 2,3 g (5,8 mmol) des Betains aus Stufe A werden in 15 ml 1n-Salzsäure in der Wärme gelöst, die Lösung eingedampft und der Rückstand mit Ethanol behandelt. Der Niederschlag wird abgesaugt, mit Ethanol gewaschen und getrocknet.
Ausbeute: 2,2 g (87,7 % der Theorie) 8-Chlor-1-cyclopropyl-6-fluor-1,4-dihydro-7-(2-methyl-2,7-diazabicyclo-[3.3.0]oct-7-yl)-4-oxo-3-chinolincarbonsäure-hydrochlorid, Schmelzpunkt: 303-305°C (unter Zersetzung).

Beispiel 36

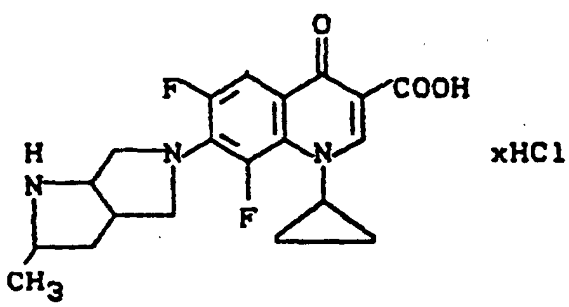

Analog Beispiel 13 wird mit 3-Methyl-2,7-diazabicyclo-[3.3.0]octan 1-Cyclopropyl-6,8-difluor-1,4-dihydro-7-(3-methyl-2,7-diazabicyclo[3.3.0]oct-7-yl)-4-oxo-3-chinolincarbonsäure erhalten und analog Beispiel 15 C. mit halbkonzentrierter Salzsäure in 1-Cyclopropyl-6,8-difluor-1,4-dihydro-7-(3-methyl-2,7-diazabicyclo[3.3.0]-oct-7-yl)-4-oxo-3-chinolincarbonsäure-Hydrochlorid, Schmelzpunkt 216-221°C (unter Zersetzung) überführt.

Beispiel 37

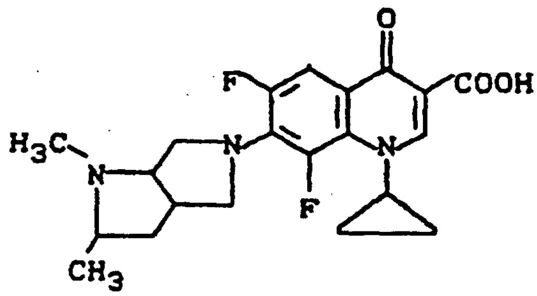

A. Eine Mischung aus 1,45 g (5 mmol) 1-Cyclopropyl-6,7,8-trifluor-1,4-dihydro-4-oxo-3-chinolincarbonsäure, 0,85 g (7,5 mmol) 1,4-Diazabicyclo[2.2.2]octan und 0,77 g (5,5 mmol) 2,3-Dimethyl-2,7-diazabicyclo[3.3.0]-octan in 15 ml Acetonitril und 7,5 ml Dimethylformamid wird 1 Stunde unter Rückfluß erhitzt. Nach dem Abkühlen wird der Niederschlag abgesaugt, mit Wasser gewaschen und aus Glykolmonomethylether umkristallisiert.
Ausbeute: 1 g (47 % der Theorie) 1-Cyclopropyl-7-(2,3-dimethyl-2,7-diazabicyclo[2.2.2]oct-7-yl)-6,8-difluor-1,4-dihydro-4-oxo-3-chinolincarbonsäure, Schmelzpunkt: 208-209°C (unter Zersetzung).

B. 0,7 g (1,7 mmol) des Betains aus Stufe A werden in 6 ml halbkonzentrierter Salzsäure heiß gelost, die Lösung filtriert und im Vakuum weitgehend konzentriert. Man versetzt mit etwa 15 ml Ethanol, kühlt im Eisbad, saugt das Salz ab, wäscht mit Ethanol und trocknet bei 100° C/1 mbar.
Ausbeute: 0,64 g (84 % der Theorie) 1-Cyclopropyl-7-(2,3-dimethyl-2,7-diazabicyclo[2.2.2]oct-7-yl)-6,8-difluor-1,4-dihydro-4-oxo-3-chinolincarbonsäure-Hydrochlorid, Schmelzpunkt: 233-236°C (unter Zersetzung).

Beispiel 38

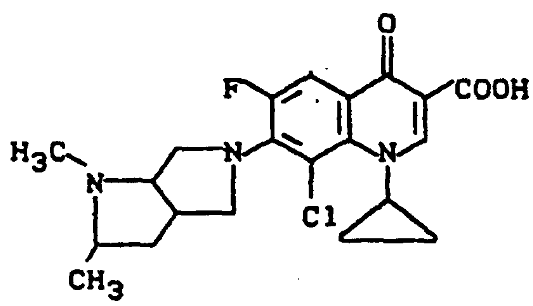

xHCl

Analog Beispiel 37 A. und B. erhält man mit 8-Chlor-1-cyclopropyl-6,7-difluor-1,4-dihydro-4-oxo-3-chinolincarbonsäure 8-Chlor-1-cyclopropyl-7-(2,3-dimethyl-2,7-diazabicyclo[2.2.2]oct-7-yl)-6-fluor-1,4-dihydro-4-oxo-3-chinolincarbonsäure-Hydrochlorid, Schmelzpunkt: 240-241°C (unter Zersetzung).

Beispiel 39

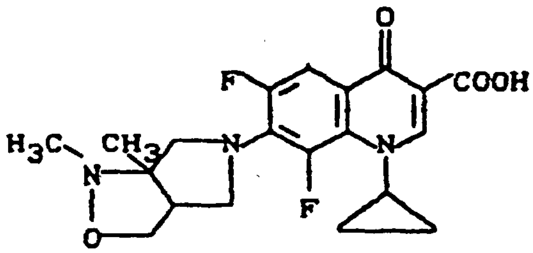

Analog Beispiel 19 wird mit 1,2-Dimethyl-3-oxa-2,7-diazabicyclo[3.3.0]octan zu 1-Cyclopropyl-6,8-difluor-1,4-dihydro-7-(1,2-dimethyl-3-oxa-2,7-diazabicyclo[3.3.0]-oct-7-yl)-4-oxo-3-chinolincarbonsäure vom Schmelzpunkt 269-271°C (unter Zersetzung) umgesetzt.

Beisipiel 40

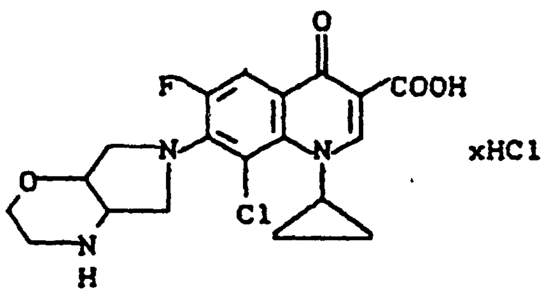

2,6 g (8,7 mmol) 8-Chlor-1-cyclopropyl-6,7-difluor-1,4-dihydro-4-oxo-3-chinolincarbonsäure werden in einer Mischung aus 25 ml Acetonitril und 12,5 ml Dimethylformamid mit 1,45 g (13 mmol) 1,4-Diazabicyclo[2.2.2]octan und 1,23 g (9,6 mmol) 2-Oxa-5,8-diazabicyclo[4.3.0]nonan versetzt und 1 Stunde unter Rückfluβ erhitzt. Die Mischung wird eingeengt, der Rückstand mit Wasser verrührt und der ungelöste Niederschlag abgesaugt und mit Wasser gewaschen. Diese rohe 1-Cyclopropyl-8-chlor-6-fluor-1,4-dihydro-7-(2-oxa-5,8-diazabicyclo[4.3.0]non-8-yl)-4-oxo-3-chinolincarbonsäure wird in 85 ml 1n-Salzsäure eingetragen und mit 6 ml konzentrierter Salzsäure versetzt. Das ausgefallene Hydrochlorid wird abgesaugt, mit Ethanol gewaschen und getrocknet.
Ausbeute: 3,0 g (77,7 % der Theorie) 8-Chlor-1-cyclopropyl-6-fluor-1,4-dihydro-7-(2-oxa-5,8-diazabicyclo-[4.3.0]non-8-yl)-4-oxo-3-chinolincarbonsäure-Hydrochlorid, Schmelzpunkt: ab 290°C Zersetzung.

Beispiel 41

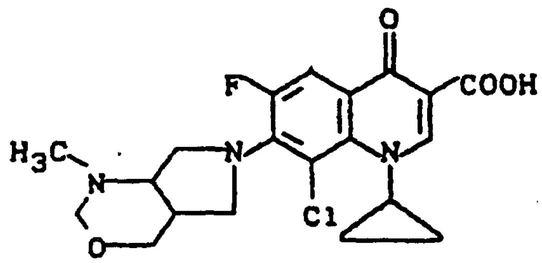

Analog Beispiel 13 erhält man mit 8-Chlor-1-cyclopropyl-6,7-difluor-1,4-dihydro-4-oxo-3-chinolincarbonsäure und 2-Methyl-4-oxa-2,8-diazabicyclo[4.3.0]nonan 8-Chlor-1-cyclopropyl-6-fluor-7-(2-methyl-4-oxa-2,8-diazabicyclo-[4.3.0]non-8-yl)-4-oxo-3-chinolincarbonsäure, Schmelzpunkt: 202-203°C (unter Zersetzung)
FAB-Massenspektrum : $^{m}/e$ 422 ($[M+H]^+$), 404 (422-$H_2O$).

Beispiel 42

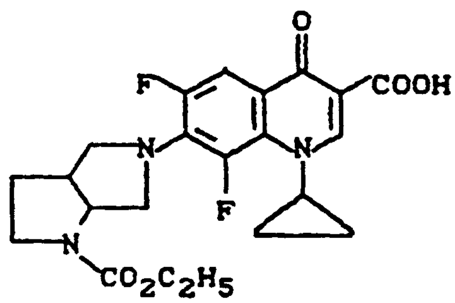

A. Analog Beispiel 13 wird mit 2,7-Diazabicyclo-[3.3.0]octan-2-carbonsäureethylester zu 1-Cyclopropyl-7-(2-ethoxycarbonyl-2,7-diazabicyclo[3.3.0]oct-7-yl)-6,8-difluor-1,4-dihydro-4-oxo-3-chinolincarbonsäure vom Schmelzpunkt 191-192°C umgesetzt.

B. 1,8 g (4 mmol) des Produktes aus Beispiel 42A werden in 30 ml konzentrierter Salzsäure unter leichtem Rückfluß während 15 Stunden erhitzt. Die Lösung wird eingeengt, der Rückstand mit Ethanol verrührt, der Niederschlag abgesaugt, mit Ethanol gewaschen und bei 120° C/12 mbar getrocknet.
Ausbeute: 1,1 g (67 % der Theorie) 1-Cyclopropyl-7-(2,7-diazabicyclo[3.3.0]oct-7-yl)-6,8-difluor-1,4-dihydro-4-oxo-3-chinolincarbonsäure-hydrochlorid, Schmelzpunkt: 273-275°C (unter Zersetzung). Das Produkt ist identisch mit der nach Beispiel 26B erhaltenen Verbindung.

Beispiel 43

A. 7,8 g (20 mmol) 1-Cyclopropyl-7-(2,8-diazabicyclo[4.3.0]non-8-yl)-6,8-difluor-1,4-dihydro-4-oxo-3-chinolincarbonsäure werden in 175 ml Ethanol eingetragen und bei etwa 70° C mit 2,4 g (25 mmol) Methansulfonsäure versetzt. Das Betain löst sich auf und beim Abkühlen fällt das Salz aus, das abgesaugt, mit Ethanol gewaschen und bei 120° C/12 mbar getrocknet wird. Es ist in Wasser leicht löslich.
Ausbeute: 8,6 g (88,6 % der Theorie) 1-Cyclopropyl-7-(2,8-diazabicyclo[4.3.0]non-8-yl)-6,8-difluor-1,4-dihy-dro-4-oxo-3-chinolincarbonsäure-Mesylat, Schmelzpunkt: 262-265°C (unter Zersetzung).
Analog erhält man:

B. 1-Cyclopropyl-7-(2,8-diazabicyclo[4.3.0]non-8-yl)-6,8-difluor-1,4-dihydro-4-oxo-3-chinolincarbonsäure-Tosylat, Schmelzpunkt: 248-250°C (unter Zersetzung).

C. 1-Cyclopropyl-7-(2,8-diazabicyclo[4.3.0]non-8-yl)-6,8-difluor-1,4-dihydro-4-oxo-3-chinolincarbonsäure-Lactat, Schmelzpunkt: 205°C-215°C nach vorhergehendem Sintern.

Beispiel 44

3,9 g (10 mmol) 1-Cyclopropyl-7-(2,8-diazabicyclo-[4.3.0]non-8-yl)-6,8-difluor-1,4-dihydro-4-oxo-3-chinolincarbonsäure werden in 50 ml Wasser suspendiert und bei Raumtemperatur mit 10 ml 1 n-Natronlauge versetzt, wobei sich das Produkt weitgehend auflöst. Von einer schwachen Trübung wird durch Filtration über ein Membranfilter abgetrennt, das Filtrat im Hochvakuum eingeengt und der Rückstand mit Ethanol verrührt, abgesaugt und getrocknet.
Ausbeute: 3,4 g (82,7 % der Theorie) 1-Cyclopropyl-7-(2,8-diazabicyclo[4.3.0]non-8-yl)-6,8-difluor-1,4-dihydro-4-oxo-3-chinolincarbonsäure-Natriumsalz; das Salz zersetzt sich langsam oberhalb 210°C ohne zu schmelzen.

Beispiel 45

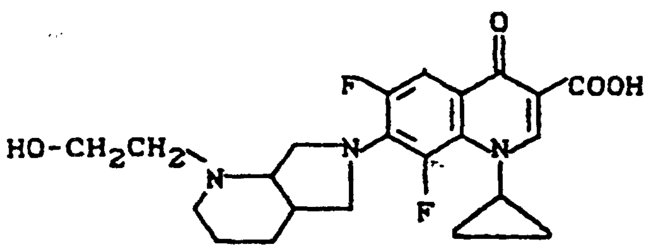

Eine Mischung von 3,9 g (10 mmol) 1-Cyclopropyl-7-(2,8-diazabicyclo[4.3.0]non-8-yl)-6,8-difluor-1,4-dihydro-4-oxo-3-chinolincarbonsäure in 100 ml Dimethylformamid mit 4,2 g Triethylamin und 2,8 g 2-Bromethanol wird 20 Stunden auf 80-100°C erhitzt. Danach wird die Lösung im Vakuum eingeengt und der erhaltene Rückstand an 200 g Kieselgel chromatographisch gereinigt (Laufmittel: $CH_2Cl_2/CH_3OH/17$ % $-NH_3$ = 30:8:1). Das Eluat wird eingeengt, mit Ethanol verrührt, abgesaugt und getrocknet.
Ausbeute: 1,8 g (41,6 % der Theorie) 1-Cyclopropyl-6,8-difluor-1,4-dihydro-7-[2-(2-hydroxyethyl)-2,8-diazabicyclo[4.3.0]non-8-yl]-4-oxo-3-chinolincarbonsäure, Schmelzpunkt: 200-206°C (unter Zersetzung).
Massenspektrum: $^{m}/e$ 433 ($M^+$), 402 ($M^+$ $-CH_2OH$), 140, 110 (100 %), 96

Beispiel 46

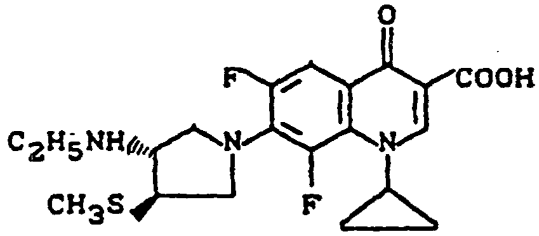

Analog Beispiel 13 wird mit trans-3-Ethylamino-4-methylthio-pyrrolidin zu 1-Cyclopropyl-7-(trans-3-ethylamino-4-methylthio)-6,8-difluor-1,4-dihydro-4-oxo-3-chinolincarbonsäure umgesetzt, Schmelzpunkt: 215-216°C (unter

Zersetzung).

Beispiel 47

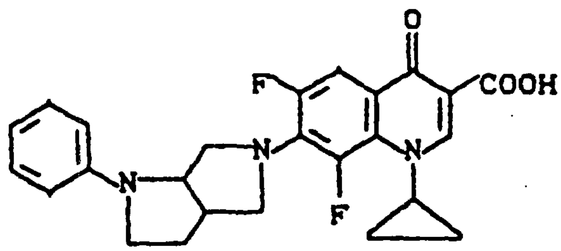

Analog Beispiel 13 wird mit 2-Phenyl-2,7-diazabicyclo-[3.3.0]octan zu 1-Cyclopropyl-6,8-difluor-1,4-dihydro-4-oxo-7-(2-phenyl-2,7-diazabicyclo[3.3.0]oct-7-yl)-3-chinolincarbonsäure umgesetzt, Schmelzpunkt: 259-260°C (unter Zersetzung).

Beispiel 48

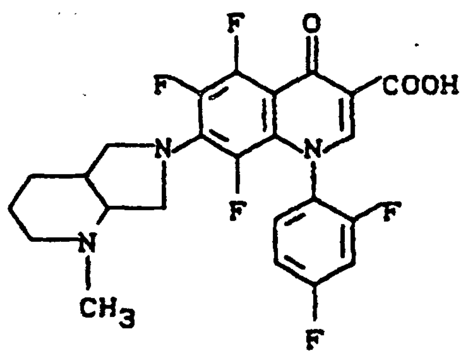

Analog Beispiel 13 erhält man mit 5,6,7,8-Tetrafluor-1-(2,4-difluorphenyl)-1,4-dihydro-4-oxo-3-chinolincarbonsäure 5,6,8-Trifluor-1-(2,4-difluorphenyl)-1,4-dihydro-7-(2-methyl-2,8-diazabicyclo[4.3.0]non-8-yl)-4-oxo-3-chinolincarbonsäure.

Beispiel 49

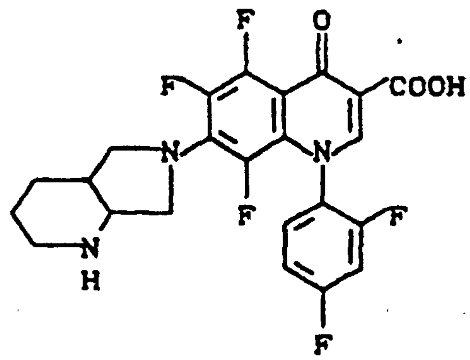

Analog Beispiel 24 erhält man mit 5,6,7,8-Tetrafluor-1-(2,4-difluorphenyl)-1,4-dihydro-4-oxo-3-chinolincarbonsäure 7-(2,8-Diazabicyclo[4.3.0]non-8-yl)-5,6,8-trifluor-1-(2,4-difluorphenyl)-1,4-dihydro-4-oxo-3-chinolincarbonsäure.

Beispiel 50

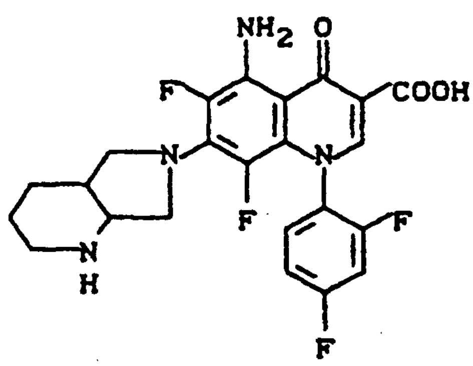

Analog Beispiel 25 erhält man mit 7-(2,8-Diazabicyclo-[4.3.0]non-8-yl)-5,6,8-trifluor-1-(2,4-difluorphenyl)-1,4-dihydro-4-oxo-3-chinolincarbonsäure 5-Amino-7-(2,8-diazabicyclo[4.3.0]non-8-yl)-6,8-difluor-1-(2,4-difluorphenyl)-1,4-dihydro-4-oxo-3-chinolincarbonsäure.

Beispiel 51

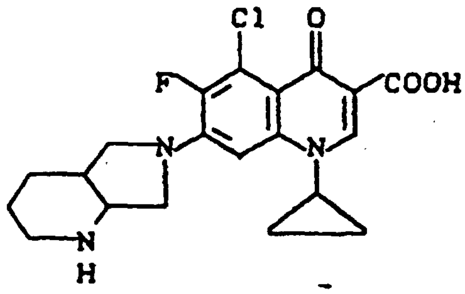

Analog Beispiel 15 A erhält man mit 5,7-Dichlor-1-cyclopropyl-6-fluor-1,4-dihydro-4-oxo-3-chinolincarbonsäure (5 Stunden Rückfluß) 5-Chlor-1-cyclopropyl-7-(2,8-diazabicyclo[4.3.0]non-8-yl)-6-fluor-1,4-dihydro-4-oxo-3-chinolincarbonsäure, Schmelzpunkt: 270°C (Zersetzung).

Beispiel 52

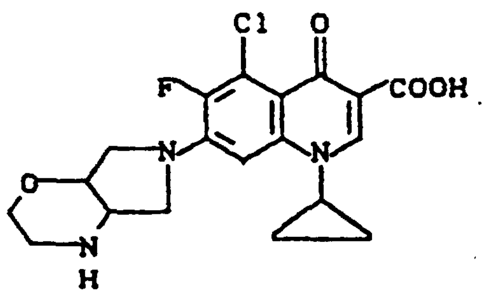

Analog Beispiel 8 erhält man mit 5,7-Dichlor-1-cyclopropyl-6-fluor-1,4-dihydro-4-oxo-3-chinolincarbonsäure (5 Stunden Rückfluß) 5-Chlor-1-cyclopropyl-6-fluor-1,4-dihydro-7-(2-oxa-5,8-diazabicyclo[4.3.0]non-8-yl)-4-oxo-3-chinolincarbonsäure.

Beispiel 53

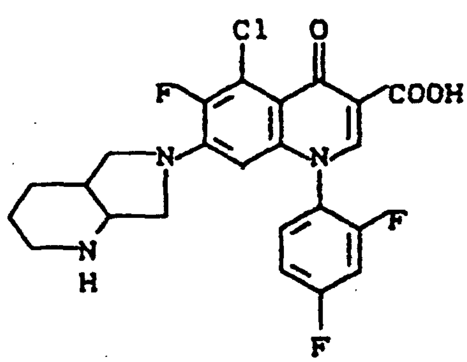

Analog Beispiel 15 A erhält man mit 5,7-Dichlor-6-fluor-1-(2,4-difluorphenyl)-1,4-dihydro-4-oxo-3-chinolincarbonsäure (5 Stunden Rückfluß) 5-Chlor-7-(2,8-diazabicyclo[4.3.0]non-8-yl)-6-fluor-1-(2,4-difluorphenyl)-1,4-dihydro-4-oxo-3-chinolincarbonsäure.

Beispiel 54

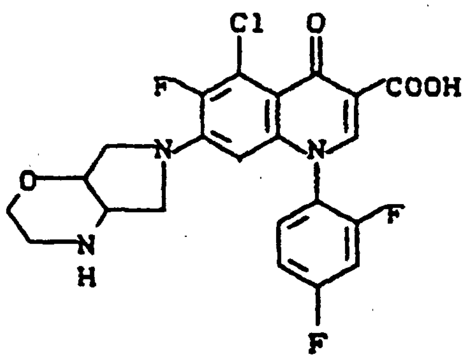

Analog Beispiel 8 erhält man mit-5,7-Dichlor-6-fluor-1-(2,4-difluorphenyl)-1,4-dihydro-4-oxo-3-chinolincarbonsäure (5 Stunden Rückfluß) 5-Chlor-6-fluor-1-(2,4-difluorphenyl)-1,4-dihydro-7-(2-oxa-5,8-diazabicyclo [4.3.0]non-8-yl)-4-oxo-3-chinolincarbonsäure.

Beispiel 55

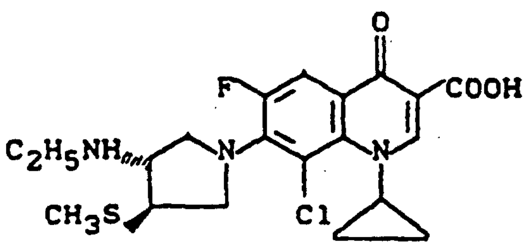

Analog Beispiel 13 wird mit trans-3-Ethylamino-4-methylthio-pyrrolidin und 8-Chlor-1-cyclopropyl-6,7-difluor-1,4-dihydro-4-oxo-3-chinolincarbonsäure zu 8-chlor-1-cyclopropyl-7-(trans-3-ethylamino-4-methylthio-1-pyrrolidinyl)-6-fluor-1,4-dihydro-4-oxo-3-chinolincarbonsäure umgesetzt, Schmelzpunkt: 217-218°C (unter Zersetzung).

Beispiel 56

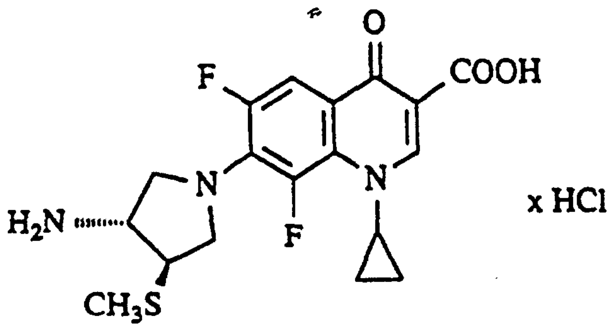

Analog Beispiel 13 und 15 erhält man mit trans-3-Amino-4-methylthiopyrrolidin 7-(trans-3-Amino-4-methylthio-1-pyrrolidinyl)-1-cyclopropyl-6,8-difluor-1,4-dihydro-4-oxo-3-chinolincarbonsäure, Schmelzpunkt: 208-211°C (unter Zersetzung) und 7-(trans-3-Amino-4-methylthio-1-pyrrolidinyl)-1-cyclopropyl-6,8-difluor-1,4-dihydro-4-oxo-3-chinolincarbonsäure-Hydrochlorid, Schmelzpunkt: 255-257°C (unter Zersetzung).

Beispiel 57

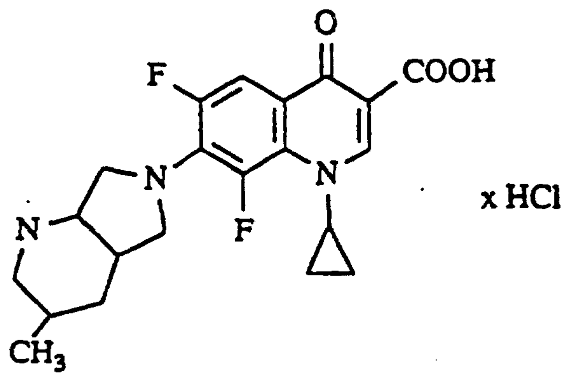

Analog Beispiel 13 und 15 erhält man mit 4-Methyl-2,8-diazabicyclo[4.3.0]-nonan 1-Cyclopropyl-6,8-difluor-1,4-dihydro-7-(4-methyl-2,8-diazabicyclo[4.3.0]non-8-yl)-4-oxo-3-chinolincarbonsäure, Schmelzpunkt: 213-215°C (unter Zersetzung) (umkristallisiert aus Glykolmonomethylether) und 1-Cyclopropyl-6,8-difluor-1,4-dihydro-7-(4-methyl-2,8-diazabicyclo[4.3.0]non-8-yl)-4-oxo-3-chinolincarbonsäure-Hydrochlorid, Schmelzpunkt: 204-212°C (unter Zersetzung).

Das Produkt besteht aus einem Gemisch von 2 Stereoisomeren.

## Patentansprüche

### Patentansprüche für folgende Vertragsstaaten : AT, BE, CH, DE, DK, FR, GB, IT, LI, LU, NL, SE

1. 7-(1-Pyrrolidinyl)-3-chinolon- und -naphthyridoncarbonsäure-Derivate der Formel (I)

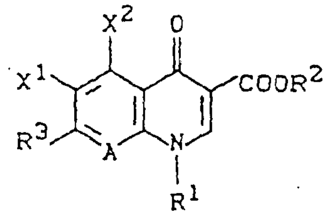

(I),

in welcher

$X^1$ für Halogen,

$X^2$ für Wasserstoff Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 4 Kohlenstoffatomen oder (5-Methyl-2-oxo-1,3-dioxol-4-yl)-methyl und

$R^3$ für einen Rest der Struktur

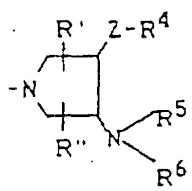

steht, worin

$R^4$ für $C_1$-$C_4$-Alkyl, Aryl, $C_1$-$C_4$-Acyl

$R^5$ für H, $C_1$-$C_4$-Alkyl, OH, $OCH_3$,

$R^6$ für H, gegebenenfalls durch Hydroxy substituiertes $C_1$-$C_4$-Alkyl, sowie Aryl, Heteroaryl, Benzyl, $C_1$-$C_4$-Alkoxycarbonyl, $C_1$-$C_4$-Acyl, (5-Methyl-2-oxo-1,3-dioxol-4-yl)-methyl oder $C_3$-$C_6$-Cycloalkyl,

R' für H, $CH_3$ oder Phenyl,

R" für H, $CH_3$ oder Phenyl, und

Z für O oder S stehen kann,

A für N oder C-$R^8$ steht, worin

$R^8$ für H, Halogen, Methyl, Cyano, Nitro oder Hydroxy steht oder auch gemeinsam mit $R^1$ eine Brücke der Struktur

$-O-CH_2-CH-CH_3$, $-S-CH_2-CH-CH_3$

oder

$-CH_2-CH_2-CH-CH_3$

bilden kann,
und deren pharmazeutisch verwendbare Hydrate und Säureadditionssalze sowie die Alkali-, Erdalkali-, Silber- und Guanidiniumsalze der zugrundeliegenden Carbonsäuren,

ausgenommen Verbindungen mit der Formel

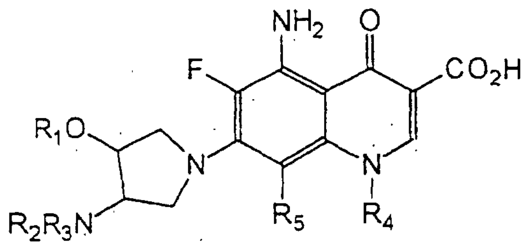

worin

$R_1$ für C1-C4 Alkyl,

$R_2/R_3$ für Wasserstoff oder C1-C4 Alkyl,

$R_4$ für Cyclopropyl, Phenyl, Halophenyl, Thienyl, wahlweise mit C1-C4 Alkyl oder Halogen substituiert, und

$R_5$ für Halogen stehen.

**2.** Verbindungen der Formel (I) gemäß Anspruch 1, in welcher

$X^1$ für Fluor oder Chlor,

$X^2$ für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,

$R^1$ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 3 Kohlenstoffatomen oder (5-Methyl-2-oxo-1,3-dioxol-4-yl)-methyl,

$R^3$ für einen Rest der Struktur

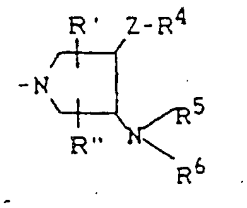

steht, worin

$R^4$ für $C_1$-$C_3$-Alkyl, $C_1$-$C_2$-Acyl

$R^5$ für H, $C_1$-$C_3$-Alkyl, OH, $OCH_3$, wobei $R^4$ und $R^5$ gemeinsam auch eine gegebenenfalls durch Methyl ein- oder zweifach substituierte $C_1$-$C_2$-Alkylenbrücke bedeuten können,

$R^6$ für H, gegebenenfalls durch Hydroxy substituiertes $C_1$-$C_3$-Alkyl, sowie Phenyl, Benzyl, $C_1$-$C_4$-Alkoxycarbonyl, $C_1$-$C_2$-Acyl, (5-Methyl-2-oxo-1,3-dioxol-4-yl)-methyl oder $C_3$-$C_5$-Alkyl,

R' für H oder $CH_3$,

R" für H oder $CH_3$, und

Z für O oder S stehen kann,

A für N oder C-$R^8$ steht, worin

$R^8$ für H, Fluor, Chlor, Brom oder Hydroxy steht oder auch gemeinsam mit $R^1$ eine Brücke der Struktur

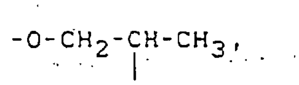

bilden kann.

**3.** Verbindungen der Formel (I) gemäß Anspruch 1, in welcher

$X^1$ für Fluor

$X^2$ für Wasserstoff, Amino, Methylamino, Fluor

$R^1$ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,

$R^2$ für Wasserstoff, Alkyl mit 1 bis 2 Kohlenstoffatomen,

$R^3$ für einen Rest der Struktur

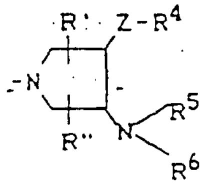

steht, worin

R4 für $C_1$-$C_2$-Alkyl,

R5 für H, $C_1$-$C_2$-Alkyl, wobei $R^4$ und $R^5$ gemeinsam auch eine gegebenenfalls durch Methyl substituierte $C_1$-$C_2$-Alkylenbrücke bedeuten können,

R6 für H, $CH_3$, $C_2H_5$, $HOCH_2CH_2$, Benzyl, $C_1$-$C_4$-Alkoxycarbonyl, $C_1$-$C_2$-Acyl,

R' für H oder $CH_3$,

R" für H oder $CH_3$, und

Z für O oder S stehen kann,

A für N oder C-$R^8$ steht, worin

$R^8$ für H, Fluor oder Chlor steht, oder auch gemeinsam mit $R^1$ eine Brücke der Struktur

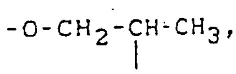

bilden kann.

**4.** Verfahren zur Herstellung von Verbindungen gemäß Anspruch 1 der Formel (I), **dadurch gekennzeichnet, daß** man Verbindungen der Formel (II)

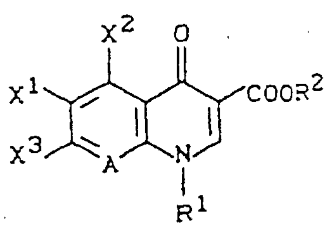 (II),

in welcher

A, $R^1$, $R^2$, $X^1$ und $X^2$ die oben angegebene Bedeutung haben und

$X^3$ für Halogen, insbesondere Fluor oder Chlor steht, mit Verbindungen der Formel (III)

$$R^3\text{-}H \qquad (III),$$

in welcher

$R^3$ die in Anspruch 1 angegebene Bedeutung hat,

gegebenenfalls in Gegenwart von Säurefängern umsetzt, und gegebenenfalls in $R^3$ enthaltene Schutzgruppen abspaltet.

**5.** Verfahren zur Herstellung von Verbindungen gemäß Anspruch 1 der Formel (I)

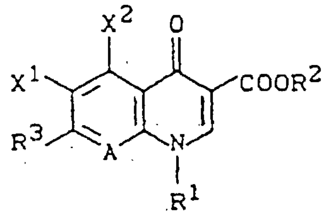

(I),

in welcher

$X^1$, $R^1$, $R^2$, $R^3$ und A die oben angegebene Bedeutung haben und

$X^2$ für Amino, Alkylamino mit 1 bis 4 Kohlenstoffastomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,

**dadurch gekennzeichnet, daß** man eine Verbindung der Formel (IV)

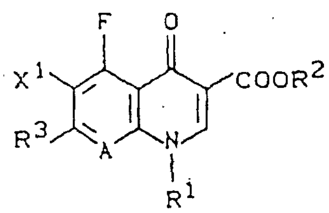

(IV),

in welcher

$X^1$, $R^1$, $R^2$, $R^3$ und A die oben angegebene Bedeutung haben,

mit Verbindungen der Formel (V)

$$X^2\text{-}H \qquad (V),$$

in welcher

$X^2$ die oben angegebene Bedeutung hat, gegebenenfalls in Gegenwart von Säurefängern umsetzt.

**6.** Verfahren zur Herstellung von Verbindungen gemäß Anspruch 1 der Formel (Ia)

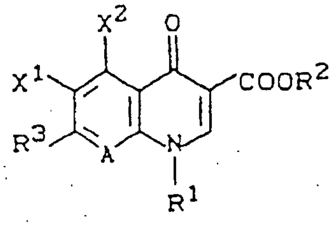

(Ia),

in welcher
$X^1$, $X^2$, $R^1$, $R^2$ und A die oben angegebene Bedeutung haben und $R^3$ für einen Rest der Struktur

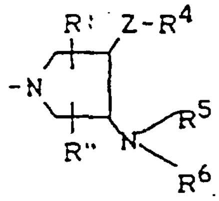

steht, worin
$R^4$, $R^5$, $R^6$, R', R" und Z die oben angegebene Bedeutung haben,
**dadurch gekennzeichnet, daß** man eine Verbindung der Formel (VI)

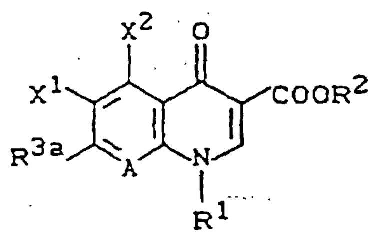

(VI),

in welcher
$X^1$, $X^2$, $R^1$, $R^2$ und A die oben angegebene Bedeutung haben und

$R^{3a}$ für einen Rest der Struktur

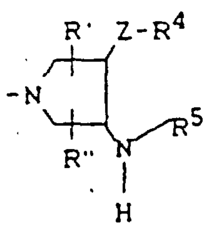

steht,
worin $R^4$, $R^5$, R', R" und Z die oben angegebene Bedeutung haben,
mit Verbindungen der Formel (VII)

$$R^6\text{-}X^a \qquad (VII),$$

in welcher

$R^6$ die oben angegebene Bedeutung hat und

$X^a$ für Chlor, Brom, Iod, Hydroxy oder Acyloxy steht,

gegebenenfalls in Gegenwart von Säurefängern umsetzt.

**7.** 7-(1-Pyrrolidinyl)-3-chinolon- und -naphthyridoncarbonsäure-Derivate der Formel (I) gemäß Anspruch 1 zur Anwendung in einem Verfahren zur Bekämpfung von Krankheiten.

**8.** Arzneimittel enthaltend Verbindungen der Formel (I) gemäß Anspruch 1.

**9.** Verwendung von Verbindungen der Formel (I) gemäß Anspruch 1 zur Herstellung von Arzneimitteln.

**10.** Verwendung von Verbindungen der Formel (I) gemäß Anspruch 1 als Tierfutterzusatzmittel.

**11.** Tierfutter bzw. Tierfutterzusatzmittel und Prämixe enthaltend Verbindungen der Formel (I) nach Anspruch 1.

**12.** Carbonsäurederivate in S,S-Konfiguration mit der Formel

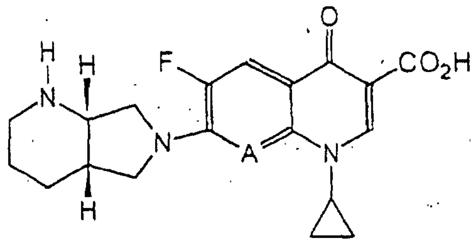

worin A für C-Cl, C-F, oder C-$OCH_3$ steht und deren pharmazeutisch verträgliche Hydrate und Salze.

**13.** Arzneimittel enthaltend eine Verbindung gemäß Anspruch 12.

**14.** Verwendung von Verbindungen gemäß Anspruch 12 zur Herstellung von Tierfutter, Tierfutterzusatzmitteln und Prämixen.

**15.** Verbindungen gemäß Anspruch 12 als antibakterielle Mittel.

**16.** Verwendung von Verbindungen gemäß Anspruch 12 zur Herstellung eines Medikamentes zur Behandlung von bakteriellen Erkrankungen.

**Patentansprüche für folgenden Vertragsstaat : GR**

**1.** 7-(1-Pyrrolidinyl)-3-chinolon- und -naphthyridoncarbonsäure-Derivate der Formel (I)

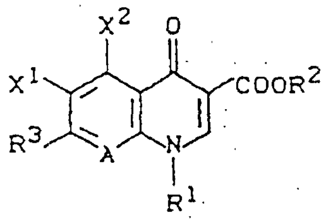

(I),

in welcher

$X^1$ für Halogen,

$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 4 Kohlenstoffatomen oder (5-Methyl-2-oxo-1,3-dioxol-4-yl)-methyl und

$R^3$ für einen Rest der Struktur

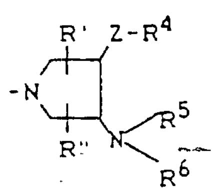

steht, worin

$R^4$ für $C_1$-$C_4$-Alkyl, Aryl, $C_1$-$C_4$-Acyl

$R^5$ für H, $C_1$-$C_4$-Alkyl, OH, $OCH_3$,

$R^6$ für H, gegebenenfalls durch Hydroxy substituiertes $C_1$-$C_4$-Alkyl, sowie Aryl , Heteroaryl, Benzyl, $C_1$-$C_4$-Alkoxycarbonyl, $C_1$-$C_4$-Acyl, (5-Methyl-2-oxo-1,3-dioxol-4-yl)-methyl oder $C_3$-$C_6$-Cycloalkyl,

R' für H, $CH_3$ oder Phenyl,

R" für H, $CH_3$ oder Phenyl, und

Z für O oder S stehen kann,

A für N oder C-$R^8$ steht, worin

$R^8$ für H, Halogen, Methyl, Cyano, Nitro oder Hydroxy steht oder auch gemeinsam mit $R^1$ eine Brücke der Struktur

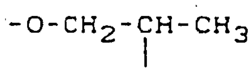, 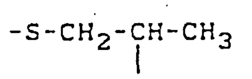

oder

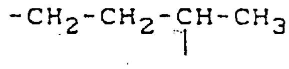

bilden kann,
und deren pharmazeutisch verwendbare Hydrate und Säureadditionssalze sowie die Alkali-, Erdalkali-, Silber- und Guanidiniumsalze der zugrundeliegenden Carbonsäuren,

ausgenommen Verbindungen mit der Formel

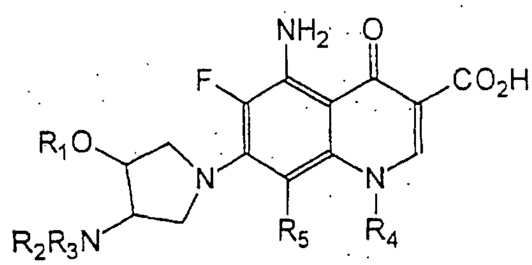

worin

$R_1$ für C1-C4 Alkyl,

$R_2/R_3$ für Wasserstoff oder C1-C4 Alkyl,

$R_4$ für Cyclopropyl, Phenyl, Halophenyl, Thienyl, wahlweise mit C1-C4 Alkyl oder Halogen substituiert, und

$R_5$ für Halogen stehen.

**2.** Verbindungen der Formel (I) gemäß Anspruch 1, in welcher

$X^1$ für Fluor oder Chlor,

$X^2$ für Wasserstoff, Amino , Alkylamino mit 1 bis 2 Kohlenstoffatomen, Dimethylamino, Hydroxy, Methoxy, Mercapto, Methylthio, Phenylthio, Fluor, Chlor,

$R^1$ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 3 Kohlenstoffatomen oder (5-Methyl-2-oxo-1,3-dioxol-4-yl)-methyl,

$R^3$ für einen Rest der Struktur

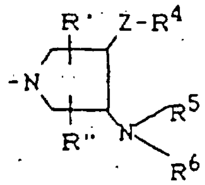

steht, worin

$R^4$ für $C_1$-$C_3$-Alkyl, $C_1$-$C_2$-Acyl

$R^5$ für H, $C_1$-$C_3$-Alkyl, OH, $OCH_3$, wobei $R^4$ und $R^5$ gemeinsam auch eine gegebenenfalls durch Methyl ein- oder zweifach substituierte $C_1$-$C_2$-Alkylenbrücke bedeuten können,

$R^6$ für H, gegebenenfalls durch Hydroxy substituiertes $C_1$-$C_3$-Alkyl, sowie Phenyl, Benzyl, $C_1$-$C_4$-Alkoxycarbonyl, $C_1$-$C_2$-Acyl, (5-Methyl-2-oxo-1,3-dioxol-4-yl)-methyl oder $C_3$-$C_5$-Alkyl,

R' für H oder $CH_3$,

R" für H oder $CH_3$, und

Z für O oder S stehen kann,

A für N oder C-$R^8$ steht, worin

$R^8$ für H, Fluor, Chlor, Brom oder Hydroxy steht oder auch gemeinsam mit $R^1$ eine Brücke der Struktur

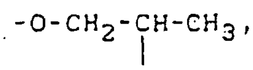

bilden kann.

**3.** Verbindungen der Formel (I) gemäß Anspruch 1, in welcher

$X^1$ für Fluor

$X^2$ für Wasserstoff, Amino, Methylamino, Fluor

$R^1$ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,

$R^2$ für Wasserstoff, Alkyl mit 1 bis 2 Kohlenstoffatomen,

$R^3$ für einen Rest der Struktur

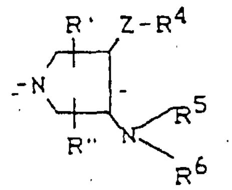

steht, worin

R4 für $C_1$-$C_2$-Alkyl,

R5 für H, $C_1$-$C_2$-Alkyl, wobei R4 und R5 gemeinsam auch eine gegebenenfalls durch Methyl substituierte $C_1$-$C_2$-Alkylenbrücke bedeuten können,

R6 für H, $CH_3$, $C_2H_5$, $HOCH_2CH_2$, Benzyl, $C_1$-$C_4$-Alkoxycarbonyl, $C_1$-$C_2$-Acyl,

R' für H oder $CH_3$,

R" für H oder $CH_3$, und

Z für O oder S stehen kann,

A für N oder C-$R^8$ steht, worin

$R^8$ für H, Fluor oder Chlor steht,

oder auch gemeinsam mit $R^1$ eine Brücke der Struktur

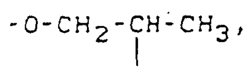

bilden kann.

**4.** Verfahren zur Herstellung von Verbindungen gemäß Anspruch 1 der Formel (I), **dadurch gekennzeichnet, daß** man Verbindungen der Formel (II)

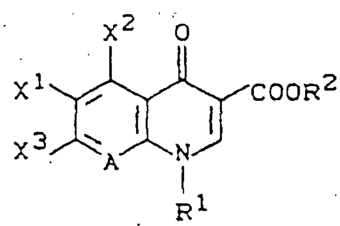 (II),

in welcher

A, $R^1$, $R^2$, $X^1$ und $X^2$ die oben angegebene Bedeutung haben und

$X^3$ für Halogen, insbesondere Fluor oder Chlor steht, mit Verbindungen der Formel (III)

$$R^3\text{-H} \qquad \text{(III)},$$

in welcher

$R^3$ die in Anspruch 1 angegebene Bedeutung hat,

gegebenenfalls in Gegenwart von Säurefängern umsetzt, und gegebenenfalls in $R^3$ enthaltene Schutzgruppen abspaltet.

**5.** Verfahren zur Herstellung von Verbindungen gemäß Anspruch 1 der Formel (I)

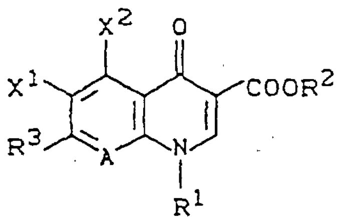

(I),

in welcher

$X^1$, $R^1$, $R^2$, $R^3$ und A die oben angegebene Bedeutung haben und

$X^2$ für Amino, Alkylamino mit 1 bis 4 Kohlenstoffastomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,

**dadurch gekennzeichnet, daß** man eine Verbindung der Formel (IV)

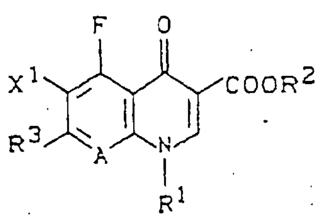

(IV),

in welcher

$X^1$, $R^1$, $R^2$, $R^3$ und A die oben angegebene Bedeutung haben,

mit Verbindungen der Formel (V)

$$X^2\text{-H} \qquad \text{(V)},$$

in welcher

$X^2$ die oben angegebene Bedeutung hat, gegebenenfalls in Gegenwart von Säurefängern umsetzt.

**6.** Verfahren zur Herstellung von Verbindungen gemäß Anspruch 1 der Formel (Ia)

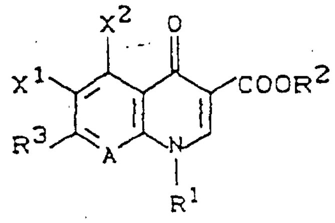

(Ia),

in welcher
$X^1$, $X^2$, $R^1$, $R^2$ und A die oben angegebene Bedeutung haben und $R^3$ für einen Rest der Struktur

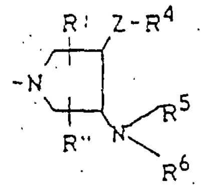

steht, worin
$R^4$, $R^5$, $R^6$, R', R" und Z die oben angegebene Bedeutung haben,
**dadurch gekennzeichnet, daß** man eine Verbindung der Formel (VI)

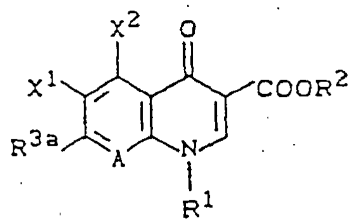

(VI),

in welcher
$X^1$, $X^2$, $R^1$, $R^2$ und A die oben angegebene Bedeutung haben und

$R^{3a}$ für einen Rest der Struktur

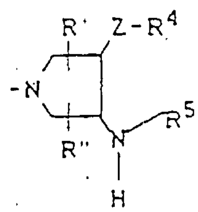

steht,
worin $R^4$, $R^5$, R', R" und Z die oben angegebene Bedeutung haben,
mit Verbindungen der Formel (VII)

$$R^6\text{-}X^a \qquad (VII),$$

in welcher

$R^6$ die oben angegebene Bedeutung hat und

$X^a$ für Chlor, Brom, Iod, Hydroxy oder Acyloxy steht,

gegebenenfalls in Gegenwart von Säurefängern umsetzt.

**7.** Verwendung von Verbindungen der Formel (I) gemäß Anspruch 1 als Tierfutterzusatzmittel.

**8.** Tierfutter bzw. Tierfutterzusatzmittel und Prämixe enthaltend Verbindungen der Formel (I) nach Anspruch 1.

**9.** Carbonsäurederivate in S,S-Konfiguration mit der Formel

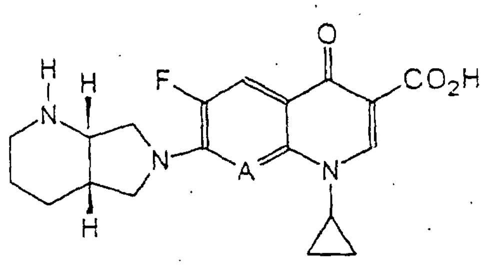

worin A für C-Cl, C-F, oder C-$OCH_3$ steht und deren pharmazeutisch verträgliche Hydrate und Salze.

**10.** Verwendung von Verbindungen gemäß Anspruch 9 zur Herstellung von Tierfutter, Tierfutterzusatzmitteln und Prämixen.

**Patentansprüche für folgenden Vertragsstaat : ES**

**1.** Verfahren zu Herstellung von 7-(1-Pyrrolidinyl)-3-chinolon- und -naphthyridoncarbonsäure-Derivaten der Formel (I)

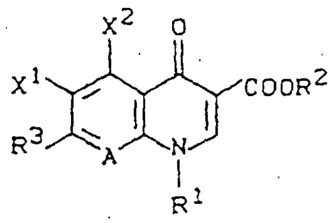

(I),

in welcher

$X^1$ für Halogen,

$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 4 Kohlenstoffatomen oder (5-Methyl-2-oxo-1,3-dioxol-4-yl)-methyl und

$R^3$ für einen Rest der Struktur

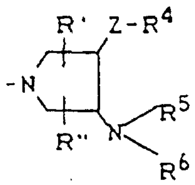

steht, worin

$R^4$ für $C_1$-$C_4$-Alkyl, Aryl, $C_1$-$C_4$-Acyl

$R^5$ für H, $C_1$-$C_4$-Alkyl, OH, $OCH_3$,

$R^6$ für H, gegebenenfalls durch Hydroxy substituiertes $C_1$-$C_4$-Alkyl, sowie Aryl, Heteroaryl, Benzyl, $C_1$-$C_4$-Alkoxycarbonyl, $C_1$-$C_4$-Acyl, (5-Methyl-2-oxo-1,3-dioxol-4-yl)-methyl oder $C_3$-$C_6$-Cycloalkyl,

R' für H, $CH_3$ oder Phenyl,

R" für H, $CH_3$ oder Phenyl, und

Z für O oder S stehen kann,

A für N oder C-$R^8$ steht, worin

$R^8$ für H, Halogen, Methyl, Cyano, Nitro oder Hydroxy steht oder auch gemeinsam mit $R^1$ eine Brücke der Struktur

$-O-CH_2-CH-CH_3$, $-S-CH_2-CH-CH_3$

oder

$-CH_2-CH_2-CH-CH_3$

bilden kann,
und deren pharmazeutisch verwendbare Hydrate und Säureadditionssalze sowie die Alkali-, Erdalkali-, Silber- und Guanidiniumsalze der zugrundeliegenden Carbonsäuren, ausgenommen Verbindungen mit der Formel

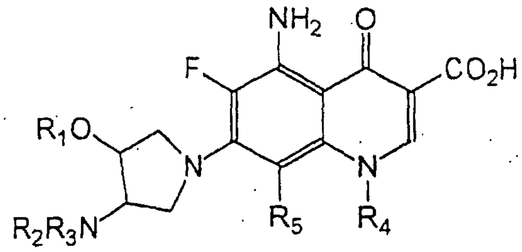

worin

$R_1$ für C1-C4 Alkyl,

$R_2/R_3$ für Wasserstoff oder C1-C4 Alkyl,

$R_4$ für Cyclopropyl, Phenyl, Halophenyl, Thienyl, wahlweise mit C1-C4 Alkyl oder Halogen substituiert, und

$R_5$ für Halogen stehen.

**dadurch gekennzeichnet, dass** man Verbindungen der Formel (II)

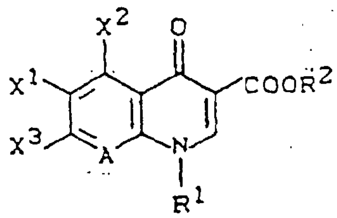

(II),

in welcher

A, $R^1$, $R^2$, $X^1$ und $X^2$ die oben angegebene Bedeutung haben und

$X^3$ für Halogen, insbesondere Fluor oder Chlor steht, mit Verbindungen der Formel (III)

$$R^3\text{-H} \quad \text{(III)},$$

in welcher
$R^3$ die oben angegebene Bedeutung hat, gegebenenfalls in Gegenwart von Säurefängern umsetzt und gegebenenfalls in $R^3$ enthaltene Schutzgruppen abspaltet und die so erhaltenem Verbindungen der Formel (I) gegebenenfalls auf an sich bekannte Weise in ihre pharmazeutisch verwendbaren Hydrate oder Säureadditionssalze oder in die Alkali-, Erdalkali-, Silber - und Guanidiniumsalze der zugrunde liegenden Carbonsäure überführt.

**2.** Verfahren gemäß Anspruch 1, in dem

$X^1$ für Fluor oder Chlor,

$X^2$ für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,

$R^1$ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, Alkyl mit 1 bis 3 Kohlenstoffatomen oder (5-Methyl-2-oxo-1,3-dioxol-4-yl)-methyl,

$R^3$ für einen Rest der Struktur

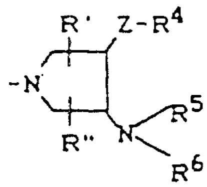

steht, worin

$R^4$ für $C_1$-$C_3$-Alkyl, $C_1$-$C_2$-Acyl

$R^5$ für H, $C_1$-$C_3$-Alkyl, OH, $OCH_3$, wobei $R^4$ und $R^5$ gemeinsam auch eine gegebenenfalls durch Methyl ein- oder zweifach substituierte $C_1$-$C_2$-Alkylenbrücke bedeuten können,

$R^6$ für H, gegebenenfalls durch Hydroxy substituiertes $C_1$-$C_3$-Alkyl, sowie Phenyl, Benzyl, $C_1$-$C_4$-Alkoxycarbonyl, $C_1$-$C_2$-Acyl, (5-Methyl-2-oxo-1,3-dioxol-4-yl)-methyl oder $C_3$-$C_5$-Alkyl,

R' für H oder $CH_3$,

R'' für H oder $CH_3$, und

Z für O oder S stehen kann,

A für N oder C-$R^8$ steht, worin

$R^8$ für H, Fluor, Chlor, Brom oder Hydroxy steht oder auch gemeinsam mit $R^1$ eine Brücke der Struktur

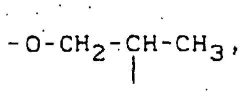

bilden kann.

**3.** Verfahren gemäß Anspruch 1, in dem

$X^1$ für Fluor

$X^2$ für Wasserstoff, Amino, Methylamino, Fluor

$R^1$ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,

$R^2$ für Wasserstoff, Alkyl mit 1 bis 2 Kohlenstoffatomen,

$R^3$ für einen Rest der Struktur

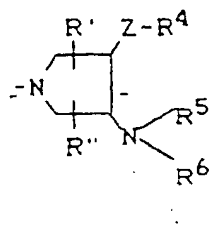

steht, worin

R⁴ für $C_1$-$C_2$-Alkyl,

$R^5$ für H, $C_1$-$C_2$-Alkyl, wobei $R^4$ und $R^5$ gemeinsam auch eine gegebenenfalls durch Methyl substituierte $C_1$-$C_2$-Alkylenbrücke bedeuten können,

$R^6$ für H, $CH_3$, $C_2H_5$, $HOCH_2CH_2$, Benzyl, $C_1$-$C_4$-Alkoxycarbonyl, $C_1$-$C_2$-Acyl,

R' für H oder $CH_3$,

R" für H oder $CH_3$, und

Z für O oder S stehen kann,

A für N oder C-$R^8$ steht, worin

$R^8$ für H, Fluor oder Chlor steht, oder auch gemeinsam mit $R^1$ eine Brücke der Struktur

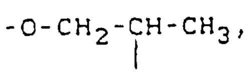

bilden kann.

**4.** Verfahren zur Herstellung von Verbindungen gemäß Anspruch 1 der Formel (I)

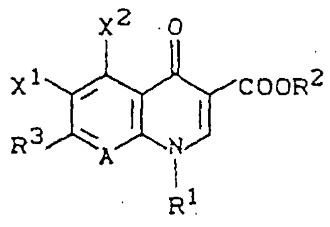

(I),

in welcher

$X^1$, $R^1$, $R^2$, $R^3$ und A die oben angegebene Bedeutung haben und

$X^2$ für Amino, Alkylamino mit 1 bis 4 KohlenstoffasLomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,

**dadurch gekennzeichnet, daß** man eine Verbindung der Formel (IV)

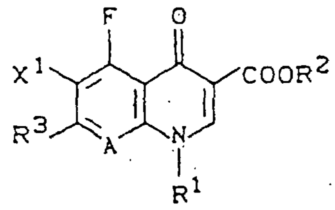

(IV),

in welcher

$X^1$, $R^1$, $R^2$, $R^3$ und A die oben angegebene Bedeutung haben,

mit Verbindungen der Formel (V)

$$X^2\text{-H} \quad (V),$$

in welcher

$X^2$ die oben angegebene Bedeutung hat, gegebenenfalls in Gegenwart von Säurefängern umsetzt.

**5.** Verfahren zur Herstellung von Verbindungen gemäß Anspruch 1 der Formel (Ia)

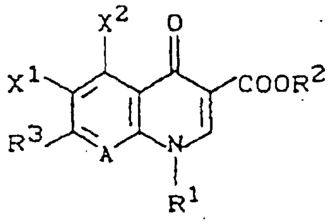

(Ia),

in welcher
$X^1$, $X^2$, $R^1$, $R^2$ und A die oben angegebene Bedeutung haben und $R^3$ für einen Rest der Struktur

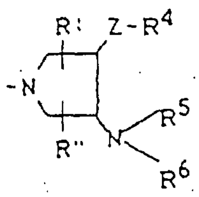

steht, worin
$R^4$, $R^5$, $R^6$, R', R" und Z die oben angegebene Bedeutung haben,
**dadurch gekennzeichnet, daß** man eine Verbindung der Formel (VI)

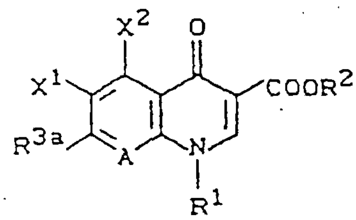

(VI),

in welcher
$X^1$, $X^2$, $R^1$, $R^2$ und A die oben angegebene Bedeutung haben und
$R^{3a}$ für einen Rest der Struktur

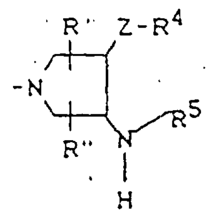

steht,
worin $R^4$, $R^5$, R', R" und Z die oben angegebene Bedeutung haben,
mit Verbindungen der Formel (VII)

$$R^6\text{-}X^a \qquad (VII)$$

in welcher

$R^6$ die oben angegebene Bedeutung hat und

$X^a$ für Chlor, Brom, Iod, Hydroxy oder Acyloxy steht,

gegebenenfalls in Gegenwart von Säurefängern umsetzt.

**6.** Verfahren zur Herstellung von Carbonsäurederivaten in S,S-Konfiguration mit der Formel

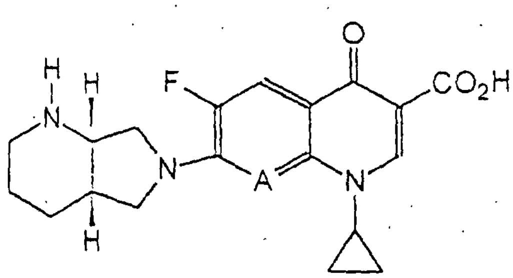

worin A für C-Cl, C-F, oder C-$OCH_3$ steht und deren pharmazeutisch verträgliche Hydrate und Salze, **dadurch gekennzeichnet, dass** man Verbindungen der Formel

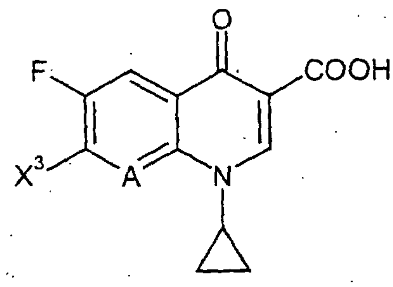

in welcher A die oben angegebene Bedeutung hat und $X^3$ für Halogen, insbesondere Fluor oder Chlor steht, mit der Verbindung der Formel $R^3$-H, in welcher $R^3$ für einen Rest der Struktur

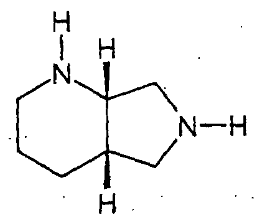

steht,
gegebenenfalls in Gegenwart von Säurefängern umsetzt und gegebenenfalls in $R^3$ enthaltene Schutzgruppen abspaltet und.die so erhaltenen Verbindungen der Formel (I) gegebenenfalls auf an sich bekannte Weise in ihre pharmazeutisch verträglichen Hydrate und Salze überführt.

## Claims

### Claims for the following Contracting States : AT, BE, CH, DE, DK, FR, GB, IT, LI, LU, NL, SE

**1.** 7-(1-Pyrrolidinyl)-3-quinolone- and -naphthyridone-carboxylic acid derivatives of formula (I):

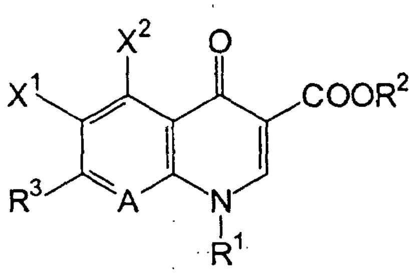

(I)

in which

$X^1$ represents halogen,

$X^2$ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen,

$R^1$ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino,

phenyl optionally substituted by 1 or 2 fluorine atoms,

$R^2$ represents hydrogen, alkyl having 1 to 4 carbon atoms or (5-methyl-2-oxo-1,3-dioxol-4-yl)-methyl, and

$R^3$ represents a radical of the structure

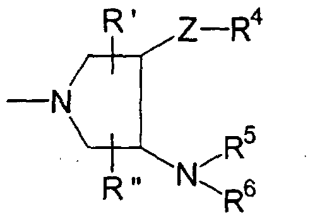

wherein

$R^4$ may represent $C_{1-4}$-alkyl, aryl, $C_{1-4}$-acyl,

$R^5$ may represent H, $C_{1-4}$-alkyl, OH, $OCH_3$,

$R^6$ may represent H, optionally hydroxyl-substituted $C_{1-4}$-alkyl, as well as aryl, heteroaryl, benzyl, $C_{1-4}$-alkoxycarbonyl, $C_{1-4}$-acyl, (5-methyl-2-oxo-1,3-dioxol-4-yl)-methyl or $C_{3-6}$-cycloalkyl,

R' may represent H, $CH_3$ or phenyl,

R" may represent H, $CH_3$ or phenyl, and

Z may represent O or S,

A represents N or C-$R^8$, wherein

$R^8$ represents H, halogen, methyl, cyano, nitro or hydroxyl or, together with $R^1$, may also form a bridge having the structure

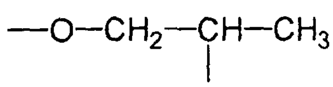 , 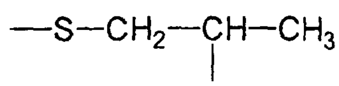

or

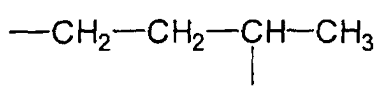

and their pharmaceutically applicable hydrates and acid addition salts as well as the alkali metal, alkaline earth metal, silver and guanidinium salts of the underlying carboxylic acids, except compounds having the formula

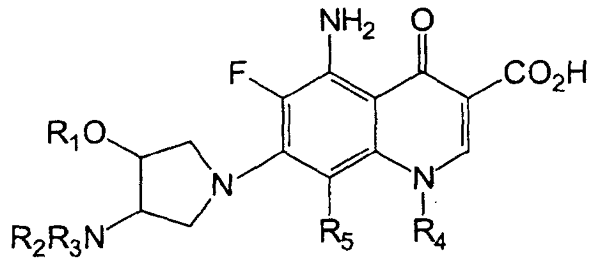

wherein

$R_1$ represents $C_{1-4}$-alkyl,

$R_2/R_3$ represent hydrogen or $C_{1-4}$-alkyl,

$R_4$ represents cyclopropyl, phenyl, halophenyl, thienyl, optionally substituted by $C_{1-4}$-alkyl or halogen, and

$R_5$ represents halogen.

**2.** The compounds of formula (I) according to claim 1, in which

$X^1$ represents fluorine or chlorine,

$X^2$ represents hydrogen, amino, alkylamino having 1 to 2 carbon atoms, dimethylamino, hydroxyl, methoxy, mercapto, methylthio, phenylthio, fluorine, chlorine,

$R^1$ represents alkyl having 1 to 3 carbon atoms, alkenyl having 2 to 3 carbon atoms, cycloalkyl having 3 to 5 carbon atoms, 2-hydroxyethyl, 2-fluoroethyl, methoxy, amino, methylamino, ethylamino, dimethylamino, phenyl optionally substituted by 1 or 2 fluorine atoms,

$R^2$ represents hydrogen, alkyl having 1 to 3 carbon atoms or (5-methyl-2-oxo-1,3-dioxol-4-yl)-methyl,

$R^3$ represents a radical of the structure

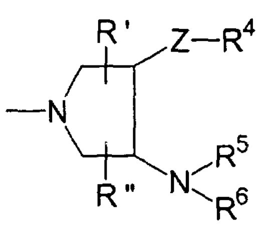

wherein

$R^4$ may represent $C_{1-3}$-alkyl, $C_{1-2}$-acyl,

$R^5$ may represent H, $C_{1-3}$-alkyl, OH, $OCH_3$, wherein $R^4$ and $R^5$ together may also denote a $C_{1-2}$-alkylene bridge optionally mono- or disubstituted by methyl,

$R^6$ may represent H, optionally hydroxyl-substituted $C_{1-3}$-alkyl, as well as phenyl, benzyl, $C_{1-4}$-alkoxycarbonyl, $C_{1-2}$-acyl, (5-methyl-2-oxo-1,3-dioxol-4-yl)-methyl or $C_{3-5}$-alkyl,

R' may represent H or $CH_3$,

R" may represent H or $CH_3$, and

Z may represent O or S,

A represents N or C-$R^8$, wherein

$R^8$ represents H, fluorine, chlorine, bromine or hydroxyl or, together with $R^1$, may also form a bridge having the structure

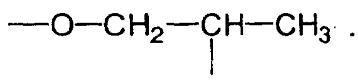

**3.** The compounds of formula (I) according to claim 1, in which

$X^1$ represents fluorine,

$X^2$ represents hydrogen, amino, methylamino, fluorine,

$R^1$ represents alkyl having 1 to 2 carbon atoms, vinyl, cyclopropyl, 2-hydroxyethyl, 2-fluoroethyl, methoxy, methylamino, 4-fluorophenyl, 2,4-difluorophenyl,

$R^2$ represents hydrogen, alkyl having 1 to 2 carbon atoms,

$R^3$ represents a radical of the structure

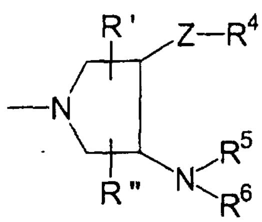

wherein

$R^4$ may represent $C_{1-2}$-alkyl,

$R^5$ may represent H, $C_{1-2}$-alkyl, wherein $R^4$ and $R^5$ together may also denote a $C_{1-2}$-alkylene bridge optionally substituted by methyl,

$R^6$ may represent H, $CH_3$, $C_2H_5$, $HOCH_2CH_2$, benzyl, $C_{1-4}$-alkoxycarbonyl, $C_{1-2}$-acyl,

R' may represent H or $CH_3$,

R" may represent H or $CH_3$, and

Z may represent O or S,

A represents N or C-$R^8$, wherein

$R^8$ represents H, fluorine or chlorine, or, together with $R^1$, may also form a bridge having the structure

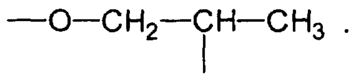

4. A process for the preparation of compounds of formula (I) according to claim 1, **characterized in that** compounds of formula (II)

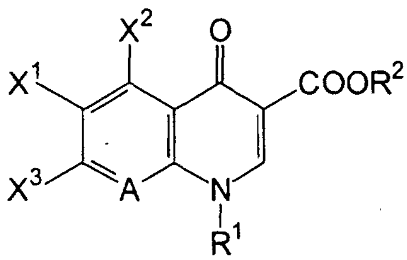

(II)

in which
A, $R^1$, $R^2$, $X^1$ and $X^2$ have the same meanings as defined above, and
$X^3$ represents halogen, particularly fluorine or chlorine,
are reacted with compounds of formula (III)

$$R^3—H \qquad (III)$$

in which
$R^3$ has the same meaning as defined in claim 1,
optionally in the presence of acid scavengers, and optionally the protective groups contained in $R^3$ are cleaved.

5. A process for the preparation of compounds of formula (I) according to claim 1,

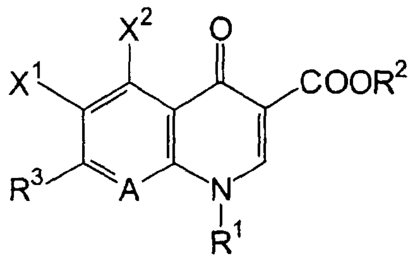

(I)

in which
$X^1$, $R^1$, $R^2$, $R^3$ and A have the same meanings as defined above, and
$X^2$ represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms or arylthio, **characterized in that** a compound of formula (IV)

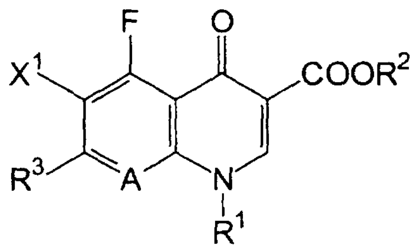

( IV )

in which
$X^1$, $R^1$, $R^2$, $R^3$ and A have the same meanings as defined above,
is reacted with compounds of formula (V)

$$X^2—H \qquad (V),$$

in which
$X^2$ has the same meaning as defined above,
optionally in the presence of acid scavengers.

**6.** A process for the preparation of compounds of formula (Ia) according to claim 1,

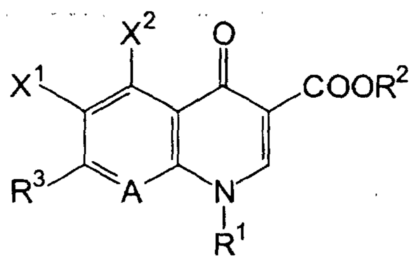

( Ia )

in which
$X^1$, $X^2$, $R^1$, $R^2$ and A have the same meanings as defined above, and
$R^3$ represents a radical of the structure

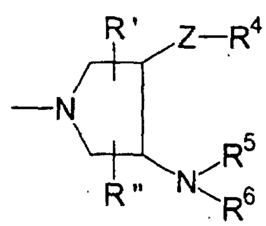

wherein
$R^4$, $R^5$, $R^6$, R', R" and Z have the same meanings as defined above,
**characterized in that** a compound of formula (VI)

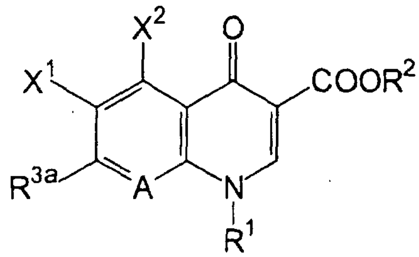

(VI)

in which
$X^1$, $X^2$, $R^1$, $R^2$ and A have the meanings as defined above, and
$R^{3a}$ represents a radical of the structure

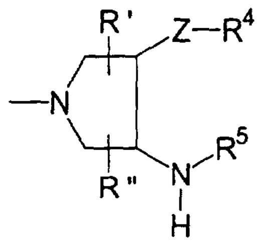

wherein
$R^4$, $R^5$, R', R" and Z have the same meanings as defined above,
is reacted with compounds of formula (VII)

$$R^6—X^a \quad \text{(VII)}$$

in which
$R^6$ has the same meaning as defined above, and
$X^a$ represents chlorine, bromine, iodine, hydroxyl or acyloxy, optionally in the presence of acid scavengers.

**7.** 7-(1-Pyrrolidinyl)-3-quinolone- and -naphthyridone-carboxylic acid derivatives of formula (I) according to claim 1, for use in a method for treating diseases.

**8.** A medicament containing the compounds of formula (I) according to claim 1.

**9.** Use of the compounds of formula (I) according to claim 1 for the preparation of medicaments.

**10.** Use of the compounds of formula (I) according to claim 1 as animal feed additive.

**11.** Animal feed and/or animal feed additive and premixes containing the compounds of formula (I) according to claim 1.

**12.** Carboxylic acid derivatives in S,S-configuration, having the formula

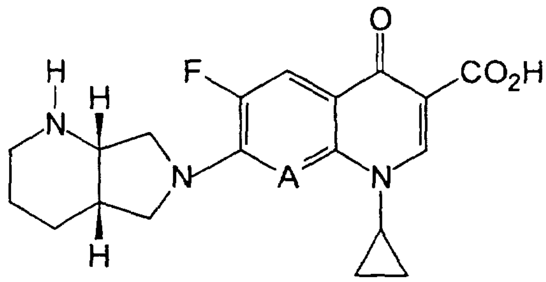

wherein A represents C-Cl, C-F or C-$OCH_3$, and their pharmaceutically acceptable hydrates and salts.

**13.** Medicaments containing a compound according to claim 12.

**14.** Use of the compounds according to claim 12 for the preparation of animal feed, animal feed additives and premixes.

**15.** The compounds according to claim 12 as antibacterial agents.

**16.** Use of the compounds according to claim 12 for the preparation of a medicament for the treatment of bacterial diseases.

**Claims for the following Contracting State : GR**

**1.** 7-(1-Pyrrolidinyl)-3-quinolone- and -naphthyridone-carboxylic acid derivatives of formula (I) :

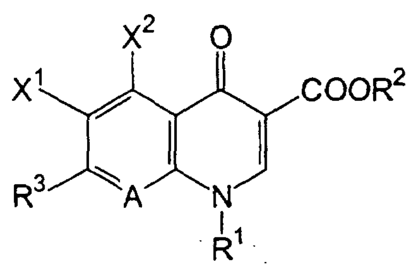

(I)

in which

$X^1$ represents halogen,

$X^2$ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen,

$R^1$ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino, phenyl optionally substituted by 1 or 2 fluorine atoms,

$R^2$ represents hydrogen, alkyl having 1 to 4 carbon atoms or (5-methyl-2-oxo-1,3-dioxol-4-yl)-methyl, and

$R^3$ represents a radical of the structure

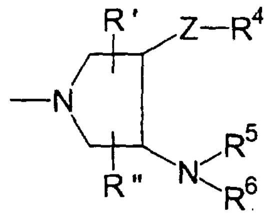

wherein

$R^4$ may represent $C_{1-4}$-alkyl, aryl, $C_{1-4}$-acyl,

$R^5$ may represent H, $C_{1-4}$-alkyl, OH, $OCH_3$,

$R^6$ may represent H, optionally hydroxyl-substituted $C_{1-4}$-alkyl, as well as aryl, heteroaryl, benzyl, $C_{1-4}$-alkoxycarbonyl, $C_{1-4}$-acyl, (5-methyl-2-oxo-1,3-dioxol-4-yl)-methyl or $C_{3-6}$-cycloalkyl,

R' may represent H, $CH_3$ or phenyl,

R" may represent H, $CH_3$ or phenyl, and

Z may represent O or S,

A represents N or C-$R^8$, wherein

$R^8$ represents H, halogen, methyl, cyano, nitro or hydroxyl or, together with $R^1$, may also form a bridge having the structure

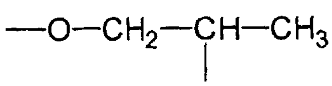 , 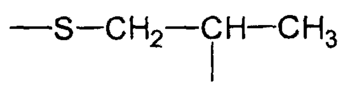

or

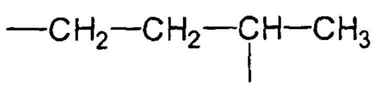

and their pharmaceutically applicable hydrates and acid addition salts as well as the alkali metal, alkaline earth metal, silver and guanidinium salts of the underlying carboxylic acids, except compounds having the formula

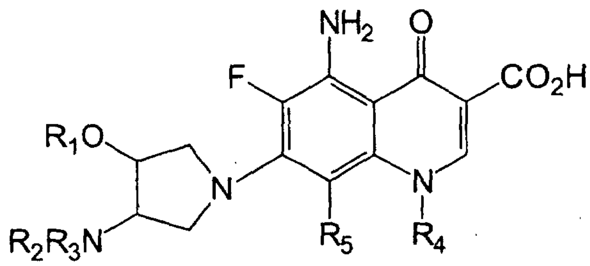

wherein

$R_1$ represents $C_{1-4}$-alkyl,

$R_2/R_3$ represent hydrogen or $C_{1-4}$-alkyl,

$R_4$ represents cyclopropyl, phenyl, halophenyl, thienyl, optionally substituted by $C_{1-4}$-alkyl or halogen, and

$R_5$ represents halogen.

**2.** The compounds of formula (I) according to claim 1, in which

$X^1$ represents fluorine or chlorine,

$X^2$ represents hydrogen, amino, alkylamino having 1 to 2 carbon atoms, dimethylamino, hydroxyl, methoxy, mercapto, methylthio, phenylthio, fluorine, chlorine,

$R^1$ represents alkyl having 1 to 3 carbon atoms, alkenyl having 2 to 3 carbon atoms, cycloalkyl having 3 to 5 carbon atoms, 2-hydroxyethyl, 2-fluoroethyl, methoxy, amino, methylamino, ethylamino, dimethylamino, phenyl optionally substituted by 1 or 2 fluorine atoms,

$R^2$ represents hydrogen, alkyl having 1 to 3 carbon atoms or (5-methyl-2-oxo-1,3-dioxol-4-yl)-methyl,

$R^3$ represents a radical of the structure

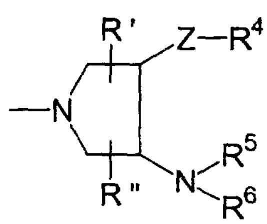

wherein

$R^4$ may represent $C_{1-3}$-alkyl, $C_{1-2}$-acyl,

$R^5$ may represent H, $C_{1-3}$-alkyl, OH, $OCH_3$, wherein $R^4$ and $R^5$ together may also denote a $C_{1-2}$-alkylene bridge, optionally mono- or disubstituted by methyl,

$R^6$ may represent H, optionally hydroxyl-substituted $C_{1-3}$-alkyl, as well as phenyl, benzyl, $C_{1-4}$-alkoxycarbonyl, $C_{1-2}$-acyl, (5-methyl-2-oxo-1,3-dioxol-4-yl)-methyl or $C_{3-5}$-alkyl,

R' may represent H or $CH_3$,

R" may represent H or $CH_3$, and

Z may represent O or S,

A represents N or C-$R^8$, wherein

$R^8$ represents H, fluorine, chlorine, bromine or hydroxyl or, together with $R^1$, may also form a bridge having the structure

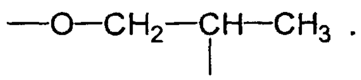

**3.** The compounds of formula (I) according to claim 1, in which

$X^1$ represents fluorine,

$X^2$ represents hydrogen, amino, methylamino, fluorine,

$R^1$ represents alkyl having 1 to 2 carbon atoms, vinyl, cyclopropyl, 2-hydroxyethyl, 2-fluoroethyl, methoxy, methylamino, 4-fluorophenyl, 2,4-difluorophenyl,

$R^2$ represents hydrogen, alkyl having 1 to 2 carbon atoms,

$R^3$ represents a radical of the structure

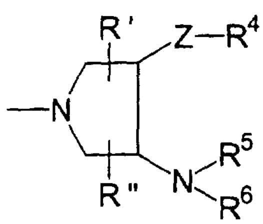

wherein

$R^4$ may represent $C_{1-2}$-alkyl,

$R^5$ may represent H, $C_{1-2}$-alkyl, wherein $R^4$ and $R^5$ together may also denote a $C_{1-2}$-alkylene bridge, optionally substituted by methyl,

$R^6$ may represent H, $CH_3$, $C_2H_5$, $HOCH_2CH_2$, benzyl, $C_{1-4}$-alkoxycarbonyl, $C_{1-2}$-acyl,

R' may represent H or $CH_3$,

R" may represent H or $CH_3$, and

Z may represent O or S,

A represents N or C-$R^8$, wherein

$R^8$ represents H, fluorine or chlorine, or, together with $R^1$, may also form a bridge having the structure

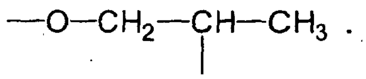

4. A process for the preparation of compounds of formula (I) according to claim 1, **characterized in that** compounds of formula (II)

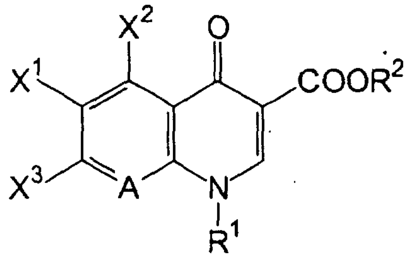

( II )

in which
A, $R^1$, $R^2$, $X^1$ and $X^2$ have the same meanings as defined above, and
$X^3$ represents halogen, particularly fluorine or chlorine,
are reacted with compounds of formula (III)

$$R^3—H \qquad (III)$$

in which
$R^3$ has the same meaning as defined in claim 1,
optionally in the presence of acid scavengers, and optionally the protective groups contained in $R^3$ are cleaved.

5. A process for the preparation of compounds of formula (I) according to claim 1,

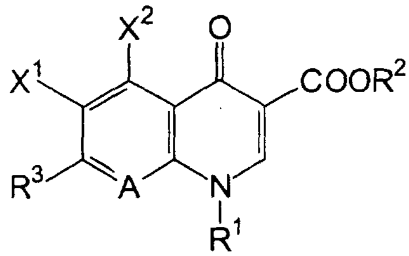

( I )

in which
$X^1$, $R^1$, $R^2$, $R^3$ and A have the same meanings as defined above, and
$X^2$ represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms or arylthio, **characterized in that** a compound of formula (IV)

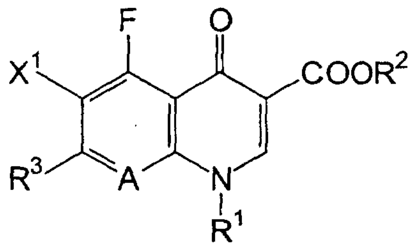

(IV)

in which
$X^1$, $R^1$, $R^2$, $R^3$ and A have the same meanings as defined above,
is reacted with compounds of formula (V)

$$X^2—H \quad (V),$$

in which
$X^2$ has the same meaning as defined above,
optionally in the presence of acid scavengers.

**6.** A process for the preparation of compounds of formula (Ia) according to claim 1,

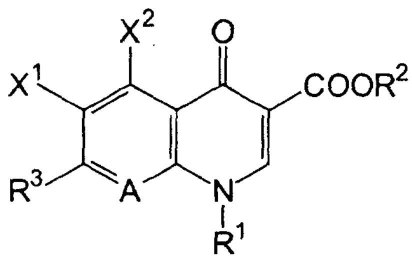

(Ia)

in which
$X^1$, $X^2$, $R^1$, $R^2$ and A have the same meanings as defined above, and
$R^3$ represents a radical of the structure

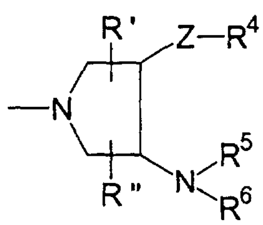

wherein
$R^4$, $R^5$, $R^6$, R', R" and Z have the same meanings as defined above,
**characterized in that** a compound of formula (VI)

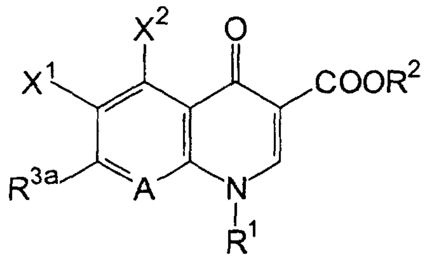

(VI)

in which
$X^1$, $X^2$, $R^1$, $R^2$ and A have the meanings as defined above, and
$R^{3a}$ represents a radical of the structure

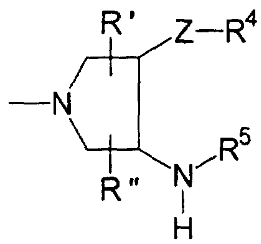

wherein
$R^4$, $R^5$, R', R" and Z have the same meanings as defined above,
is reacted with compounds of formula (VII)

$$R^6—X^a \quad \text{(VII)}$$

in which
$R^6$ has the same meaning as defined above, and
$X^a$ represents chlorine, bromine, iodine, hydroxyl or acyloxy, optionally in the presence of acid scavengers.

**7.** Use of the compounds of formula (I) according to claim 1, as animal feed additive.

**8.** Animal feed and/or animal feed additive and premixes containing the compounds of formula (I) according to claim 1.

**9.** Carboxylic acid derivatives in S,S-configuration, having the formula

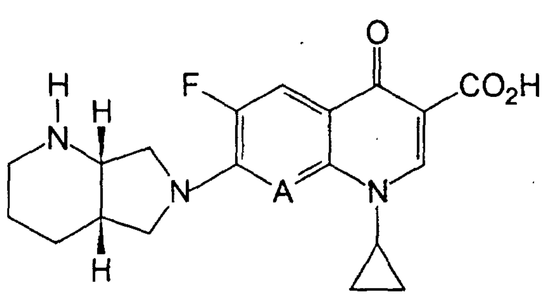

wherein A represents C-Cl, C-F or C-$OCH_3$, and their pharmaceutically acceptable hydrates and salts.

**10.** Use of the compounds according to claim 9 for the preparation of animal feed, animal feed additives and premixes.

**Claims for the following Contracting State : ES**

**1.** Process for the preparation of 7-(1-Pyrrolidinyl)-3-quinolone- and -naphthyridone-carboxylic acid derivatives of formula (I):

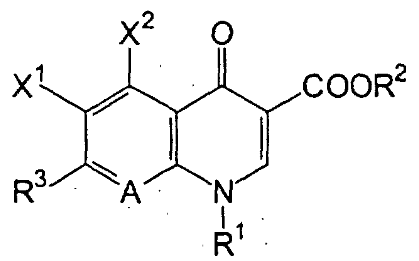

(I)

in which

$X^1$ represents halogen,

$X^2$ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen,

$R^1$ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino, phenyl optionally substituted by 1 or 2 fluorine atoms,

$R^2$ represents hydrogen, alkyl having 1 to 4 carbon atoms or (5-methyl-2-oxo-1,3-dioxol-4-yl)-methyl, and

$R^3$ represents a radical of the structure

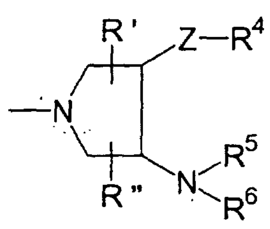

wherein

$R^4$ may represent $C_{1-4}$-alkyl, aryl, $C_{1-4}$-acyl,

$R^5$ may represent H, $C_{1-4}$-alkyl, OH, $OCH_3$,

$R^6$ may represent H, optionally hydroxyl-substituted $C_{1-4}$-alkyl, as well as aryl, heteroaryl, benzyl, $C_{1-4}$-alkoxycarbonyl, $C_{1-4}$-acyl, (5-methyl-2-oxo-1,3-dioxol-4-yl)-methyl or $C_{3-6}$-cycloalkyl,

R' may represent H, $CH_3$ or phenyl,

R" may represent H, $CH_3$ or phenyl, and

Z may represent O or S,

A represents N or C-$R^8$, wherein

$R^8$ represents H, halogen, methyl, cyano, nitro or hydroxyl or, together with $R^1$, may also form a bridge having the structure

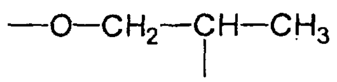 , 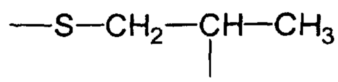

or

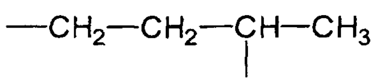

and their pharmaceutically applicable hydrates and acid addition salts as well as the alkali metal, alkaline earth metal, silver and guanidinium salts of the underlying carboxylic acids, except compounds having the formula

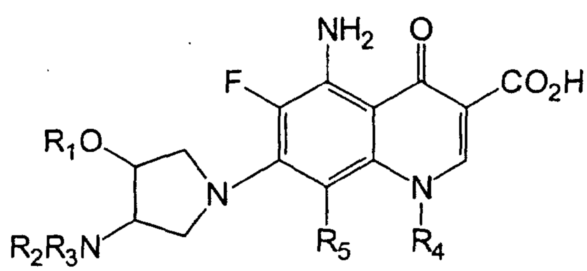

wherein

$R_1$ represents $C_{1-4}$-alkyl,

$R_2$/$R_3$ represent hydrogen or $C_{1-4}$-alkyl,

$R_4$ represents cyclopropyl, phenyl, halophenyl, thienyl, optionally substituted by $C_{1-4}$-alkyl or halogen, and

$R_5$ represents halogen,

**characterized in that** compounds of formula (II)

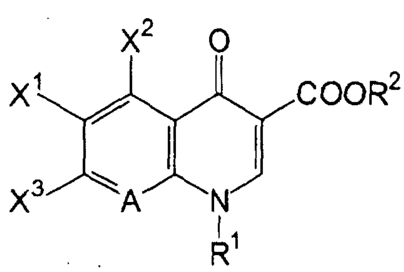

(II)

in which

A, $R^1$, $R^2$, $X^1$ and $X^2$ have the same meanings as defined above, and
$X^3$ represents halogen, particularly fluorine or chlorine,
are reacted with compounds of formula (III)

$$R^3—H \quad \text{(III)}$$

in which $R^3$ has the same meaning as defined above,
optionally in the presence of acid scavengers, and optionally the protective groups contained in $R^3$ are cleaved, and optionally the compounds of formula (I) thus obtained are converted in a manner known as such, to the pharmaceutically applicable hydrates or acid addition salts thereof or the alkali metal, alkaline earth metal, silver and guanidinium salts of the underlying carboxylic acids.

**2.** The process according to claim 1, in which

$X^1$ represents fluorine or chlorine,

$X^2$ represents hydrogen, amino, alkylamino having 1 to 2 carbon atoms, dimethylamino, hydroxyl, methoxy, mercapto, methylthio, phenylthio, fluorine, chlorine,

$R^1$ represents alkyl having 1 to 3 carbon atoms, alkenyl having 2 to 3 carbon atoms, cycloalkyl having 3 to 5 carbon atoms, 2-hydroxyethyl, 2-fluoroethyl, methoxy, amino, methylamino, ethylamino, dimethylamino, phenyl optionally substituted by 1 or 2 fluorine atoms,

$R^2$ represents hydrogen, alkyl having 1 to 3 carbon atoms or (5-methyl-2-oxo-1,3-dioxol-4-yl)-methyl,

$R^3$ represents a radical of the structure

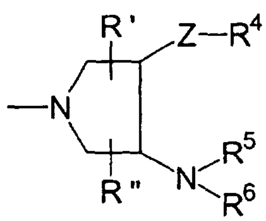

wherein

$R^4$ may represent $C_{1-3}$-alkyl, $C_{1-2}$-acyl,

$R^5$ may represent H, $C_{1-3}$-alkyl, OH, $OCH_3$, wherein $R^4$ and $R^5$ together may also denote a $C_{1-2}$-alkylene bridge optionally mono-or disubstituted by methyl,

$R^6$ may represent H, optionally hydroxyl-substituted $C_{1-3}$-alkyl, as well as phenyl, benzyl, $C_{1-4}$-alkoxycarbonyl, $C_{1-2}$-acyl, (5-methyl-2-oxo-1,3-dioxol-4-yl)-methyl or $C_{3-5}$-alkyl,

R' may represent H or $CH_3$,

R" may represent H or $CH_3$, and

Z may represent O or S,

A represents N or C-$R^8$, wherein

$R^8$ represents H, fluorine, chlorine, bromine or hydroxyl or, together with $R^1$, may also form a bridge

having the structure

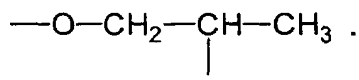

**3.** The process according to claim 1, in which

$X^1$ represents fluorine,

$X^2$ represents hydrogen, amino, methylamino, fluorine,

$R^1$ represents alkyl having 1 to 2 carbon atoms, vinyl, cyclopropyl, 2-hydroxyethyl, 2-fluoroethyl, methoxy, methylamino, 4-fluorophenyl, 2,4-difluorophenyl,

$R^2$ represents hydrogen, alkyl having 1 to 2 carbon atoms,

$R^3$ represents a radical of the structure

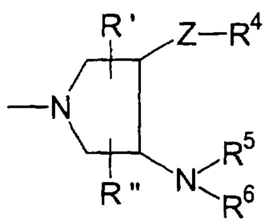

wherein

$R^4$ may represent $C_{1-2}$-alkyl,

$R^5$ may represent H, $C_{1-2}$-alkyl, wherein $R^4$ and $R^5$ together may also denote a $C_{1-2}$-alkylene bridge optionally substituted by methyl,

$R^6$ may represent H, $CH_3$, $C_2H_5$, $HOCH_2CH_2$, benzyl, $C_{1-4}$-alkoxycarbonyl, $C_{1-2}$-acyl,

R' may represent H or $CH_3$,

R" may represent H or $CH_3$, and

Z may represent O or S,

A represents N or C-$R^8$, wherein

$R^8$ represents H, fluorine or chlorine, or, together with $R^1$, may also form a bridge having the structure

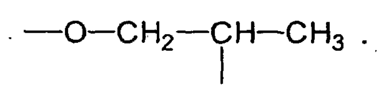

**4.** A process for the preparation of compounds of formula (I) according to claim 1,

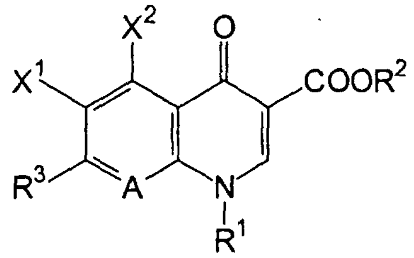

(I)

in which
$X^1$, $R^1$, $R^2$, $R^3$ and A have the same meanings as defined above, and
$X^2$ represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms or arylthio, **characterized in that** a compound of formula (IV)

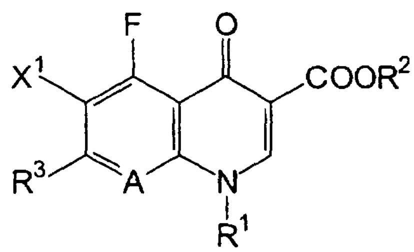

(IV)

in which
$X^1$, $R^1$, $R^2$, $R^3$ and A have the same meanings as defined above,
is reacted with compounds of formula (V)

$$X^2—H \quad (V),$$

in which
$X^2$ has the same meaning as defined above,
optionally in the presence of acid scavengers.

**5.** A process for the preparation of compounds of formula (Ia) according to claim 1,

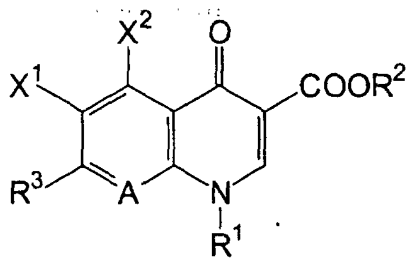

(Ia)

in which
$X^1$, $X^2$, $R^1$, $R^2$ and A have the same meanings as defined above, and
$R^3$ represents a radical of the structure

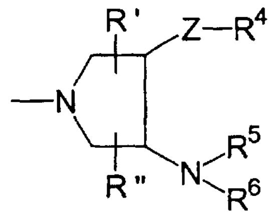

wherein
$R^4$, $R^5$, $R^6$, R', R" and Z have the same meanings as defined above,
**characterized in that** a compound of formula (VI)

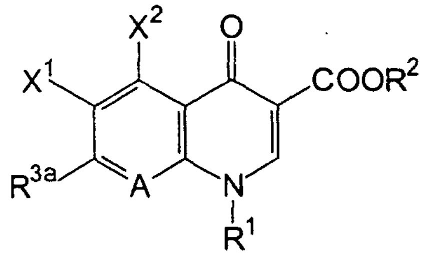

(VI)

in which
$X^1$, $X^2$, $R^1$, $R^2$ and A have the meanings as defined above, and
$R^{3a}$ represents a radical of the structure

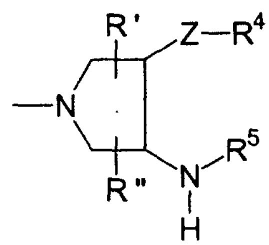

wherein
$R^4$, $R^5$, R', R" and Z have the same meanings as defined above,
is reacted with compounds of formula (VII)

$$R^6—X^a \qquad \text{(VII)}$$

in which
$R^6$ has the same meaning as defined above, and
$X^a$ represents chlorine, bromine, iodine, hydroxyl or acyloxy, optionally in the presence of acid scavengers.

**6.** Process for the preparation of carboxylic acid derivatives in S,S-configuration, having the formula

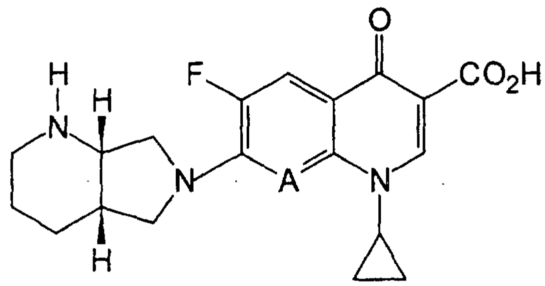

wherein A represents C-Cl, C-F or C-$OCH_3$, and pharmaceutically acceptable hydrates and salts thereof, **characterized in that** compounds of formula

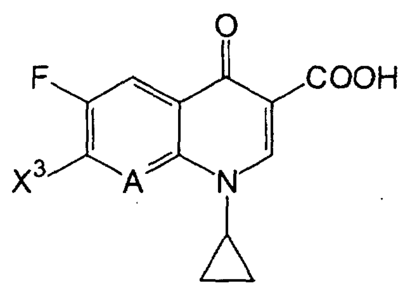

in which A has the same meaning as defined above, and $X^3$ represents halogen, particularly fluorine or chlorine, are reacted with the compound of formula $R^3$-H, in which $R^3$ represents a radical of the structure

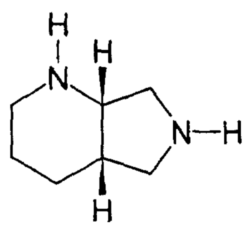

optionally in the presence of acid scavengers, and optionally the protective groups contained in $R^3$ are cleaved, and optionally the compounds of formula (I) thus obtained are converted in a manner known as such, to the pharmaceutically acceptable hydrates or acid addition salts thereof.

## Revendications

### Revendications pour les Etats contractants suivants : AT, BE, CH, DE, DK, FR, GB, IT, LI, LU, NL, SE

1. Dérivés d'acides 7-(1-pyrrolidinyl)-3-quinolone- et -naphtyridone-carboxyliques de formule (I)

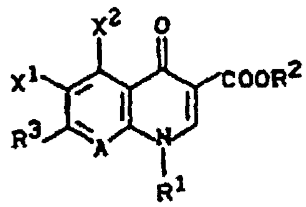

(I),

dans laquelle

- $X^1$ représente un halogène,
- $X^2$ est l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
- $R^1$ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
- $R^2$ est l'hydrogène, un groupe alkyle ayant 1 à 4 atomes de carbone ou un groupe (5-méthyl-2-oxo-1,3-dioxol-4-yl)méthyle et
- $R^3$ est un reste de structure

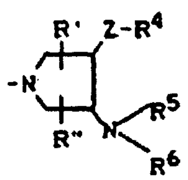

où

- $R^4$ peut représenter un groupe alkyle en $C_1$ à $C_4$, aryle, acyle en $C_1$ à $C_4$
- $R^5$ peut représenter H, un groupe alkyle en $C_1$ à $C_4$, OH, $OCH_3$,
- $R^6$ peut représenter H, un groupe alkyle en $C_1$ à $C_4$ éventuellement substitué par un radical hydroxy, ainsi qu'un groupe aryle, hétéroaryle, benzyle, (alkoxy en $C_1$ à $C_4$) carbonyle, acyle en $C_1$ à $C_4$, (5-méthyl-2-oxo-1,3-dioxol-4-yl)méthyle ou cycloalkyle en $C_3$ à $C_6$,
- R' peut représenter H, un groupe $CH_3$ ou phényle,
- R" peut représenter H, $CH_3$ ou un groupe phényle, et
- Z peut représenter O ou S,

A représente N ou un groupe C-$R^8$, dans lequel

- $R^8$ est l'hydrogène, un halogène, un groupe méthyle, cyano, nitro ou hydroxy, ou peut aussi former conjointement avec $R^1$ un pont de structure

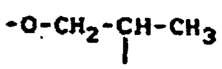,
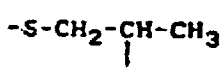

oder

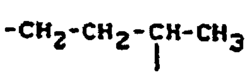

et leurs hydrates et leurs sels d'addition d'acides pharmaceutiquement utilisables ainsi que les sels de métaux alcalins, de métaux alcalino-terreux, d'argent et de guanidinium, des acides carboxyliques correspondants, à l'exception de composés de formule

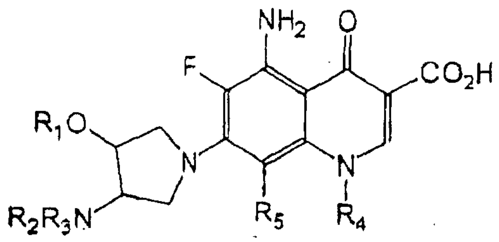

dans laquelle

$R_1$ est un groupe alkyle en $C_1$ à $C_4$,

$R_2/R_3$ représentent l'hydrogène ou un groupe alkyle en $C_1$ à $C_4$,

$R_4$ est un groupe cyclopropyle, phényle, halogénophényle, thiényle, portant au choix un substituant alkyle en $C_1$ à $C_4$ ou halogéno, et

$R_5$ est un halogène.

**2.** Composés de formule (I) suivant la revendication 1, formule dans laquelle

$X^1$ représente le fluor ou le chlore,

$X^2$ est l'hydrogène, un groupe amino, alkylamino ayant 1 ou 2 atomes de carbone, diméthylamino, hydroxy, méthoxy, mercapto, méthylthio, phénylthio, le fluor, le chlore,

$R^1$ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,

$R^2$ est l'hydrogène, un groupe alkyle ayant 1 à 3 atomes de carbone ou (5-méthyl-2-oxo-1,3-dioxol-4-yl)méthyle,

$R^3$ représente un reste de structure

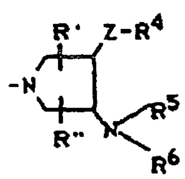

où

$R^4$ peut représenter un groupe alkyle en $C_1$ à $C_3$, acyle en $C_1$ ou $C_2$

$R^5$ peut représenter H, un groupe alkyle en $C_1$ à $C_3$, OH, $OCH_3$, $R^4$ et $R^5$ pouvant aussi former ensemble un pont alkylène en $C_1$ ou $C_2$ éventuellement substitué une ou deux fois par un radical méthyle,

$R^6$ peut représenter H, un groupe alkyle en $C_1$ à $C_3$ éventuellement substitué par un radical hydroxy, ainsi qu'un groupe phényle, benzyle, (alkoxy en $C_1$ à $C_4$)carbonyle, acyle en $C_1$ ou $C_2$, (5-méthyl-2-oxo-1,3-dioxol-4-yl)méthyle ou alkyle en $C_3$ à $C_5$,

R' peut représenter H ou un groupe $CH_3$,

R" peut représenter H ou un groupe $CH_3$, et

Z peut représenter O ou S,

A représente N ou un groupe C-$R^8$, dans lequel

$R^8$ représente l'hydrogène, le fluor, le chlore, le brome ou un groupe hydroxy ou peut aussi former conjointement avec $R^1$ un pont de structure

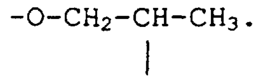

3. Composés de formule (I) suivant la revendication 1, formule dans laquelle

$X^1$ est le fluor,
$X^2$ est l'hydrogène, un groupe amino, méthylamino, le fluor,
$R^1$ est un groupe alkyle ayant 1 ou 2 atomes de carbone, vinyle, cyclopropyle, 2-hydroxyéthyle, 2-fluoréthyle, , méthoxy, méthylamino, 4-fluorophényle, 2,4-difluorophényle,
$R^2$ est l'hydrogène, un groupe alkyle ayant 1 ou 2 atomes de carbone,
$R^3$ est un reste de structure

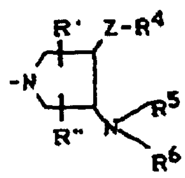

où
$R^4$ peut être un groupe alkyle en $C_1$ ou $C_2$

$R^5$ peut représenter H, un groupe alkyle en $C_1$ ou $C_2$, $R^4$ et $R^5$ pouvant former ensemble un pont alkylène en $C_1$ ou $C_2$ éventuellement substitué par un radical méthyle,
$R^6$ peut être l'hydrogène, un groupe $CH_3$, $C_2H_5$, $HOCH_2CH_2$, benzyle, (alkoxy en $C_1$ à $C_4$)carbonyle, acyle en $C_1$ ou $C_2$,
R' peut représenter H ou $CH_3$,
R" peut représenter H ou $CH_3$, et
Z peut représenter O ou S,

A représente l'azote ou un groupe C-$R^8$ dans lequel

$R^8$ est l'hydrogène, le fluor, le chlore, ou peut aussi former conjointement avec $R^1$ un pont de structure

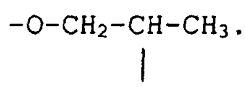

4. Procédé de production de composés de formule (I) suivant la revendication 1, **caractérisé en ce qu'**on fait réagir des composés de formule (II)

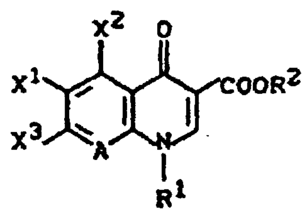 (II),

dans laquelle

A, $R^1$, $R^2$, $X^1$ et $X^2$ ont la définition indiquée ci-dessus et

$X^3$ représente un halogène, en particulier le fluor ou le chlore, avec des composés de formule (III)

$$R^3\text{-H} \qquad (III)$$

dans laquelle

$R^3$ a la définition indiquée dans la revendication 1,

le cas échéant en présence d'accepteurs d'acides, et on élimine éventuellement des groupes protecteurs contenus dans $R^3$.

**5.** Procédé de production de composés suivant la revendication 1, de formule (I)

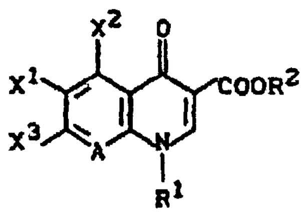

(I),

dans laquelle

$X^1$, $R^1$, $R^2$, $R^3$ et A ont la définition indiquée ci-dessus et,

$X^2$ est un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio,

**caractérisé en ce qu'**on fait réagir un composé de formule (IV)

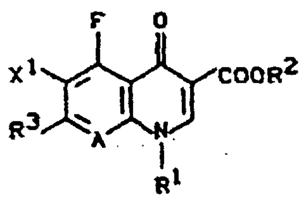

(IV),

dans laquelle

$X^1$, $R^1$, $R^2$, $R^3$ et A ont la définition indiquée ci-dessus, avec des composés de formule (V)

$$X^2\text{-H} \qquad (V),$$

dans laquelle

$X^2$ a la définition indiquée ci-dessus, le cas échéant en présence d'accepteurs d'acides.

**6.** Procédé de production de composés suivant la revendication 1, de formule (Ia)

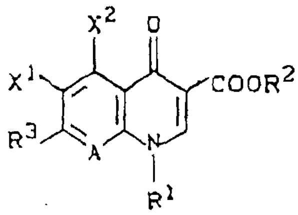

(Ia),

dans laquelle
$X^1$, $X^2$, $R^1$, $R^2$ et A ont la définition indiquée ci-dessus, et $R^3$ représente un reste de structure

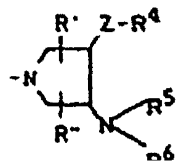

où $R^4$, $R^5$, $R^6$, R', R'' et Z ont la définition indiquée ci-dessus,
**caractérisé en ce qu'**on fait réagir un composé de formule (VI)

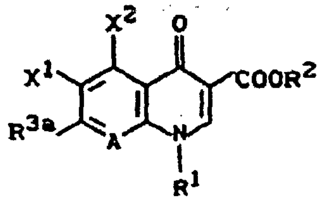

(VI),

dans laquelle
$X^1$, $X^2$, $R^1$, $R^2$ et A ont la définition indiquée ci-dessus et
$R^{3a}$ représente un reste de structure

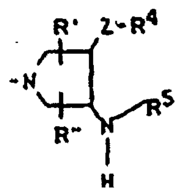

où $R^4$, $R^5$, R', R'' et Z ont la définition indiquée ci-dessus, avec des composés de formule (VII)

$$R^6\text{-}X^a \qquad \text{(VII)},$$

dans laquelle

$R^6$ a la définition indiquée ci-dessus et
$X^a$ représente le chlore, le brome, l'iode, un groupe hydroxy ou acyloxy,

le cas échéant en présence d'accepteurs d'acides.

**7.** Dérivés d'acides 7-(1-pyrrolidinyl)-3-quinolone- et -naphtyridone-carboxyliques de formule (I) suivant la revendication 1, destinés à être utilisés dans un procédé pour combattre des maladies.

**8.** Médicaments contenant des composés de formule (I) suivant la revendication 1.

**9.** Utilisation de composés de formule (I) suivant la revendication 1 pour la préparation de médicaments.

**10.** Utilisation de composés de formule (I) suivant la revendication 1 comme additifs pour des aliments pour animaux.

**11.** Aliments pour animaux ou additifs pour des aliments pour animaux et mélanges préalables contenant des composés de formule (I) suivant la revendication 1.

**12.** Dérivés d'acides carboxyliques à configuration S,S de formule

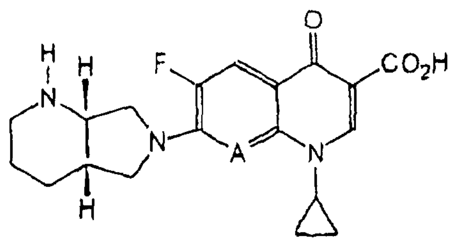

dans laquelle A représente C-Cl, C-F ou C-$OCH_3$ et leurs hydrates et sels acceptables du point de vue pharmaceutique.

**13.** Médicament contenant un composé suivant la revendication 12.

**14.** Utilisation de composés suivant la revendication 12 pour la préparation d'aliments pour animaux, d'additifs pour des aliments pour animaux et de mélanges préalables.

**15.** Composés suivant la revendication 12, destinés à être utilisés comme agents antibactériens.

**16.** Utilisation de composés suivant la revendication 12 pour la préparation d'un médicament destiné au traitement de maladies bactériennes.

**Revendications pour l'Etat contractant suivant : GR**

**1.** Dérivés d'acides 7-(1-pyrrolidinyl)-3-quinolone- et -naphtyridone-carboxyliques de formule (I)

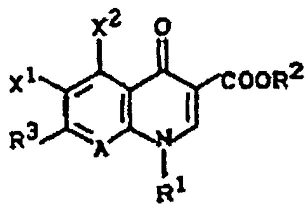

(I),

dans laquelle

$X^1$ représente un halogène,

$X^2$ est l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,

$R^1$ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,

$R^2$ est l'hydrogène, un groupe alkyle ayant 1 à 4 atomes de carbone ou un groupe (5-méthyl-2-oxo-1,3-dioxol-4-yl)méthyle et

$R^3$ est un reste de structure

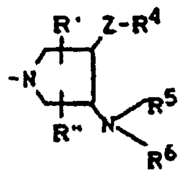

où

$R^4$ peut représenter un groupe alkyle en $C_1$ à $C_4$, aryle ou acyle en $C_1$ à $C_4$,

$R^5$ peut représenter H, un groupe alkyle en $C_1$ à $C_4$, OH, $OCH_3$,

$R^6$ peut représenter H, un groupe alkyle en $C_1$ à $C_4$ éventuellement substitué par un radical hydroxy, ainsi qu'un groupe aryle, hétéroaryle, benzyle, (alkoxy en $C_1$ à $C_4$)carbonyle, acyle en $C_1$ à $C_4$, (5-méthyl-2-oxo-1,3-dioxol-4-yl)méthyle ou cycloalkyle en $C_3$ à $C_6$,

R' peut représenter H, un groupe $CH_3$ ou phényle,

R" peut représenter H, $CH_3$ ou un groupe phényle, et

Z peut représenter O ou S,

A représente N ou un groupe C-$R^8$, dans lequel

$R^8$ est de l'hydrogène, un halogène, un groupe méthyle, cyano, nitro ou hydroxy, ou peut aussi former conjointement avec $R^1$ un pont de structure

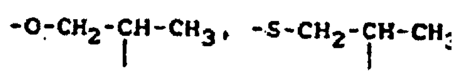

oder

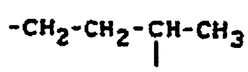

et leurs hydrates et leurs sels d'addition d'acides pharmaceutiquement utilisables ainsi que les sels de métaux alcalins, de métaux alcalino-terreux, d'argent et de guanidinium, des acides carboxyliques correspondants, à l'exception de composés de formule

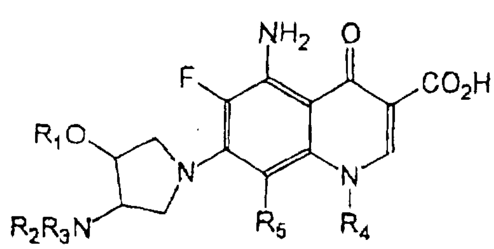

dans laquelle

$R_1$ est un groupe alkyle en $C_1$ à $C_4$,

$R_2/R_3$ représentent l'hydrogène ou un groupe alkyle en $C_1$ à $C_4$,

$R_4$ est un groupe cyclopropyle, phényle, halogénophényle, thiényle, portant au choix un substituant alkyle en $C_1$ à $C_4$ ou halogéno, et

$R_5$ est un halogène.

**2.** Composés de formule (I) suivant la revendication 1, formule dans laquelle

$X^1$ représente le fluor ou le chlore,

$X^2$ est l'hydrogène, un groupe amino, alkylamino ayant 1 ou 2 atomes de carbone, diméthylamino, hydroxy, méthoxy, mercapto, méthylthio, phénylthio, le fluor, le chlore,

$R^1$ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,

$R^2$ est l'hydrogène, un groupe alkyle ayant 1 à 3 atomes de carbone ou (5-méthyl-2-oxo-1,3-dioxol-4-yl)méthyle,

$R^3$ représente un reste de structure

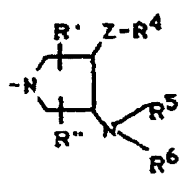

où

$R^4$ peut être un groupe alkyle en $C_1$ à $C_3$, acyle en $C_1$ ou $C_2$,

$R^5$ peut représenter H, un groupe alkyle en $C_1$ à $C_3$, OH, $OCH_3$, $R^4$ et $R^5$ pouvant aussi former ensemble un pont alkylène en $C_1$ ou $C_2$ éventuellement substitué une ou deux fois par un radical méthyle,

$R^6$ peut représenter H, un groupe alkyle en $C_1$ à $C_3$ éventuellement substitué par un radical hydroxy, ainsi qu'un groupe phényle, benzyle, (alkoxy en $C_1$ à $C_4$)carbonyle, acyle en $C_1$ ou $C_2$, (5-méthyl-2-oxo-1,3-dioxol-4-yl)méthyle ou alkyle en $C_3$ à $C_5$,

R' peut représenter H ou un groupe $CH_3$,

R" peut représenter H ou un groupe $CH_3$, et

Z peut représenter O ou S,

A représente N ou un groupe C-$R^8$, dans lequel

$R^8$ représente l'hydrogène, le fluor, le chlore, le brome ou un groupe hydroxy ou peut aussi former conjointement avec $R^1$ un pont de structure

$$-O-CH_2-CH-CH_3.$$

**3.** Composés de formule (I) suivant la revendication 1, formule dans laquelle

$X^1$ est le fluor,

$X^2$ est l'hydrogène, un groupe amino, méthylamino, le fluor,

$R^1$ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,

$R^2$ est l'hydrogène, un groupe alkyle ayant 1 ou 2 atomes de carbone,

$R^3$ est un reste de structure

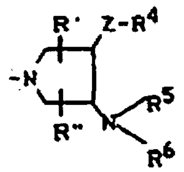

où

- $R^4$ peut être un groupe alkyle en $C_1$ ou $C_2$,
- $R^5$ peut représenter H, un groupe alkyle en $C_1$ ou $C_2$, $R^4$ et $R^5$ pouvant former ensemble un pont alkylène en $C_1$ ou $C_2$ éventuellement substitué par un radical méthyle,
- $R^6$ peut être l'hydrogène, un groupe $CH_3$, $C_2H_5$, $HOCH_2CH_2$, benzyle, (alkoxy en $C_1$ à $C_4$)carbonyle, acyle en $C_1$ ou $C_2$,
- R' peut représenter H ou $CH_3$,
- R" peut représenter H ou $CH_3$, et
- Z peut représenter O ou S,

A représente l'azote ou un groupe C-$R^8$ dans lequel

$R^8$ est l'hydrogène, le fluor ou le chlore, ou peut aussi former conjointement avec $R^1$ un pont de structure

$$-O-CH_2-CH-CH_3.$$

**4.** Procédé de production de composés suivant la revendication 1 de formule (I), **caractérisé en ce qu'**on fait réagir des composés de formule (II)

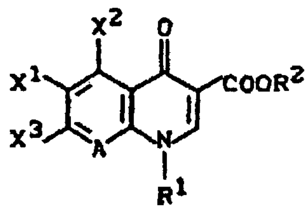

(II),

dans laquelle

A, $R^1$, $R^2$, $X^1$ et $X^2$ ont la définition indiquée ci-dessus et
$X^3$ représente un halogène, en particulier le fluor ou le chlore, avec des composés de formule (III)

$$R^3\text{-H} \quad \text{(III)}$$

dans laquelle
$R^3$ a la définition indiquée dans la revendication 1,

le cas échéant en présence d'accepteurs d'acides, et on élimine éventuellement des groupes protecteurs contenus dans $R^3$.

**5.** Procédé de production de composés suivant la revendication 1, de formule (I)

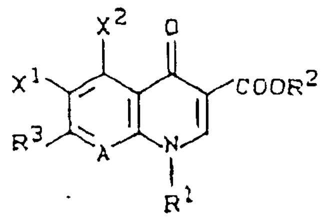

(I),

dans laquelle

| | |
|---|---|
| $X^1$, $R^1$, $R^2$, $R^3$ et A | ont la définition indiquée ci-dessus et, |
| $X^2$ | est un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio, |

**caractérisé en ce qu'**on fait réagir un composé de formule (IV)

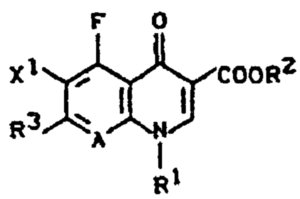

(IV),

dans laquelle
$X^1$, $R^1$, $R^2$, $R^3$ et A ont la définition indiquée ci-dessus, avec des composés de formule (V)

$$X^2\text{-H} \qquad (V),$$

dans laquelle

$X^2$ a la définition indiquée ci-dessus, le cas échéant en présence d'accepteurs d'acides.

**6.** Procédé de production de composés suivant la revendication 1, de formule (Ia)

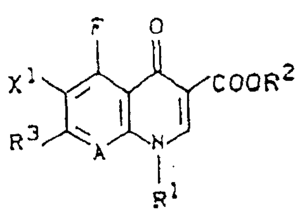

(Ia),

dans laquelle

$X^1$, $X^2$, $R^1$, $R^2$ et A ont la définition indiquée ci-dessus, et $R^3$ représente un reste de structure

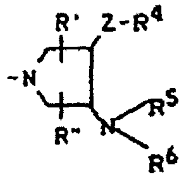

où $R^4$, $R^5$, $R^6$, R', R" et Z ont la définition indiquée ci-dessus,
**caractérisé en ce qu'**on fait réagir un composé de formule (VI)

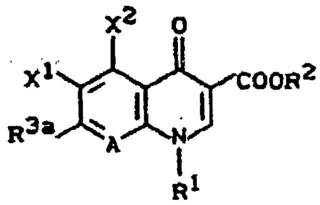

(VI),

dans laquelle
$X^1$, $X^2$, $R^1$, $R^2$ et A ont la définition indiquée ci-dessus et
$R^{3a}$ représente un reste de structure

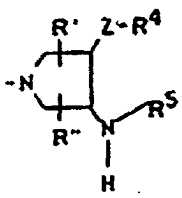

où $R^4$, $R^5$, R', R" et Z ont la définition indiquée ci-dessus, avec des composés de formule (VII)

$$R^6\text{-}X^a \qquad \text{(VII)},$$

dans laquelle

$R^6$ a la définition indiquée ci-dessus et

$X^a$ représente le chlore, le brome, l'iode, un groupe hydroxy ou acyloxy, le cas échéant en présence d'accepteurs d'acides.

**7.** Utilisation de composés de formule (I) suivant la revendication 1 comme additifs pour des aliments pour animaux.

**8.** Aliments pour animaux ou additifs pour des aliments pour animaux et mélanges préalables, contenant des composés de formule (I) suivant la revendication 1.

**9.** Dérivés d'acides carboxyliques à configuration S,S de formule

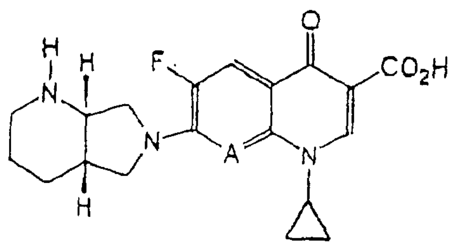

dans laquelle A représente C-Cl, C-F ou C-$OCH_3$ et leurs hydrates et sels acceptables du point de vue pharmaceutique.

**10.** Utilisation de composés suivant la revendication 9, pour la préparation d'aliments pour animaux, d'additifs pour des aliments pour animaux et de mélanges préalables.

**Revendications pour l'Etat contractant suivant : ES**

**1.** Procédé de production de dérivés d'acides 7-(1-pyrrolidinyl)-3-quinolone- et -naphtyridone-carboxyliques de formule (I)

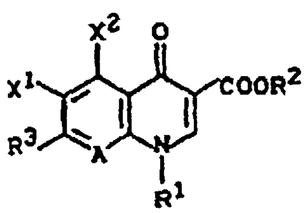

(I),

dans laquelle

$X^1$ représente un halogène,

$X^2$ est l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,

$R^1$ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,

$R^2$ est l'hydrogène, un groupe alkyle ayant 1 à 4 atomes de carbone ou un groupe (5-méthyl-2-oxo-1,3-dioxol-4-yl)méthyle et

$R^3$ est un reste de structure

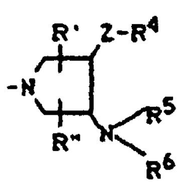

où

$R^4$ peut être un groupe alkyle en $C_1$ à $C_4$, aryle, acyle en $C_1$ à $C_4$,

$R^5$ peut être H, un groupe alkyle en $C_1$ à $C_4$, OH, $OCH_3$,

- $R^6$ peut représenter H, un groupe alkyle en $C_1$ à $C_4$ éventuellement substitué par un radical hydroxy, ainsi qu'un groupe aryle, hétéroaryle, benzyle, (alkoxy en $C_1$ à $C_4$)carbonyle, acyle en $C_1$ à $C_4$, (5-méthyl-2-oxo-1,3-dioxol-4-yl)méthyle ou cycloalkyle en $C_3$ à $C_6$,
- R' peut représenter H, un groupe $CH_3$ ou phényle,
- R" peut représenter H, $CH_3$ ou un groupe phényle, et
- Z peut représenter O ou S,

A représente N ou un groupe C-$R^8$, dans lequel

$R^8$ est l'hydrogène, un halogène, un groupe méthyle, cyano, nitro ou hydroxy, ou peut aussi former conjointement avec $R^1$ un pont de structure

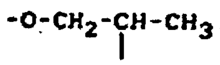, 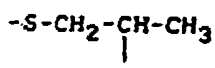

oder

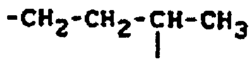

et leurs hydrates et leurs sels d'addition d'acides pharmaceutiquement utilisables ainsi que les sels de métaux alcalins, de métaux alcalino-terreux, d'argent et de guanidinium, des acides carboxyliques correspondants, à l'exception de composés de formule (II)

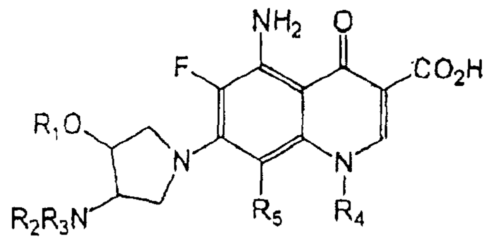

dans laquelle

- $R_1$ est un groupe alkyle en $C_1$ à $C_4$,
- $R_2/R_3$ représentent l'hydrogène ou un groupe alkyle en $C_1$ à $C_4$,
- $R_4$ est un groupe cyclopropyle, phényle, halogénophényle, thiényle, portant au choix un substituant alkyle en $C_1$ à $C_4$ ou halogéno, et
- $R_5$ est un halogène,

**caractérisé en ce qu'**on fait réagir des composés de formule (II)

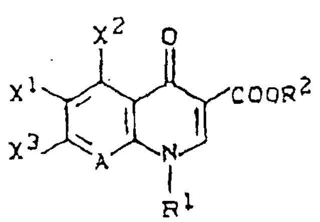 (II),

dans laquelle

A, $R^1$, $R^2$, $X^1$ et $X^2$ ont la définition indiquée ci-dessus et
$X^3$ est un halogène, notamment le fluor ou le chlore, avec des composés de formule (III)

$$R^3 - H \quad (III),$$

dans laquelle

$R^3$ a la définition indiquée ci-dessus,

le cas échéant en présence d'accepteurs d'acides et on élimine éventuellement des groupes protecteurs contenus dans $R^3$, et on transforme éventuellement de manière connue les composés de formule (I) ainsi obtenus en leurs hydrates ou sels d'addition d'acides acceptables du point de vue pharmaceutique ou en sels de métaux alcalins, de métaux alcalinoterreux, d'argent et de guanidinium de l'acide carboxylique de base.

**2.** Procédé suivant la revendication 1, dans lequel

$X^1$ représente le fluor ou le chlore,
$X^2$ est l'hydrogène, un groupe amino, alkylamino ayant 1 ou 2 atomes de carbone, diméthylamino, hydroxy, méthoxy, mercapto, méthylthio, phénylthio, le fluor, le chlore,
$R^1$ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
$R^2$ est l'hydrogène, un groupe alkyle ayant 1 à 3 atomes de carbone ou (5-méthyl-2-oxo-1,3-dioxol-4-yl)méthyle,
$R^3$ représente un reste de structure

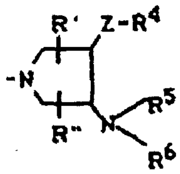

où

$R^4$ peut être un groupe alkyle en $C_1$ à $C_3$, acyle en $C_1$ ou $C_2$,
$R^5$ peut représenter H, un groupe alkyle en $C_1$ à $C_3$, OH, $OCH_3$, $R^4$ et $R^5$ pouvant aussi former ensemble un pont alkylène en $C_1$ ou $C_2$ éventuellement substitué une ou deux fois par un radical méthyle,
$R^6$ peut représenter H, un groupe alkyle en $C_1$ à $C_3$ éventuellement substitué par un radical hydroxy, ainsi qu'un groupe phényle, benzyle, (alkoxy en $C_1$ à $C_4$)carbonyle, acyle en $C_1$ ou $C_2$, (5-méthyl-2-oxo-1,3-dioxol-4-yl)méthyle ou alkyle en $C_3$ à $C_5$,
R' peut représenter H ou un groupe $CH_3$,
R" peut représenter H ou un groupe $CH_3$, et
Z peut représenter O ou S,

A représente N ou un groupe C-$R^8$, dans lequel

$R^8$ représente l'hydrogène, le fluor, le chlore, le brome ou un groupe hydroxy ou peut aussi former conjointement avec $R^1$ un pont de structure

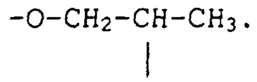

**3.** Procédé suivant la revendication 1, dans lequel

- $X^1$ est le fluor,
- $X^2$ est l'hydrogène, un groupe amino, méthylamino, le fluor,
- $R^1$ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
- $R^2$ est l'hydrogène, un groupe alkyle ayant 1 ou 2 atomes de carbone,
- $R^3$ est un reste de structure

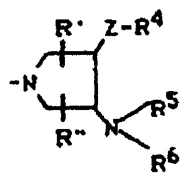

où

- $R^4$ peut représenter un groupe alkyle en $C_1$ ou $C_2$,
- $R^5$ peut représenter H, un groupe alkyle en $C_1$ ou $C_2$, $R^4$ et $R^5$ pouvant aussi éventuellement former ensemble un pont alkylène en $C_1$ ou $C_2$ substitué par un radical méthyle,
- $R^6$ peut être l'hydrogène, un groupe $CH_3$, $C_2H_5$, $HOCH_2CH_2$, benzyle, (alkoxy en $C_1$ à $C_4$)carbonyle, acyle en $C_1$ ou $C_2$,
- R' peut représenter H ou $CH_3$,
- R" peut représenter H ou $CH_3$, et
- Z peut représenter O ou S,

A représente l'azote ou un groupe C-$R^8$ dans lequel

$R^8$ est l'hydrogène, le fluor, le chlore ou peut aussi former conjointement avec $R^1$ un pont de structure

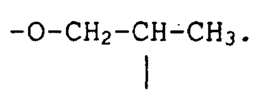

**4.** Procédé de production de composés suivant la revendication 1 de formule (I)

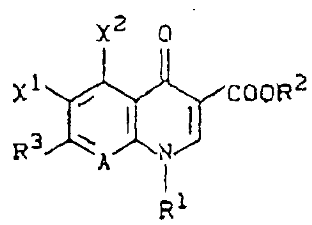

(I),

dans laquelle

$X^1$, $R^1$, $R^2$, $R^3$ et A ont la définition indiquée ci-dessus et

$X^2$ est un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone,

mercapto, alkylthio ayant 1 à 4 atomes de carbone ou arylthio,

**caractérisé en ce qu'**on fait réagir un composé de formule (IV)

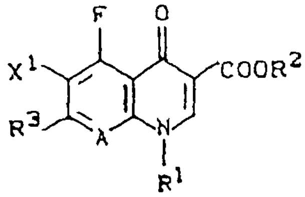

(IV),

dans laquelle
$X^1$, $R^1$, $R^2$, $R^3$ et A ont la définition indiquée ci-dessus, avec des composés de formule (V)

$X^2$-H (V),

dans laquelle

$X^2$ a la définition indiquée ci-dessus, le cas échéant en présence d'accepteurs d'acides.

**5.** Procédé de production de composés suivant la revendication 1, de formule (Ia)

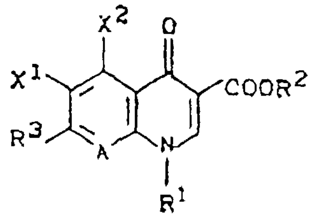

(Ia),

dans laquelle
$X^1$, $X^2$, $R^1$, $R^2$ et A ont la définition indiquée ci-dessus, et
$R^3$ représente un reste de structure

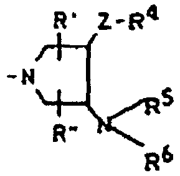

où $R^4$, $R^5$, $R^6$, R', R" et Z ont la définition indiquée ci-dessus,
**caractérisé en ce qu'**on fait réagir un composé de formule (VI)

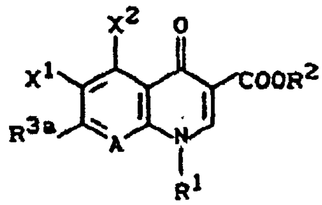

(VI),

dans laquelle

$X^1$, $X^2$, $R^1$, $R^2$ et A ont la définition indiquée ci-dessus et
$R^{3a}$ représente un reste de structure

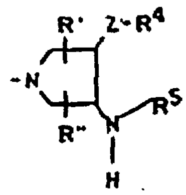

où $R^4$, $R^5$, R', R" et Z ont la définition indiquée ci-dessus, avec des composés de formule (VII)

$$R^6\text{-}X^a \quad (VII),$$

où

$R^6$ a la définition indiquée ci-dessus et
$X^a$ représente le chlore, le brome, l'iode, un groupe hydroxy ou acyloxy,

le cas échéant en présence d'accepteurs d'acides.

**6.** Procédé de production de dérivés d'acides carboxyliques à configuration S,S de formule

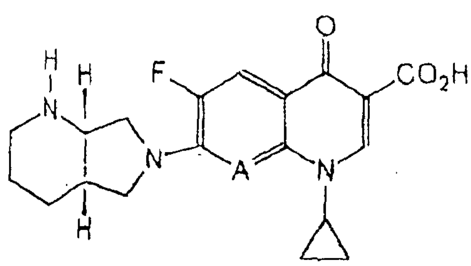

dans laquelle A représente C-Cl, C-F ou C-$OCH_3$,
et de leurs hydrates et sels acceptables du point de vue pharmaceutique,
**caractérisé en ce qu'**on fait réagir des composés de formule

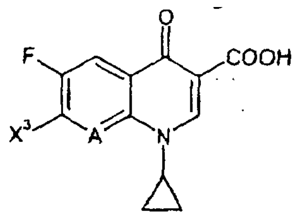

dans laquelle A a la définition indiquée ci-dessus et $X^3$ est un halogène, en particulier le fluor ou le chlore, avec le composé de formule $R^3$-H dans laquelle $R^3$ représente un reste de structure

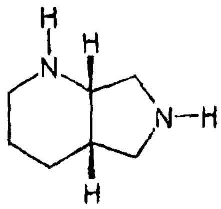

le cas échéant en présence d'accepteurs d'acides, et on élimine éventuellement les groupes protecteurs contenus dans $R^3$, et on transforme éventuellement d'une manière connue les composés de formule (I) ainsi obtenus en hydrates et sels acceptables du point de vue pharmaceutique.